(19) Europäisches Patentamt
European Patent Office
Office européen des brevets

(11) EP 0 093 376 B2

(12) NEW EUROPEAN PATENT SPECIFICATION

(45) Date of publication and mention of the opposition decision:
**07.04.1999 Bulletin 1999/14**

(45) Mention of the grant of the patent:
**21.03.1990 Bulletin 1990/12**

(21) Application number: **83104061.3**

(22) Date of filing: **26.04.1983**

(51) Int Cl.[6]: **C07D 205/08**, C07D 417/12, C07D 417/14, C07D 401/06, C07D 413/14, C07D 277/40, C07D 277/42, A61K 31/365, A61K 31/42, A61K 31/425

(54) **1-Sulfo-2-azetidinone derivatives, their production and use**

Derivate der 2-Azetidinon-1-sulfonsäure, ihre Herstellung und Verwendung

Dérivés de l'acide 2-azétidinone-1-sulfonique, leur préparation et leur utilisation

(84) Designated Contracting States:
**AT BE CH DE FR IT LI LU NL SE**

(30) Priority: **30.04.1982 JP 73728/82**
**31.05.1982 JP 93463/82**

(43) Date of publication of application:
**09.11.1983 Bulletin 1983/45**

(73) Proprietor: **Takeda Chemical Industries, Ltd.**
**Chuo-ku, Osaka (JP)**

(72) Inventors:
- **Kishimoto, Shoji**
  **Takarazuka Hyogo 665 (JP)**
- **Matsuo, Taisuke**
  **Deceased (JP)**
- **Ochiai, Michihiko**
  **Suita Osaka 565 (JP)**

(74) Representative:
**von Kreisler, Alek, Dipl.-Chem. et al**
**Patentanwälte**
**von Kreisler-Selting-Werner**
**Postfach 10 22 41**
**50462 Köln (DE)**

(56) References cited:

| | |
|---|---|
| **EP-A- 0 037 380** | **EP-A- 0 073 061** |
| **GB-A- 2 068 957** | **GB-A- 2 071 650** |
| **GB-A- 2 091 723** | **GB-A- 2 091 724** |

Remarks:
The file contains technical information submitted after the application was filed and not included in this specification

EP 0 093 376 B2

## Description

This invention relates to novel 1-sulfo-2-oxoazetidine derivatives having excellent antimicrobial and β-lactamase-inhibitory activities, processes for preparing the same, and the use of the same, and intermediates and a process therefor.

Heretofore, various 2-oxoazetidine derivatives have been synthesized and reported in e.g. Tetrahedron, *34* (1978), 1731-1767; Chemical Reviews, *76* (1976), 113-155; Synthesis (1973), 327-346, etc.

EP-A-4 053 816, published after the filing date of the present invention, discloses racemic 1-sulfo-2-acetidinone derivatives, thus not the enantiomeric products instantly claimed.

This invention relates to a novel 1-sulfo-2-oxoazetidine derivative of the formula:

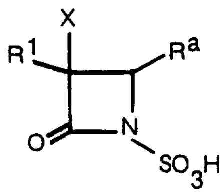

(I)

wherein

X is a hydrogen atom or a methoxy group and

(1) when $R^1$ is a $C_{1-6}$ alkoxycarbonylamino, a $C_{7-9}$ aralkyloxycarbonylamino or a group of the formula:

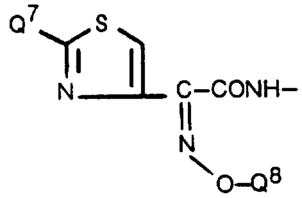

wherein

$Q^7$ is amino group or a protected amino group; and $Q^8$ is a $C_{1-6}$ alkyl group, a group of the formula $-CH_2COOQ^9$ or a group of the formula

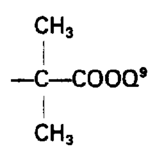

wherein $COOQ^9$ is carboxyl or a $C_{7-9}$ aralkyloxycarbonyl group which may be substituted with nitro group;

$R^a$ is a group of the formula: $-COQ^{0a}$ wherein $Q^{0a}$ is hydrazino, carbamoylhydrazino, $C_{1-6}$-alkoxy-carbonylhydrazino or carbamoyl($C_{1-4}$)alkylamino, or a group of the formula: $-(CH_2)_n a-R^{4b}$ wherein $n^a$ is an integer of 1 to 3, and $R^{4b}$ is carbamoylamino, (N-sulfocarbamoyl)amino pyridinio, $C_{1-6}$-alkylsulfinyl, $C_{1-6}$-alkylsulfonyl, 1-$C_{1-6}$-alkoxyimino-$C_{1-6}$-alkyl or formylamino group,

(2) when $R^1$ is a group of the formula:

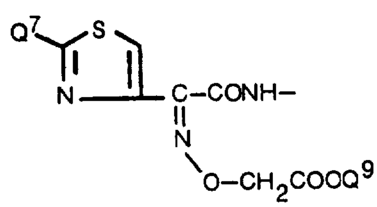

wherein

$Q^7$ and $COOQ^9$ are the same as defined above;

$R^a$ is a $C_{1-6}$-alkylaminocarbonyl group,

(3) when $R^1$ is D-2-(6,7-dihydroxychromone-3-carboxamido)-2-(4-hydroxyphenyl)-acetamido $R^a$ is carbamoyl group,

(4) when $R^1$ is a group of the formula:

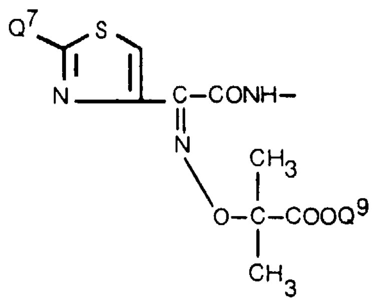

wherein

$Q^7$ and $COOQ^9$ are the same as defined above;

$R^a$ is carboxyl; a group of the formula: $-CH_2CH_2R^{4c}$ wherein $R^{4c}$ is a $C_{1-6}$-alkoxycarbonyl or $C_{2-4}$-acyloxy group; monochloroacetoxymethyl; hydroxymethyl or propyl group,

(5) when $R^1$ is a $C_{1-6}$-alkoxycarbonylamino group; $R^a$ is $-CH_2OSO_2CH_3$,

(6) when $R^1$ is a group of the formula:

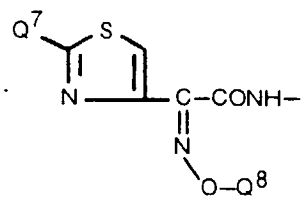

wherein

$Q^7$ and $Q^8$ are the same as mentioned above;

$R^a$ is sulfaminocarbonyloxymethyl, halo-$C_{1-6}$-alkylcarbonylcarbamoyloxymethyl or pyrazolyl or isoxazolyl group which may be substituted with a $C_{1-6}$-alkyl or/and $C_{2-7}$-acyl group, or

(7) when $R^1$ is a group of the formula:

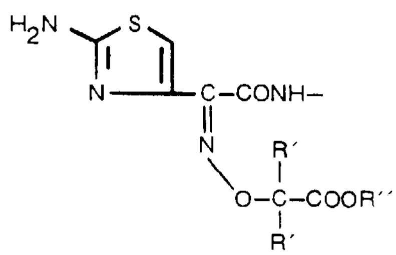

wherein

R' is hydrogen atom or a $C_{1-6}$-alkyl group and R'' is hydrogen atom, a $C_{1-6}$-alkyl group or a $C_{7-9}$-aralkyl group which may be substituted with nitro group;

$R^a$ is carbamoyloxymethyl and the derivative has the (3S, 4S)-configuration; or a pharmaceutically acceptable salt or ester thereof and a process for the preparation thereof, and the use thereof.

The inventors have found that the 1-sulfo-2-oxoazetidine derivative (I) can be obtained by sulfonating a com-

pound of the formula:

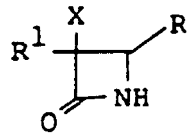

(II)

wherein R, $R^1$ and X are as defined above, or a salt or ester thereof, or by acylating a compound of the formula

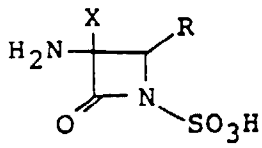

(III)

wherein R and X are as defined above, or a salt or ester thereof, and that the resulting compound (I) possesses excellent antimicrobial and β-lactamase-inhibitory activities, and accomplished this invention on the basis of these findings.

In the foregoing formulas (I), (II) and (III), the symbol R is an organic residue attached to the 2-oxoazetidine nucleus at the 4-position through a carbon atom in said organic residue, i.e. a residue derived from an organic compound by removal of one hydrogen atom attached to a carbon atom tnereof. Such organic residue includes, for example, alkyl, cycloalkyl, alkenyl, alkynyl, cycloalkenyl, aryl, a heterocyclic group, and the like, which may optionally be substituted by one to seven, preferably one to three substituents. Hereinafter, in this specification, any group which may optionally be substituted will be designated by a superscript asterisk "*". For example, an alkyl which may optionally be substituted will be represented by "alkyl*". In such cases, the number of the substituents is not restricted to one, and some substituted groups may have two to a few substituents which may be the same or different. The alkyl is preferably a straight or branched-chain lower alkyl having 1 to 6 carbon atoms, such as methyl, ethyl, n-propyl, isopropyl, n-butyl, isobutyl, sec-butyl, tert-butyl, n-pentyl, isopentyl, n-hexyl, isohexyl or the like. The cycloalkyl preferably has 3 to 8 carbon atoms and includes, for example, cyclopropyl, cyclobutyl, cyclopentyl, cyclohexyl, cycloheptyl, adamantyl, etc. The alkenyl is preferably a straight or branched-chain lower alkenyl having 2 to 6 carbon atoms such as vinyl, ally), isopropenyl, 2-methallyl, 2-butenyl, 3-butenyl or the like. The alkynyl is preferably a straight or branched-chain lower alkynyl having 2 to 6 carbon atoms such as ethynyl, 1-propynyl, 2-propynyl or the like. The cycloalkenyl includes, for example, those having 3 to 8 carbon atoms such as 1-cyclopropenyl, 1-cyclobutenyl, 1-cyclopentenyl, 2-cyclopentenyl, 3-cyclopentenyl, 1-cyclohexenyl, 2-cyclohexenyl, 3-cyclohexenyl, 1-cycloheptenyl, 1,4-cyclohexadienyl, etc. Among others, a cycloalkenyl having 4 to 6 carbon atoms is preferred. The aryl includes, for example, phenyl, α-naphthyl, β-naphthyl, biphenyl, anthryl and the like. Of these, phenyl and naphthyl are usually advantageous. The heterocyclic group includes, for example, 5 to 8-membered heterocyclic rings having one to a few hetero-atoms such as nitrogen (inclusive of N-oxide), oxygen and sulfur, as well as fused rings corresponding thereto, which have an available bonding site at a carbon atom thereof. Examples of such heterocyclic group which are usually advantageous include 2- or 3-pyrrolyl, 2- or 3-furyl, 2- or 3-thienyl, 2-or 3-pyrrolidinyl, 2-, 3- or 4-pyridyl, N-oxido-2-, 3- or 4-pyridyl, 2-, 3- or 4-piperidinyl, 2-, 3- or 4-pyranyl, 2-, 3- or 4-thiopyranyl, pyrazinyl, 2-, 4- or 5-thiazolyl, 2-, 4- or 5-oxazolyl, 3-, 4- or 5-isothiazolyl, 3-, 4- or 5-isoxazolyl, 2-, 4- or 5-imidazolyl, 3-, 4- or 5-pyrazolyl, 3- or 4-pyridazinyl, N-oxido-3- or 4-pyridazinyl, 2-, 4- or 5-pyrimidinyl, N-oxido-2-, 4- or 5-pyrimidinyl, piperazinyl, 4- or 5-(1,2,3-thiadiazolyl), 3- or 5-(1,2,4-thiadiazolyl), 1,3,4-thiadiazolyl, 1,2,5-thiadiazolyl, 4- or 5-(1,2,3-oxadiazolyl), 3- or 5-(1,2,4-oxadiazolyl), 1,3,4-oxadiazolyl, 1,2,5-oxadiazolyl, 1,2,3- or 1,2,4-triazolyl, 1H- or 2H-tetrazolyl, pyrido[2,3-d]pyrimidyl, benzopyranyl, 1,8-, 1,5-, 1,6-, 1,7- 2,7- or 2,6-naphthyridyl, quinolyl, thieno[2,3-b]pyridyl and the like. Among others, a 5- or 6-membered heterocyclic ring having one to four hetero-atoms selected from nitrogen and sulfur, such as thienyl, thiazolyl, thiadiazolyl, triazolyl, tetrazolyl or the like is preferred.

Of these groups, the alkyl, alkenyl and alkynyl groups may be substituted with 1 to 3 substituents such as, for example, cycloalkyl*, cycloalkenyl*, aryl*, a heterocyclic group*, alkoxycarbonyl, acyl, oxo, halogen, cyano, hydroxy, alkoxy, aryl*-oxy, acyloxy, carbamoyloxy, hydroxysulfonyloxy, alkylsulfonyloxy, aryl*-sulfonyloxy, nitro, amino, carboxy, aminocarbonyl, alkylthiocarbonyl, mercapto, alkylthio, aminoalkylthio, acylaminoalkylthio, aralkyl*-thio, aryl*-thio, heterocycle*-thio, quaternary ammonium* or the like. The substituted alkyl group includes, for example, a group of the formula [A]:

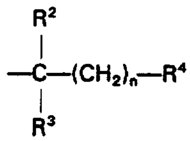

[A]

wherein n is an integer of 0 to 3; $R^2$ and $R^3$ which may be the same or different, stand for hydrogen, alkyl, cycloalkyl*, aralkyl*, aryl*, heterocyclic group*, alkoxycarbonyl or acyl, or $R^2$ and $R^3$ taken together stand for oxo; and $R^4$ is hydrogen, alkyl, cycloalkyl*, aryl*, a heterocyclic group*, halogen, cyano, hydroxy, alkoxy, aryl*-oxy, aralkyl*-oxy, acyloxy, carbamoyloxy, hydroxysulfonyloxy, alkylsulfonyloxy, aryl*-sulfonyloxy, sulfoxy, nitro, amino, azido, carboxy, alkoxycarbonyl, alkoxycarbonylalkyloxy, aminocarbonyl, alkylthiocarbonyl, acyl, mercapto, alkylthio, aminoalkylthio, acylaminoalkylthio, aralkyl*-thio, aryl*-thio, heterocycle*-thio or quaternary ammonium*. In the substituent on the alkyl, alkenyl and alkynyl group, and the group represented by $R^2$, $R^3$ or $R^4$, the alkoxy is preferably a straight or branched-chain lower alkoxy having 1 to 6 carbon atoms such as methoxy, ethoxy, n-propoxy, isopropoxy, n-butoxy, isobutoxy, sec-butoxy, tert-butoxy, n-pentyloxy, isopentyloxy, n-hexyloxy, isohexyloxy or the like. Aralkyl includes, for example, benzyl, phenethyl, phenylpropyl, naphthylmethyl, etc. The halogen includes fluorine, chlorine, bromine and iodine. The quaternary ammonium group includes, for example, a group of the formula:

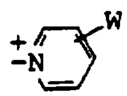

wherein W is hydrogen, an alkyl, carbamoyl, carboxyl, sulfo or alkoxyl, which may be derived from pyridine derivatives such as pyridine, carbamoyl-substituted pyridine (e.g. nicotinamide, isonicotinamide, etc.), carboxyl-substituted pyridine (e.g. nicotinic acid, isonicotinic acid, etc.), sulfa-substituted pyridine (e.g. pyridine sulfonic acid, etc.); quinolinium, etc. These quaternary ammonium may form an inner salt with sulfo group at 1-position of the 2-oxoazetidine nucleus. The acyl includes, for example, formyl, alkylcarbonyl, aryl*-carbonyl, aralkyl*-carbonyl, heterocycle*-acetyl, etc. as well as the acyl groups in $R^1$ mentioned below. Of these, for example, $C_{1-6}$ alkylcarbonyl (e.g. acetyl, propionyl, n-butyryl, isobutyryl, n-pentanoyl, n-hexanoyl, etc.), benzoyl which may be substituted (e.g. benzoyl, 4-hydroxybenzoyl, 4-methoxybenzoyl, etc.), $C_{7-9}$ aralkylcarbonyl which may be substituted (e.g. phenylacetyl, 4-hydroxyphenylacetyl, 4-methoxyphenylacetyl, etc.), 5-membered heterocyclic -carbonyl or -acetyl group containing at least one of oxygen, nitrogen and sulfur, which may be substituted (e.g. 2-thienylcarbonyl, 2-furylcarbonyl, 2-, 4- or 5-thiazolylacetyl, 2- or 3-thienylacetyl, 2- or 3-furylacetyl, 2-amino-4- or 5-thiazolylacetyl, etc.) are preferred. And, the alkyl moiety of alkylsulfonyloxy, alkylthiocarbonyl, alkylthio, aminoalkylthio, acylaminoalkylthio and alkoxycarbonylalkyloxy; the alkoxyl moiety of alkoxycarbonyl and alkoxycarbonylalkyloxy; and the acyl moiety or acyloxy and acylaminoalkylthio have the same meanings as mentioned above.

The substituents (1 to 5, preferably 1 to 3 substituents) which can be present on the cycloalkyl, cycloalkenyl, aralkyl, aryl, heterocyclic and quaternary ammonium groups include, for example, alkyl, alkoxy, alkenyl, aryl, aralkyl, mercapto, alkylthio, arylthio, aralkylthio, alkylsulfonyl, arylsulfonyl, aralkylsulfonyl, trihaloalkyl, hydroxy, oxo, thioxo, halogen, nitro, amino, cyano, carbamoyl, carboxy, acyl, acyloxy, acylamino, hydroxyalkyl, carboxyalkyl, haloalkyl, mono- or dialkylaminoalkyl and the like (wherein the alkyl, alkoxy, alkenyl, aryl, aralkyl, acyl and halogen are those as exemplified above).

When the organic residue R attached to the azetidine ring through a carbon atom therein contains an amino group, said amino group may be substituted or protected, and a carboxyl and a hydroxyl groups, if any, may be likewise protected. The substituents which can be present on the amino group include the acyl in $R^1$ mentioned below, as well as alkyl, alkoxy, hydroxyalkyl, aralkyl*, aryl*, heterocyclic group*, sulfa, alkylsulfonyl, aralkyl*-sulfonyl, aryl*-sulfonyl, alkoxycarbonyl, aralkyl*-oxycarbonyl, aryl*-oxycarbonyl and the like (wherein the alkyl, alkoxy, aralkyl*, aryl* and heterocyclic group* are those as exemplified above). Optionally the amino group, taken together with such substituent, may form a cyclic amino group such as pyrrolidino, piperidino, morpholino, piperazino or the like. The protective group for amino includes, for example, those exemplified below as the "protective group for amino" for $R^1$. The protective group for carboxyl includes any group which can be conventionally used as a carboxy-protecting group in the fields of β-lactam and other organic chemistry, such as ester residues (e.g., methyl, ethyl, n-propyl, isopropyl, tert-butyl, tert-amyl, benzyl, p-nitrobenzyl, p-methoxybenzyl, benzhydryl, phenacyl, phenyl, p-nitrophenyl, methoxymethyl, ethoxymethyl, benzyloxymethyl, acetoxymethyl, pivaloyloxymethyl, β-methylsulfonylethyl, β-trimethylsilylethyl, methylthiomethyl, trityl, β,β,β-trichloroethyl, β-iodoethyl, trimethylsilyl, dimethylsilyl, acetylmethyl, p-nitrobenzoylmethyl, p-mesylbenzoylmethyl, phthalimido-methyl, propionyloxymethyl, 1,1-dimethylpropyl, 3-methyl-3-butenyl, succinimidomethyl,

3,5-di-tert-butyl-4-hydroxybenzyl, mesylmethyl, benzenesulfonylmethyl, phenylthiomethyl, dimethylaminoethyl, pyridine-1-oxido-2-methyl, methylsulfinylmethyl, bis(p-methoxyphenyl)methyl, 2-cyano-1,1-dimethylethyl, etc.), silyl, and the like. The protective group for hydroxyl includes any group which can be conventionally used as a hydroxy-protecting group in the fields of β-lactam and other organic chemistry, such as ester residues, e.g., acetyl, chloroacetyl, etc.; esterified carboxyl groups, e.g., β,β,β-trichloroethoxycarbonyl, β-trimethylsilylethoxycarbonyl, etc.; ether residues, e. g., tert-butyl, benzyl, p-nitrobenzyl, trityl, methylthiomethyl, β-methoxyethoxymethyl, etc.; silylether residues, e.g., trimethylsilyl, tert-butyldimethylsilyl, etc.; acetal residues, e.g., 2-tetrahydropyranyl, 4-methoxy-4-tetrahydropyranyl, etc. and the like. The choice of the above-mentioned hydroxy-protecting group is not critical in the present invention, as is the case with the amino- and carboxy-protecting groups.

Preferred examples of the organic residue R are groups represented by the formula [A], which may be accompanied with the proviso that when both $R^2$ and $R^3$ are hydrogen, $R^4$ is other than hydrogen or an alkyl (especially, a straight-chain alkyl) group; or when one of $R^2$ and $R^3$ is hydrogen, and the other of them is an alkyl (especially, a straight-chain alkyl) group, and n is zero, $R^4$ is other than hydrogen. Among groups [A], a group of the formula [B]:

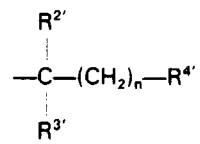 [B]

wherein n is as defined above, $R^{2'}$ and $R^{3'}$, which may be the same or different, respectively stand for hydrogen or alkyl, or $R^{2'}$ and $R^{3'}$ taken together stand for oxo, and $R^{4'}$ is hydrogen, alkyl, aryl*, halogen, cyano, hydroxyl, alkoxyl, aralkyl*-oxy, acyloxy, carbomoyloxy, alkylsulfonyloxy, sulfo-oxy, amino which may be substituted or protected, azido, carboxyl which may be protected, alkoxycarbonyl, alkoxycarbonylalkyloxy, alkylthio, heterocycle*-thio or quaternary ammonium* group, with the proviso that when both $R^{2'}$ and $R^{3'}$ are hydrogen, $R^{4'}$ is other than hydrogen or an alkyl (especially, a straight-chain alkyl) group; when one of $R^{2'}$ and $R^{3'}$ is hydrogen, and the other of them is an alkyl (especially, a straight-chain alkyl) group, and n is zero, $R^4$ is other than hydrogen. And, more favorable ones among the groups [A] and [B] are (1) the case that $R^2$ and $R^3$, $R^{2'}$ and $R^{3'}$ taken together stand for oxo; and $R^4$, $R^{4'}$ is amino group which may be substituted or protected, and n is zero, i.e. a group of the formula [C]:

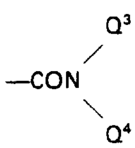 [C]

wherein $Q^3$ and $Q^4$, which may be the same or different, respectively stand for hydrogen; alkyl*; alkoxy; aralkyl*; aryl*; heteroxydic* group; sulfa; alkylsulfonyl; amino; aralkyl*-sulfonyl; aryl*-sulfonyl; alkoxycarbonyl; aralkyl*-oxycarbonyl, aryl*-oxycarbonyl; the acyl or protective group as mentioned below in the symbol $R^1$, preferably, a group of the formula;

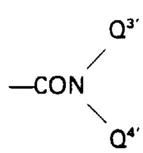

wherein $Q^{3'}$ and $Q^{4'}$ which may be the same or different, respectively stand for hydrogen; $C_{1-6}$ alkyl ; $C_{1-6}$ alkoxyl; amino; carboxy-$C_{1-6}$ alkyl; $C_{1-6}$ alkylcarbonyl which may be substituted with a halogen; sulfa; phenyl; benzoyl; carbamoyl-$C_{1-6}$ alkyl; p-nitrobenzyloxycarbonyl; or β-$C_{1-6}$ alkylsulfonyl-$C_{1-6}$ alkoxycarbonyl, and especially, carbamoyl, mono- or di-$C_{1-6}$ alkylcarbamoyl, phenylcarbamoyl, sulfocarbamoyl, $C_{1-6}$ alkoxycarbamoyl, carboxy-$C_{1-6}$ alkylcarbamoyl, etc., (2) the case that $R^2$ and $R^3$, $R^{2'}$ and $R^{3'}$, taken together, respectively stand for oxo; and $R^4$, $R^{4'}$ is hydroxyl group, alkoxyl, aryl*-oxy, aralkyl*-oxy or alkoxycarbonylalkyloxy group; and n is zero, i.e. a group of the formula [D]:

$$-COQ^5 \qquad [D]$$

wherein $Q^5$ is hydroxyl group which may be protected; alkoxyl; aryloxy which may be substituted; aralkyloxy which may be substituted; or alkoxycarbonylalkyloxy group, preferably, a group of the formula;

$$-COQ^{5'}$$

wherein $Q^{5'}$ is $C_{1-6}$ alkoxyl, p-nitrobenzyloxy or $C_{1-6}$ alkoxycarbonyl-$C_{1-6}$, alkyloxy group, (3) the case that $R^2$ and $R^3$, $R^{2'}$ and $R^{3'}$ are both hydrogen, for example, a group of the formula [E]:

$$-(CH_2)_m-X^1-Q^6 \qquad [E]$$

wherein m is an integer of 1 to 4, $X^1$ is -NH-, -S-, -O- or a direct bond, and $Q^6$ is hydrogen; carbamoyl; an acyl which may be substituted; an alkyl; a heterocyclic group which may be substituted; or a carboxyl group which may be protected, among them [E], a group of the formula;

$$-(CH_2)_m-X^1-Q^{6'}$$

wherein m and $X^1$ are as defined above, and $Q^{6'}$ is an $C_{1-6}$ alkylcarbonyl which may be substituted with a halogen; $C_{1-6}$ alkyl; benzoyl; 5- or 6-membered nitrogen-containing heterocyclic group (e.g. pyrrolyl, thiazolyl, thiadiazolyl, diazolyl, oxazolyl, oxadiazolyl, triazolyl, tetrazolyl, etc.) which may be substituted with a $C_{1-6}$ alkyl group; or 5- or 6-membered nitrogen-containing heterocyclic-thioacetyl which heterocyclic ring may be substituted with a $C_{1-6}$, alkyl. Concrete examples of the group [C] include carbamoyl, N-methylcarbamoyl, N-ethylcarbamoyl, N-propylcarbamoyl, N-isopropylcarbamoyl, N-butyl-carbamoyl, N,N-dimethylcarbamoyl, N,N-diethylcarbamoyl, N-sulfocarbamoyl. N-methoxycarbamoyl, N-ethoxycarbamoyl, N-propoxycarbamoyl, N-isopropoxycarbamoyl, N-hydroxycarbamoyl, N-aminocarbamoyl, [1-(L)-benzyloxycarbonylethyl]aminocarbonyl, [1-(L)-carboxyethyl]aminocarbonyl, N-phenylcarbamoyl, N,N-diphenylcarbamoyl, N-(p-chlorophenyl)carbamoyl, N-benzylcarbamoyl, N,N-dibenzylcarbamoyl, N-hydroxymethylcarbamoyl, N-hydroxyethylcarbamoyl, N-chloromethylcarbamoyl, N-acetylcarbamoyl, N-propionylcarbamoyl, N-carboxycarbamoyl, N-methoxycarbonylcarbamoyl, N-ethoxycarbonylcarbamoyl, N-phenoxycarbonylcarbamoyl and the like. Concrete examples of the group [D] include carboxyl, methoxycarbonyl, ethoxycarbonyl, propoxycarbonyl, isopropoxycarbonyl, n-butyloxycarbonyl, isobutyloxycarbonyl, sec-butyloxycarbonyl, pentoxycarbonyl, benzyloxycarbonyl, phenethyloxycarbonyl, methoxycarbonylmethyloxycarbonyl, ethoxycarbonylmethyloxycarbonyl, phenyloxycarbonyl, p-chlorophenyloxycarbonyl, hydroxymethyloxycarbonyl, sulfomethyloxycarbonyl and the like. Concrete examples of the group [E] include an acylaminoalkyl (e.g. acetylaminomethyl, propionylaminomethyl, n-butyrylaminomethyl, isobutyrylaminomethyl, acetylaminoethyl, propionylaminoethyl, acetylaminopropyl, acetylaminobutyl, benzoylaminomethyl, benzoylaminoethyl, benzoylaminopropyl, formylaminomethyl, phenylacetylaminomethyl, 4-hydroxyphenylacetylaminomethyl, 2-thienylcarbonylaminomethyl, 2-furylcarbonylaminomethyl, thienylacetylaminomethyl, 2-amino-4-thiazolylacetylamino-methyl, etc.), a carbamoylaminoalkyl (e.g. carbamoylaminomethyl, carbamoylaminoethyl carbamoyl-aminopropyl, etc.), an acyloxyalkyl (e.g. acetoxymethyl, propionyloxymethyl, isopropionyloxymethyl, acetoxyethyl, 2-(2-chloroacetamido-4-thiazolyl)-(Z)-2-methoxyiminoacetoxymethyl, 2-(2-amino-4-thiazolyl)-(Z)-2-methoxyiminoacetoxymethyl, 2-thienylacetoxymethyl, 2-furylacetoxymethyl, thiazolyl-acetoxymethyl, 2-amino-4-thiazolylacetoxymethyl, benzoyloxymethyl, benzoyloxyethyl, 4-hydroxy-benzoyloxymethyl, 4-methoxybenzoyloxymethyl, monochloroacetoxymethyl, trichloroethoxycarbonyloxymethyl, acetoacetoxymethyl, (1-methyl-1H-tetrazol-5-yl)thioacetoxymethyl, (1-N,N-dimethylaminoethyl-1H-tetrazol-5-yl)thioacetoxymethyl, (1-carboxymethyl-1H-tetrazol-5-yl)thioacetoxymethyl, (2-methyl-thiadiazol-5-yl)thioacetoxymethyl, (2-methyloxadiazol-5-yl)thioacetoxymethyl, (1-methyl-1H-triazol-5-yl) thioacetoxymethyl, etc.), carbamoyloxyalkyl (e.g. carbamoyloxymethyl, carbamoyloxyethyl, carbamoyloxypropyl, carbamoyloxyisopropyl, etc.), a hetrocyclic-thioalkyl (e.g. thienylthiomethyl, thienylthioethyl, thienylthiopropyl, furylthiomethyl, furylthioethyl, thiazolylthiomethyl, thiazolylthioethyl, 2-aminothiazolylthiomethyl, 2-aminothiazolylthioethyl, 2-aminothiazolylthioisopropyl, (2-methyl-thiadiazol-5-yl)thiomethyl, (2-methyloxadiazol-5-yl)thiomethyl, oxazolylthiomethyl, (1-methyl-1H-triazol-5-yl)thiomethyl, (1-methyl-1H-tetrazol-5-yl)thiomethyl, (1-N,N-dimethylaminoethyl-1H-tetrazol-5-yl)thiomethyl, (1-carboxymethyl-1H-tetrazol-5-yl)thiomethyl, (1-methyl-1H-tetrazol-5-yl)thioethyl, (1-methyl-1H-tetrazol-5-yl)thiopropyl, etc.), a alkylthioalkyl (e.g. methylthiomethyl, methylthioethyl, methylthiopropyl, methylthioisopropyl, ethylthiomethyl, ethylthioethyl, propylthiomethyl, propylthioethyl, isopropylthiomethyl, n-butylthiomethyl, isobutylthiomethyl, sec-butylthiomethyl, etc.) a carboxy- or esterified carboxyalkyl group (e.g. carboxymethyl, carboxyethyl, carboxypropyl, carboxyisopropyl, carboxy-n-butyl, carboxyisobutyl, carboxy-sec-butyl, methoxycarbonylmethyl, ethoxycarbonylmethyl, methoxycarbonylethyl, propoxycarbonylmethyl, isopropoxycarbonyl methyl, phenoxycarbonylmethyl, phenoxycarbonylethyl, benzyloxycarbonylmethyl, benzyloxycarbonylethyl, p-hydroxybenzyloxycarbonylmethyl, p-methoxybenzyloxycarbonylmethyl, carboxymethyloxycarbonylmethyl, etc.) and the like.

In the foregoing Formulas (I), (II) and (III), $R^1$ is an amino group which may optionally be acylated or protected, and the acyl group in the acylated amino group includes any of the conventional acyl groups on the 6- and 7-amino groups of known penicillin derivatives and cephalosporin derivatives, respectively. Examples of the acyl group include (i) a group of the formula [F]:

$$R^5\text{-CO-} \qquad [F]$$

wherein $R^5$ is a lower alkyl or a heterocyclic* group, (ii) a group of the formula [G]:

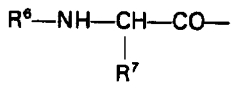

[G]

wherein $R^6$ is hydrogen, an amino acid residue, an amino-protective group or a group $R^8\text{-}(CH_2)_{n_1}\text{-CO-}$ where $R^8$ is a heterocyclic* group and n, is an integer of 0 to 2, and $R^7$ is a lower alkyl, phenyl*, heterocycle*-carbonylamino or a heterocyclic group, (iii) a group of the formula [H]:

$$R^9\text{-}R^{10}\text{-CO-} \qquad [H]$$

wherein $R^9$ is a group

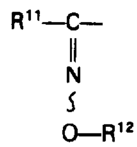

where $R^{11}$ is alkyl*, a heterocyclic* group or phenyl* and $R^{12}$ is hydrogen, a lower alkyl, lower alkenyl or a group $-R^{13}\text{-}R^{14}$ where $R^{13}$ is a lower alkylene or lower alkenylene and $R^{14}$ is phenyl*, carboxyl or an ester thereof, or mono- or di-(lower alkyl)amino, and $R^{10}$ is a direct bond or a group

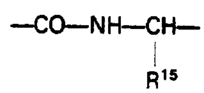

where $R^{15}$ is a lower alkyl, phenyl* or thiazolyl*, (iv) a group of the formula [I]:

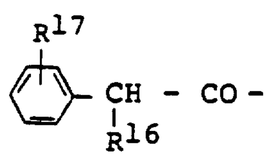

[I]

wherein $R^{16}$ is hydroxy, hydroxysulfonyloxy, carboxy, ureido*, sulfamoyl*, sulfa, phenoxy*carbonyl or formyloxy and $R^{17}$ is hydrogen, a lower alkyl, a lower alkoxy, halogen, nitro or hydroxy, (v) a group of the formula [J]:

$$R^{18}\text{-}R^{19}\text{-}CH_2\text{-CO-} \qquad [J]$$

wherein $R^{18}$ is cyano, phenyl*, phenoxy*, a lower alkyl*, alkenylene* or a heterocyclic* group and $R^{19}$ is a direct bond

or -S-, and the like.

In symbols $R^5$ through $R^{19}$, the alkyl, heterocyclic group, alkoxy and halogen include those exemplified above for R. The amino acid residue includes, for example, glycyl, alanyl, valyl, leucyl, isoleucyl, seryl, threonyl, cysteinyl, cystyl, methionyl, α- or β-aspartyl, α- or γ-glutamyl, lysyl, arginyl, phenylalanyl, phenyl-glycyl, tyrosyl, histidyl, tryptophanyl, prolyl and the like. The protective group for amino includes those exemplified below as the "protective group for amino" for $R^1$. The alkylene has preferably a straight or branched-chain lower alkylene having 1 to 3 carbon atoms and includes, for example, methylene, ethylene, propylene, isopropylene, etc. The alkenylene is preferably a straight or branched-chain lower alkenylene having 2 to 4 carbon atoms such as vinylene, propenylene, or the like. The carboxylic ester includes lower alkyl esters having 1 to 6 carbon atoms in said alkyl moiety such as methyl ester, ethyl ester, propyl ester, n-butyl ester, isobutyl ester, tert-butyl ester, etc. The substituents on the heterocyclic* group, phenyl*, thiazolyl*, phenoxy*carbonyl and phenoxy* include those substituents on the heterocyclic* group and aryl* described above for R. In addition, the substituent on the thiazolyl* may include, for example, an acylamino having 2 to 4 carbon atoms substituted with alkyl, alkoxy, halogen, hydroxy, amino, or the like, and the substituent on the heterocyclic* group may include, for example, phenyl substituted with alkyl, alkoxy, halogen, nitro, amino, etc. The substituent on the ureido* includes, for example, sulfa in the form of salt with a suitable cation such as sodium or potassium; carbamoyl; sulfamoyl; amidino; an alkyl having 1 to 3 carbon atoms; and the like. The substituent on the sulfamoyl* includes, for example, a lower alkyl having 1 to 3 carbon atoms, amidino and the like. The substituent on the lower alkyl* includes, for example, halogen, hydroxy, cyano, trifluoromethyl and the like. The substituent on the alkenylene* includes, for example, carboxy, cyano and the like.

The formula

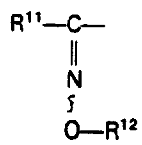

for $R^9$ represents either the *syn* isomer

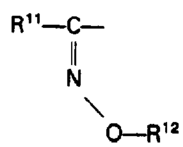

or the *anti* isomer

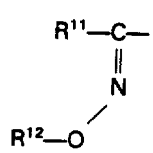

or a mixture thereof.

Among them, preferable one of the groups [G] is a group of the formula:

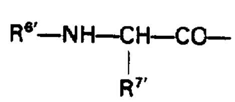

wherein $R^{6'}$ is an amino protective group or a group $R^8$-$(CH_2)_{n_1}$-CO- where $R^8$ is a heterocyclic* group and $n_1$ is an integer of 0 to 2, and $R^{7'}$ is phenyl* or heterocyclic* group; preferable one of the groups [H] is a group of the formula:

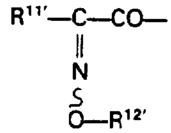

wherein $R^{11'}$ is a heterocyclic* group or phenyl*, $R^{12'}$ is hydrogen, a lower alkyl or a group $-R^{13}-R^{14'}$ wherein $R^{13}$ is as defined above and $R^{14'}$ is carboxyl or an esterified carboxyl group; and preferable one of the groups [J] is a group of the formula:

$$R^{18'}-R^{19}-CH_2-CO-$$

wherein $R^{18'}$ is a heterocyclic* group and $R^{19}$ is as defined above, and the like.

Especially, in view of the antibiotic activities, a group of the formula:

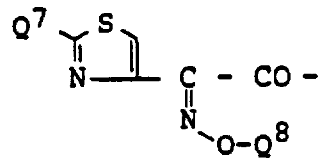

wherein $Q^7$ is amino or a protected amino group, and $Q^8$ is a lower alkyl, a lower alkenyl, a group $-CH_2COOQ^9$ or a group

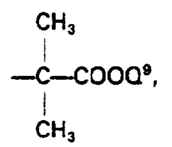

and $COOQ^9$ is carboxyl or an esterified carboxyl group, is more useful as the acyl moiety of the acylated amino group for $R^1$.

In the above-mentioned acyl groups, examples of the acyl group $R^5$-CO- include 3-(2,6-dichloro-phenyl)-5-methylisoxazol-4-yl-carbonyl, 4-ethyl-2,3-dioxo-1-piperazinocarbonyl and the like.

Examples of the acyl group

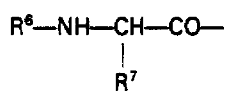

include D-alanyl, benzyl - N$^{\alpha}$ - carbobenzoxy - $\gamma$ - D - glutamyl - D - alanyl, D - phenylglycyl - D - alanyl, N - carbobenzoxy -D - alanyl, N - carbobenzoxy - D - phenylglycyl, D - alanyl -D - phenylglycyl, $\gamma$ - D - glutamyl - D - alanyl, 2 - (4 - ethyl - 2,3 - dioxo - 1 - piperazinocarboxamido) - 2 - phenylacetyl, 2 - (4 - ethyl - 2,3 - dioxo - 1 - piperazinocarboxamido)-2 - (4 - sulfoxyphenyl)acetyl, N - (4 - ethyl - 2,3 - dioxo - 1 - piperazinocarbonyl) - D - alanyl, N - (4 - ethyl - 2,3 - dithiooxo - 1 - piperazinocarbonyl) - D - phenylglycyl, 2,2 - bis - (4 - ethyl - 2, 3 - dioxo - 1 - piperazinocarboxamido)acetyl, 2 - (2 - amino - 4 - thiazolyl) - 2 - (4 - ethyl - 2,3 - dioxo - 1 - piperazinocarboxamido)acetyl, 2 - (4 - hydroxy - 6 - methylnicotinamido) - 2 - phenylacetyl, 2 - (4 - hydroxy - 6 - methylnicotinamido) - 2 - (4 - hydroxyphenyl)acetyl, 2 - {5,8 - dihydro - 2 - (4 - formyl - 1 - piperazinyl) - 5 - oxopyrido - [2,3- d]pyrimidine - 6 - carboxamido} - 2 - phenylacetyl, 2 - (3,5 - dioxo - 1,2,4 - triazine - 6 - carboxamido) - 2 - (4 - hydroxyphenyl)acetyl, 2 - (3 - furfurideneamino - 2 - oxoimidazolidine - 1 - carboxamido) - 2 - phenylacetyl, 2 - (coumarin - 3 - carboxamido) - 2 - phenylacetyl, 2 - (4 - hydroxy - 7 - methyl -1,8 -naphthyridine - 3 - carboxamido) - 2 - phenylacetyl, 2 - (4 -hydroxy -7 - trifluoromethyl- quinoline - 3 - carboxamido) - 2 - phenylacetyl, N - [2 - (2 - amino - 4 - thiazolyl)acetyl] - D - phenylglycyl, 2-(6-bromo-1-ethyl-1,4-dihydro-4-oxothieno[2,3-b]pyridine - 3 - carboxamido) - 2 - phenylacetyl, 2 - (4 - ethyl - 2,3 - dioxo - 1 - piperazinocarboxamido) - 2 - thienylacetyl, 2 - (4 - n - penthyl - 2,3 - dioxo - 1 - piperazinocarboxamido) - 2 - thienylacetyl, 2 - (4 - n - octyl - 2,3 - dioxo - 1 - piperazinocarboxamido) - 2 - thienylacetyl, 2 - (4 - cyclohexyl - 2,3 - dioxo - 1 - piperazinocarboxamido) - 2 - thienylacetyl, 2 - [4 - (2 - phenylethyl) - 2,3 - dioxo - 1 - piperazinocarboxamido]-2-thienylacetyl, 2-(3-methylsulfonyl-2-oxoimidazolidine - 1 - carboxamido) - 2 - phenylacetyl, 2 - (3 - furfurideneamino - 2 - oxoimidazolidine - 1 - carboxamido) - 2 - (4 - hydroxyphenyl)acetyl, 2 - (4 - ethyl - 2,3 - dioxo - 1 - piperazinocarboxamido) - 2 - (4 - benzyloxyphenyl)acetyl, 2 - (4 - ethyl - 2,3 - dioxo - 1 - piperazinocarboxamido) - 2 - (4 - methoxyphenyl)acetyl, 2 - (8 - hydroxy - 1,5 - naphthyridine - 7 - carboxamido) - 2 - phenylacetyl, 2 - (2 - amino - 4 - thiazolyl) - 2 - formamidoacetyl, 2 - (2 - amino - 4 - thiazolyl) - 2 - acetamidoacetyl, and the like.

Examples of the acyl group $R^9$-$R^{10}$-CO- include N - [2 - (2 - amino - 4 - thiazolyl) - 2 - methoxyiminoacetyl] - D - alanyl, N - [2 - (2 - amino - 4 - thiazolyl) - 2 - methoxyiminoacetyl] - D - phenylglycyl, 2 - (2 - amino - 4 - thiazolyl) - 2 - [2 - (2 - amino - 4 - thiazolyl) - 2 - methoxyiminoacetamido]acetyl, 2 - (2 - chloroacetamido - 4 - thiazolyl) - 2 - methoxyiminoacetyl, 2 - (2 - amino - 4 - thiazolyl) - 2 - methoxyiminoacetyl, 2 - (2 - amino - 4 - thiazolyl) - 2 - ethoxyiminoacetyl, 2 - (2 - amino 4 - thiazolyl) - 2 - propoxyiminoacetyl, 2 - (2 - amino 4 - thiazolyl) - 2 - butoxyiminoacetyl, 2 - (2 - amino -4 - thiazolyl) - 2 - benzyloxyiminoacetyl, 2 - (2 - amino- 4 - thiazolyl) - 2 - allyloxyimino-acetyl, 2 - (2 - amino - 4 - thiazolyl) - 2 - [(1 - methyl - 1 - carboxyethyl)oxyimino]acetyl, 2 - (2 - amino - 4 - thiazolyl) - 2 - [(1 - methyl 1 - methoxycarbonylethyl)oxyimino]acetyl, 2 - (2 - amino - 4 - thiazolyl) - 2 - carboxymethyloxyiminoacetyl, 2 - (2 - amino - 4 - thiazolyl) - 2 - carboxyvinyloxyiminoacetyl, 2 - (2 - amino - 5 -chloro 4 - thiazolyl) - 2 - methoxyiminoacetyl, 2 - (2 amino - 5 - bromo - 4 - thiazolyl) - 2 - methoxyiminoacetyl, 2 - (2 - amino - 4 - thiazolyl) - 2 - oxyiminoacetyl, 2 - (2 - amino 4 - thiazolyl) - 2 - carboxyethyloxyiminoacetyl, 2 - (2 - amino - 4 - thiazolyl) - 2 - methoxycarbonylethyloxyiminoacetyl, 2 - thienyl - 2 - methoxyiminoacetyl, 2 - furyl - 2 - methoxyiminoacetyl, 2 - (1,2,4 - thiadiazol - 3 - yl) - 2 - methoxyiminoacetyl, 2 - (1,2,4 - thiadiazolyl) - 2 - methoxyiminoacetyl, 2 - (1,3,4 - thiadiazolyl) - 2 - methoxyiminoacetyl, 2 - (4 - hydroxyphenyl) - 2 - methoxyiminoacetyl, 2 - phenyl - 2 - methoxyiminoacetyl, 2 - phenyl - 2 - oxyiminoacetyl, 2 - [4 - (γ - D - glutamyloxy)phenyl] - 2 - oxyiminoacetyl, 2 - [4 - (3 - amino - 3 - carboxypropoxy)phenyl] - 2 - oxyiminoacetyl, and the like.

Examples of the acyl group

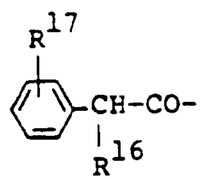

include α-sulfophenylacetyl, α-hydroxyphenylacetyl, α-ureidophenylacetyl, α-sulfoureidophenylacetyl, α-sulfamoylphenylacetyl, α-phenoxycarbonylphenylacetyl, α-(p-tolyloxycarbonyl)phenylacetyl, α-formyloxyphenylacetyl and the like.

Examples of the acyl group $R^{18}$-$R^{19}$-$CH_2$-CO- include cyanoacetyl, phenylacetyl, phenoxyacetyl, trifluoromethylthioacetyl, cyanomethylthioacetyl, 1H-tetrazolyl-1-acetyl, thienylacetyl, 2-(2-amino-4-thiazolyl)acetyl, 4-pyridylthioacetyl, 2-thienylthioacetyl, 3,5-dichloro-1,4-dihydro-4-oxopyridine-1-acetyl, β-carboxyvinylthioacetyl, 2-(2-aminomethylphenyl)acetyl and the like.

The amino, carboxyl and/or hydroxyl group in the above-exemplified acyl groups may be protected by a protective group.

The protective group for amino includes those described below as the "protective group for amino".

The protective groups for carboxyl or hydroxyl include those described above for R.

As the protective group for amino for $R^1$ which may optionally be protected, any of those used for this purpose in the field of β-lactam or peptide synthesis may conveniently be employed. Examples of such amino-protecting group include aromatic acyl groups such as phthaloyl, p-nitrobenzoyl, p-tert-butyl-benzoyl, p-tert-butylbenzenesulfonyl, benzenesulfonyl, toluenesulfonyl, etc., aliphatic acyl groups such as formyl, acetyl, propionyl, monochloroacetyl, dichloroacetyl, trichloroacetyl, methanesulfonyl, ethanesulfonyl, trifluoroacetyl, maleyl, succinyl, etc., and esterified carboxyl groups such as methoxycarbonyl, ethoxycarbonyl, t-butoxycarbonyl, isopropoxycarbonyl, 2-cyanoethoxycarbonyl, β,β,β-trichloroethoxycarbonyl, β-trimethylsilylethoxycarbonyl, β-methylsulfonylethoxycarbonyl, benzyloxycarbonyl, p-nitrobenzyloxycarbonyl, p-methoxybenzyloxycarbonyl, diphenylmethyloxycarbonyl, methoxymethyloxycarbonyl, acetylmethyloxycarbonyl, isobornyloxycarbonyl, phenyloxycarbonyl, etc., as well as non-acyl amino-protecting groups such as trityl, 2-nitrophenylthio, benzylidene, 4-nitrobenzylidene, trialkylsilyl, benzyl, p-nitrobenzyl, proton, etc. The choice of amino-protecting group is not critical in the present invention.

The objective compound (I) of this invention may have the 1-sulfo group and the carboxyl group or groups in R and/or $R^1$ in their free form. Alternatively, it may be in the form of a pharmaceutically acceptable salt formed with a non-toxic cation, e.g., sodium, potassium, etc., a basic amino acid e.g., arginine, ornithine, lysine, histidine, etc., a polyhydroxyalkylamine, e.g., N-methylglucamine, diethanolamine, triethanolamine, trishydroxymethylaminomethane, etc. or the like. When R and/or $R^1$ contains a basic group, the compound (I) may be in the form of a salt formed with an organic acid, e.g., acetic acid, tartaric acid, methanesulfonic acid, etc., an inorganic acid, e.g., hydrochloric acid,

hydrobromic acid, sulfuric acid, phosphoric acid, etc., an acidic amino acid, e.g., aspartic acid, glutamic acid, etc., or the like. In addition, when R and/or $R^1$ contains a carboxyl group, the compound (I) may be converted into its biologically active ester derivative conducive to increased blood concentration and prolonged in vivo activity. The ester groups effective in such cases include, for example, α-alkoxy-α-substituted-methyl groups (e.g. alkoxymethyl or α-alkoxyethyl such as methoxymethyl, ethoxymethyl, isopropoxymethyl, α-methoxyethyl, α-ethoxyethyl, etc.), alkylthiomethyl groups such as methylthiomethyl, ethylthiomethyl, isopropylthiomethyl, etc., acyloxymethyl or α-acyloxy-α-substituted-methyl groups such as pivaloyloxymethyl, α-acetoxybutyl, etc., α-alkoxycarbonyloxy-α-substituted-methyl groups such as ethoxycarbonyloxymethyl, α-ethoxycarbonyloxyethyl, etc., and the like.

The compound (I) of this invention can be present as various steric isomers (e.g., *cis, trans, syn, anti* and optically active isomers) as is the case with the starting compound (II) as mentioned below, and it is to be understood that the present invention encompasses these individual isomers as well as mixtures thereof. These isomers can be used as a medicament either singly or in admixture.

Accordingly, a type of the steric isomer of the compound (I) of this invention, for example, may be described by the formula:

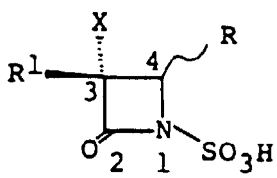

wherein R, $R^1$ (β-configuration) and X (α-configuration) are as defined above, and the wave line means *trans* or *cis* configuration of the group R to the group $R^1$ at 3-position.

The objective compound (I) of this invention include the compounds listed below;

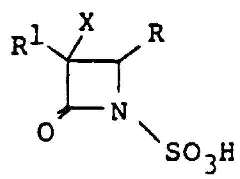

| Compound | $R_1$ | X | R |
|---|---|---|---|
| 1 | 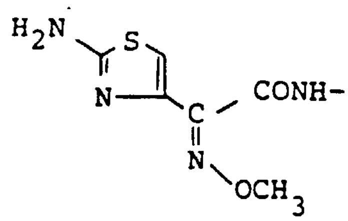 | H | 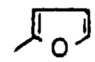 |
| 2 | 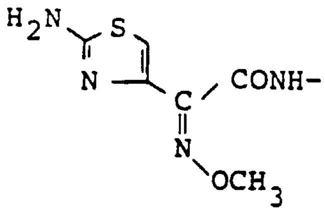 | H | $-CH_2OCH_3$ |
| 3 | 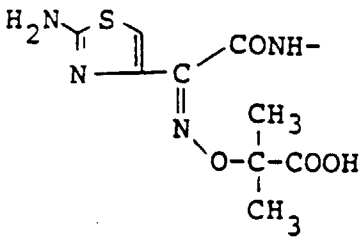 | H | $-CH_2OCH_3$ |
| 4 | 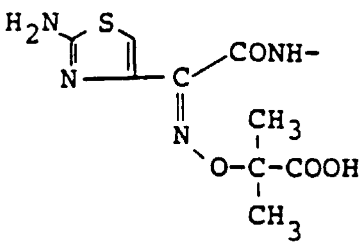 | H | $-CH_2(CH_2)_3OCONH_2$ |

Among others, preferable one of the objective compound (I) in this invention may be a compound represented by the formula:

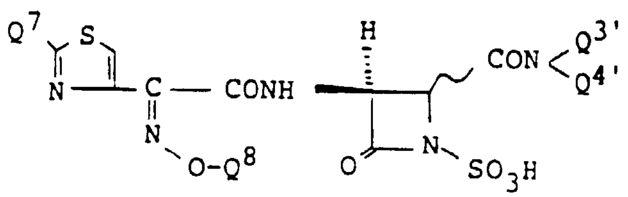

wherein $Q^{3'}$, $Q^{4'}$, $Q^7$, $Q^8$ and wave line are as defined above, or a salt or ester thereof. In the above formula, when $Q^7$ is amino group, the compound of the formula may form an inner salt between the amino group of $Q^7$ and sulfa group at 1-position.

The objective compounds (I) and salts or esters thereof, in a crystalline or non-crystalline form, are valuable antibiotics active against a variety of gram-positive or gram-negative bacteria, are applied as medicaments to human beings or domestic animals and are used with safety as antimicrobial agents for the treatment of infections caused by gram-positive or gram-negative bacteria. In addition, the antimicrobial agents of this invention are added to animal rations as disinfectants for preservation of feeds. The antibiotics can also be used in the form of an aqueous formulation having a concentration in the range of 0.1 to 100 ppm (i.e., 0.1 to 100 parts of the antibiotic per million parts of the solution) as antimicrobial preparations in order to destroy and inhibit growth of harmful bacteria, for example, on a medical and dental equipment or in order to inhibit growth of harmful bacterial life in an industrial aqueous medium, for example, water-based paints or paper mill white water.

The objective compounds (I) and their salts or esters can be used singly or in combination with one or more other active components in any of various pharmaceutical preparations such as capsules, tablets and powders as well as solutions, suspensions and elixers. These preparations can be administered orally, intravenously or intramuscularly.

The oral tablets may contain a conventional excipient such as, for example, a bindery e.g., syrup, gum arabic, gelatin, sorbitol, gum tragacanth or polyvinylpyrrolidone; a filler, e.g., lactose and other sugars, corn starch, calcium phosphate, sorbitol or glycine; a lubricant, e.g., magnesium stearate, talc, polyethylene glycol or silica; a disintegrator, e.g., potato starch; or an available wetting agent such as sodium lauryl sulfate. The tablets may be coated in a manner well known in the art. The oral liquid preparations may be in such dosage forms as aqueous or oil suspensions, solutions, emulsions, syrups, elixers, etc., or in the form of lyophilisates for extemporaneous dissolution in water or a suitable solvent. These liquid preparations may contain a suspending agent, e.g., sorbitol syrup, methyl cellulose, glucose/sugar syrup, gelatin, hydroxyethyl-cellulose, carboxymethyl-cellulose or aluminum stearate gel; a hydrogenated edible oil, e.g., almond oil, coconut oil fractions, oily esters, propylene glycol or ethyl alcohol; or a preservative, e.g., methyl or propyl p-hydroxybenzoate or sorbic acid. Suppositories may contain a conventional suppository base, e.g., theobroma oil or other glycerides.

Injectable compositions can be made available in ampules or other unit-dose containers with the addition of a preservative. These compositions may be in such dosage forms as suspensions, solutions or emulsions in an oily or aqueous vehicle, and may contain a suitable adjuvant or adjuvants such as a suspending agent, stabilizer and/or dispersing agent. Alternatively, the active component may be in the powder form reconstitutable with a suitable solvent such as sterilized pyrogen-free water prior to use.

The active component also can be formulated into suitable dosage forms absorbable through the mucous membranes of the nose and throat or the bronchial tissues, for example, powders, liquid sprays or inhalants, lozenges, throat paints, etc. For ophthalmological or otological application, it can be administered as liquid or semi-solid capsules or as drops for instillation. In addition it may be formulated with hydrophobic or hydrophilic pharmaceutical bases in such dosage forms as ointments, creams, lotions, paints, powders, etc. to provide pharmaceutical preparations for external application.

In addition to the carriers, these preparations may contain other components such as stabilizers, binding agents, antioxidants, preservatives, lubricants, suspending agents, rheology modifiers, or flavoring agents. Moreover, other active component or components can be incorporated in the composition to provide for a broader antimicrobial spectrum.

For administration to domestic animals, the active component of this invention can be formulated with time-release media to provide intramammary preparations.

The compounds (I) of this invention can be applied to mammals as therapeutic agents for microbial infections in the treatment of, for example, respiratory tract infections, urinary tract infections, suppurative infections, bile duct infections, intestinal infections, gynecological infections, surgical infections, etc. The daily dosage varies with the condition of the patient to be treated, the weight of the host, the route and frequency of administration and the particular parentheral procedure suitable for general infections or oral procedure employed for intestinal infections. Generally the oral daily dosage comprises the active component in an amount of about 15-600 mg/kg of body weight of the patient in one or more doses. The daily dosage suitable for administration to an adult human is about 10 to about 200 mg/kg/body weight as the active ingredient, which can suitably be administered daily in 2 to 4 doses of about 2.5 to 100 mg/kg each, for example, by a parenteral injection.

A pharmaceutical composition containing the compound (I) can be administered, for example, in various solid or liquid orally ingestable unit dosage forms. The liquid or solid composition may contain 0.5 to 99% of the active component. The preferred concentration range of the active component is about 10 to about 60%. The composition generally contains about 15 to 1500 mg of the active component in each unit dose, and it is generally preferred to use a unit dose in the range of about 250 to 1000 mg as the active component.

In addition to the uses mentioned above, the compounds (I) of this invention and their salts and esters may be used in combination with a β-lactam antibiotic since they possess β-lactamase-inhibitory activities. Examples of such β-lactam antibiotic include penicillin antibiotics such as benzylpenicillin, phenoxymethylpenicillin, carbenicillin, ampicillin, amoxicillin, sulbenicillin, etc., cephalosporin antibiotics such as cephaloridine, cephalothin, cefazolin, cephalexin, cefoxitin, cephacetrile, cefamandole, cefmenoxime, cefsulodin, cefotiam, cefotaxime, cephapirin, ceftizoxime, cefradin, cephaloglycin, etc. and the like.

The 1-sulfo-2-oxoazetidine derivatives (I) that are the objective compounds of this invention can be prepared, for example, by sulfonating a compound (II).

The compound (II) may be used as the starting material in the process of this invention in the form of salts with various acids or bases, or esters or silyl derivatives. The compound (II) includes the *cis*- and *trans*-isomers because it has substituents at the 3- and 4-positions. In addition, since the 3- and 4-carbon atoms are asymmetric, theoretically there exist at least four stereoisomers in a total. These stereoisomers may be used either singly or in admixture. This is also the case when the group R or $R^1$ contains an asymmetric carbon, and the resulting stereoisomers may also be

used either singly or in admixture.

As salts and esters of the compound (II), use is made of, for example, those exemplified above for the compound (I), and so on. The compound (II) may also be silylated by the silylating agent. The silylating agent may be a compound of the formula:

$$P^1P^2P^3Si{\cdot}Hal,$$

wherein each of $P^1$, $P^2$ and $P^3$ is a hydrocarbon residue such as a lower alkyl of 1 to 4 carbon atoms (e.g. methyl, ethyl, n-propyl, i-propyl, n-butyl, etc.), an aryl group (e.g. phenyl, tolyl, etc.) or the like, and Hal is halogen, preferably chlorine or bromine, and one or two of $P^1$, $P^2$ and $P^3$ may be halogen, preferably chlorine or bromine, and one of $P^1$, $P^2$ and $P^3$ may be hydrogen. Furthermore, hexa-alkyl($C_1$-$C_4$)cyclotrisilazane, octaalkyl($C_1$-$C_4$)cyclotetrasilazane, tri-alkyl($C_1$-$C_4$)silylacetamide, bis-tri-alkyl($C_1$-$C_4$)silylacetamide may be used as the silylating agent. The preferred silylating agent is a group of the formula:

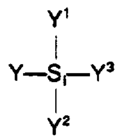

wherein Y stands for a reactive group to be liberated from the silyl compound, $Y^1$ and $Y^2$ respectively stand for a lower ($C_{1-4}$)alkyl, phenyl, benzyl or a lower ($C_{1-4}$) alkoxyl group, and $Y^3$ stands for t-butyl or isopropyl group.

As the lower alkyl represented by $Y^1$ and $Y^2$, there may be mentioned methyl, ethyl, n-propyl, isopropyl, n-butyl, t-butyl and the like, and as the lower alkoxy represented by $Y^1$ and $Y^2$, there may be mentioned methoxy, ethoxy, n-propoxy, isopropoxy, n-butoxy, t-butoxy and the like.

As the reactive group to be liberated from the silyl compound may be exemplified halogen (e.g. chloro, bromo); N-(trialkylsilyl)trifluoroacetimidoyloxy group; N-(trialkylsilyl)acetimidoyloxy group; an acylamino group such as formylamino, acetylamino, propionylamino, butylylamino or trifluoroacetylamino; a (trialkylsilyl)amino group such as (tri-t-butyldimethylsilyl)amino, isopropyldimethylsilylamino or (chloro-methyldimethylsilyl)amino; amino; an alkylamino group such as methylamino, ethylamino or propylamino; an N,N-dialkylamino group such as N,N-dimethylamino, N-chloromethyl-N-methylamino, N,N-diethylamino, N,N-dipropylamino, N-methyl-N-ethylamino, N-methyl-N-propylamino or N-ethyl-N-propylamino; or a heterocyclic group such as imidazolyl. As the alkyl moiety in said reactive group, ones having 1 to 4 carbon atoms are preferable, and it is exemplified by methyl, ethyl, n-propyl, isopropyl, n-butyl and t-butyl. Specific examples of the silyl compounds as described above, there may be mentioned N,O-bis(t-butyldimethylsilyl)trifluoroacetamide, N,O-bis(isopropyldimethylsilyl)acetamide, bis(dimethyl-isopropylsilyl)acetamide, isopropyldimethylsilylacetamide, bis(dimethyl-tert-butylsilyl)acetamide, N-methyl-N-t-butyldimethylsilylacetamide, N-methyl-N-isopropyldimethylsilyltrifluoroacetamide, N-t-butyl-dimethylsilyldiethylamine, 1,3-bis(chloromethyl)-1,1,3,3-tetra-t-butyldimethyldisilazane, N-isopropyl-dimethylsilylimidazole, t-butyldiphenylchlorosilane, isopropyldiethylchlorosilane, isopropylmethyldichlorosilane, tert-butyldimethylchlorosilane, isopropyldimethylchlorosilane or t-butyldiethylchlorosilane. Among them, tert-butyldimethylchlorosilane or isopropyldimethylchlorosilane is preferable. The present silylating reaction is followed by a *per se* conventional one. The reaction temperature of the silylation is in the range of about 0° to 50°C, preferably not higher than 38°C, usually a room temperature (about 20°C), and the reaction time is from several minutes (about 10 minutes) to about 24 hours. The reaction is conducted conveniently in, for example, ethyl acetate, dioxane, tetrahydrofuran, N,N-dimethylacetamide, N,N-dimethylformamide, dichloromethane, chloroform, benzene, toluene, acetone, methylethylketone, or acetonitrile, or an optional mixture of them, or any other solvent which is inert to this reaction. This reaction can be conducted also in the presence of an inorganic base such as sodium hydroxide, potassium hydroxide, sodium hydrogen carbonate, sodium carbonate or potassium carbonate or a trialkylamine such as triethylamine, tributylamine; a triaralkylamine such as tribenzylamine; an organic tertiary amine such as N-methylmorpholine, N-methylpiperidine, N,N-dialkylaniline, N,N-dialkylbenzylamine, pyridine, picoline or lutidine; or an organic base such as 1,5-diazabicyclo[2,2,2]octane or 1,8-diazabicyclo[5,4,4]undecene-7, and when the base is liquid, it can be used also as a solvent. Thus obtained silyl derivative of the compound (II) may be employed as the starting material in the present sulfonating reaction, as it is i.e. in the form of a reaction mixture, or after isolated or purified by means of the conventional method as mentioned below.

The sulfonation reaction means that a sulfa group is introduced into the compound (II) at the 1-position, and is accomplished by reacting the compound (II) with, for example, sulfuric anhydride or its reactive derivative. The reactive derivative of sulfuric anhydride includes, for example, its adducts such as sulfuric anhydride-base (e.g. sulfuric anhydride-pyridine, sulfuric anhydride-trimethylamine, sulfuric anhydride-picoline, sulfuric anhydride-lutidine, etc.), sulfuric anhydride-N,N-dimethylformamide, sulfuric anhydride-dioxane, sulfuric anhydride-chlorosulfonic acid and the like.

For this reaction, about 1 to 10 moles, preferably about 1 to 5 moles of sulfuric anhydride or its reactive derivative is used for each mole of the compound (II). The reaction temperature is about -78 to 80°C, preferably about -20 to 60°C. The reaction may be carried out in the presence of a solvent. In such cases, conventional organic solvents including water, ethers such as dioxane, tetrahydrofuran, diethyl ether, etc., esters such as ethyl acetate, ethyl formate, etc., halogenated hydrocarbons such as chloroform, dichloromethane, etc., hydrocarbons such as benzene, toluene, n-hexane, etc., amides such as N,N-dimethylformamide, N,N-dimethylacetamide, etc. and the like can be used singly or in admixture. The reaction goes to completion usually in scores of minutes to scores of hours depending on the particular starting compound (II), sulfonating agent, reaction temperature and solvent employed, but in some cases it takes scores of days to complete the reaction. After completion of the reaction, the reaction mixture can be subjected to a suitable purification and separation procedure known *per se* such as solvent extraction, recrystallization, chromatography or the like to give the desired compound (I) of any purity.

Alternatively, the objective compound (I) of this invention can be prepared by acylating a compound of the formula:

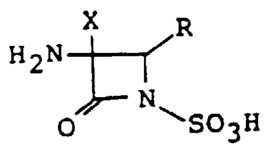

(III)

wherein R and X are as defined above.

The acylation according to this process is accomplished by reacting the compound (III) (inclusive of its various salts, esters and silyl derivatives) with an acylating agent in an amount of at least 1 mole, preferably 1.2 to 4 moles per each mole of the compound (III). The acylating agent used in this reaction may be either the organic carboxylic acid comprising the acyl group in $R^1$ [R°COOH wherein R°CO is an acyl group defined above as the acyl group of the acylated amino group for $R^1$] or its reactive derivative at the carboxyl group.

The reactive derivative of the organic carboxylic acid includes, for example, acid anhydrides, active amides, active esters, acid halides, active thioesters, etc., which are exemplified below.

1) Acid anhydrides:

The acid anhydrides include, for example, mixed acid anhydrides with a hydrohalic acid (e.g., hydrochloric acid, hydrobromic acid, etc.), a monoalkylcarbonic acid, an aliphatic carboxylic acid (e.g., acetic acid, pivalic acid, valeric acid, isovaleric acid, trichloroacetic acid, etc.), and an aromatic carboxylic acid (e.g., benzoic acid, etc.), as well as symmetrical acid anhydrides.

2) Active amides:

The active amides include, for example, amides with pyrazole, imidazole, a 4-substituted imidazole, dimethylpyrazole, benzotriazole, etc.

3) Active esters:

The active esters include, for example, such esters as methyl, ethyl, methoxymethyl, propargyl, 4-nitrophenyl, 2,4-dinitrophenyl, trichlorophenyl, pentachlorophenyl, and mesylphenyl esters, as well as the esters of the above-mentioned carboxylic acids with 1-hydroxy-1H-2-pyridone, N-hydroxysuccinimide, N-hydroxyphthalimide or the like.

The choice of reactive derivative of an organic carboxylic acid depends on the particular acid used. When the acylating agent used is a free carboxylic acid, the reaction is preferably carried out in the presence of a condensation agent. Examples of such condensation agent include N,N'-dicyclohexylcarbodiimide, N-cyclohexyl-N'-morpholinoethylcarbodiimide, N-cyclohexyl-N'-(4-diethylaminocyclohexyl)carbodiimide, N-ethyl-N'-(3-dimethylaminopropyl)carbodiimide, and the like.

The acylation is usually carried out in a solvent. The solvent includes water, acetone, dioxane, acetonitrile, methylene chloride, chloroform, dichloroethane, tetrahydrofuran, ethyl acetate, N,N-dimethylformamide, N,N-dimethylacetamide, pyridine and other conventional organic solvents which do not affect the reaction. Of these, hydrophilic solvents may be used in combination with water.

The acylation may also be carried out in the presence of a base such as an alkali metal carbonate, a trialkylamine (e.g., trimethylamine, triethylamine, tributylamine, N-methylmorpholine and N-methylpiperidine, etc.), an

N,N-dialkylaniline, an N,N-dialkylbenzylamine, pyridine, picoline, lutidine, 1,5-diazabicyclo[4,3,0]nonan-5-ene, 1,4-diazabicyclo[2,2,2]octane, 1,8-diazabicyclo[5,4,4]undecene-7 and the like. Among the bases and the above-mentioned condensation agents, liquid compounds can also function as a solvent as well. The reaction temperature is not critical, and the reaction is usually carried out under cooling to room temperature, and goes to completion in a few minutes to several ten hours. The resulting compound (I) can be isolated and purified in a manner known *per se* such as concentration, conversion of liquid nature from acidic to basic or vice versa, change of solvent, solvent extraction, crystallization, recrystallization, fractional distillation, chromatography and the like.

When the starting compound (III) or its salt and/or the acylating agent used in the acylation contains an asymmetric carbon in the molecule, the stereoisomers may be used either singly or in admixture. If the acylation causes the formation of two or more of stereoisomeric products, the individual stereoisomers can be isolated in a conventional manner such as column chromatography, recrystallization or the like, if necessary.

The group R in the objective compound (I) may be transformed into another group while the compound (I) retains the 1-sulfa group, which leads to the formation of another objective compound of this invention. For instance, if R is acetoxymethyl, methanesulfonyloxymethyl, iodomethyl or the like, it can be transformed into another desired group by the reaction with a nucleophilic reagent. The nucleophilic agents that can be used include, for example, alkyl*-thiols, aryl*-thiols, heterocycle*-thiols, pyridines* and the like and they can provide the objective compounds (I) wherein R is a substituted thiomethyl, a quaternary ammonium*-methyl or the like. The alkyl*, aryl*, heterocyclic* group and quaternary ammonium* in these nucleophilic agents are as defined above. The reaction is preferably carried out in an aqueous solution, or in a water-miscible solvent such as acetone, acetonitrile, N,N-dimethylformamide or the like, or a mixture of water and such water-miscible solvent. There are cases in which the addition of a base such as an alkali carbonate, an alkali phosphate or the like is beneficial. The reaction is usually conducted at a temperature in the range of 20 to 100°C. The resulting compound (I) can be isolated and purified by a procedure known *per se* as is the case with those obtained by sulfonation or acylation.

When the objective compound (I) thus obtained has a protective group, the protective group can be removed if necessary. Removal of such protective group can be accomplished by a conventional method such as the one involving the use of an acid, a base or hydrazine, reduction, and the method comprising the use of an iminohalogenating agent and, then, an iminoetherifying agent, if necessary, followed by hydrolysis. The choice of any such method depends on the particular protective group to be removed. When the protective group is removed with an acid, the acids that can be used include inorganic acids such as hydrochloric acid, sulfuric acid, phosphoric acid, etc. and organic acids such as formic acid, acetic acid, trifluoroacetic acid, propionic acid, benzenesulfonic acid, p-toluenesulfonic acid, etc. as well as acidic ion-exchange resins, and the choice of acid depends on the particular protective group and other conditions. In the case in which the protective group is removed with a base, the bases that can be used include inorganic bases such as alkali metal (e.g., sodium, potassium, etc.) or alkaline earth metal (e.g. calcium, magnesium, etc.) hydroxides, carbonates, etc. and organic bases such as metal alkoxides, organic amines, quaternary ammonium salts, etc. as well as basic ion-exchange resins, and the choice of base depends on the particular protective group and other conditions.

When the removal of a protective group with an acid or base is conducted in a solvent, an hydrophilic organic solvent, water or a mixed solvent is generally used.

When the protective group is removed by reduction, for example, the reduction with a combination of a metal such as tin or zinc or a metal compound such as chromium dichloride or chromium acetate and an organic or inorganic acid such as acetic acid, propionic acid or hydrochloric acid, catalytic reduction in the presence of a metal hydrogenation catalyst or the like may be employed depending on the particular protective group and other conditions. The catalysts that can be used in the catalytic reduction include, for example, platinum catalysts such as platinum wire, platinum sponge, platinum black, platinum oxide, colloidal platinum, etc., palladium catalysts such as palladium sponge, palladium black, palladium oxide, palladium on barium sulfate, palladium on barium carbonate, palladium on charcoal, palladium on silica gel, colloidal palladium, etc., nickel catalysts such as reduced nickel, nickel oxide, Raney nickel, Urushihara nickel, etc. and the like.

In the case of reduction with a metal and an acid, a metal compound of such metal as iron, chromium or the like and an inorganic acid such as hydrochloric acid or an organic acid such as formic acid, acetic acid or propionic acid are used. The removal of a protective group by reduction is usually conducted in a solvent, and the solvent which is conveniently used in the catalytic reduction includes, for example, alcohols such as methanol, ethanol, propyl alcohol, isopropylalcohol, etc., ethyl acetate and the like. The reduction with a metal and an acid is conveniently conducted in water, acetone or the like, but if the acid is liquid, it can also function as a solvent.

In any of the methods involving the use of an acid or a base or reduction, the reaction is usually carried out under cooling to under warming.

When the protective group is removed by reacting an iminohalogenating agent and then an iminoetherifying agent, if necessary, followed by hydrolysis, the iminohalogenating agents that can be used include, for example, phosphorus trichloride, phosphorus pentachloride, phosphorus tribromide, phosphorus oxychloride, thionyl chloride, phosgene, etc. The reaction temperature is not critical, but the reaction is usually conducted at room temperature to under cooling. The resulting reaction product is then reacted with an iminoetherifying agent including alcohols and metal alkoxides. The alcohols include alkanols such as methanol, ethanol, propanol, isopropanol, n-butanol, tert-butanol, etc. as well as those compounds in which the alkyl moieties of the above-mentioned alcohols are substituted with an alkoxy group such as methoxy, ethoxy, propoxy, isopropoxy, butoxy or the like. The metal alkoxides include alkali metal alkoxides such as sodium alkoxides, potassium alkoxides, etc. and alkaline earth alkoxides such as calcium alkoxides, barium alkoxides, etc. derived from such alcohols as above.

In the case, for example, where the protective group is a residue of an organic carboxylic acid and a substituent such as free amino, hydroxyl, mercapto, carboxyl or sulfa group is attached to the carbon atom adjacent to the carbonyl of the carboxylic residue, it is advantageous to carry out a preliminary treatment for enhancing the adjacent group effect of such substituent to increase the reactivity of the carbonyl group prior to the deprotecting reaction. This will be illustrated, for example, with the case where the substituent on the carbon adjacent to the carbonyl is a free amino group. In such a case, the protective group can be removed by transforming the amino group into a thioureido group followed by deacylation, or by application of any other known method for cleaving a peptide linkage. The temperature at which the reaction is carried out is not critical and is selected depending on the particular protective group and deprotecting method, although it is preferred to carry out the reaction under mild conditions such as under cooling to under warming.

In the above-mentioned reactions, when R and/or $R^1$ is a carboxyl-containing group, a derivative at the carboxyl group may be converted into the corresponding free carboxyl compound, and this invention is naturally intended to include such case.

The deprotected compound (I) thus obtained may be converted into a desired salt in a conventional manner as mentioned above.

Compound (I), containing a sulfa group, generally can react with a base to form salts. Thus, compound (I) may be obtained as a salt, and the salt thus obtained may further be converted into the free form or into another salt. Compound (I) obtained in the free form may be converted into its salt. Compound (I) in the form of a salt can be converted into the free form, for example, using an acid. As the acid, for example, an inorganic acid such as hydrochloric acid, sulfuric acid or phosphoric acid, or an organic acid such as formic acid, acetic acid or p-toluenesulfonic acid is advantageously employed depending on the particular salt and other conditions. Alternatively, an acidic ion-exchange resin or the like may be used. As a solvent, a hydrophilic organic solvent such as acetone, tetrahydrofuran, methanol, ethanol, dioxane or the like, water or a mixed solvent is generally employed. The reaction is usually carried out at room temperature, but it may be carried out under cooling or under warming. The reaction time depends on the particular acid and solvent employed and the reaction temperature selected, and it is generally preferred that the reaction be completed in a short period. The resulting compound (I) in the free form can be isolated in a known manner as described above.

The starting compound (II) used in this invention can be prepared, for example, by the following processes or analogous processes.

Process 1)

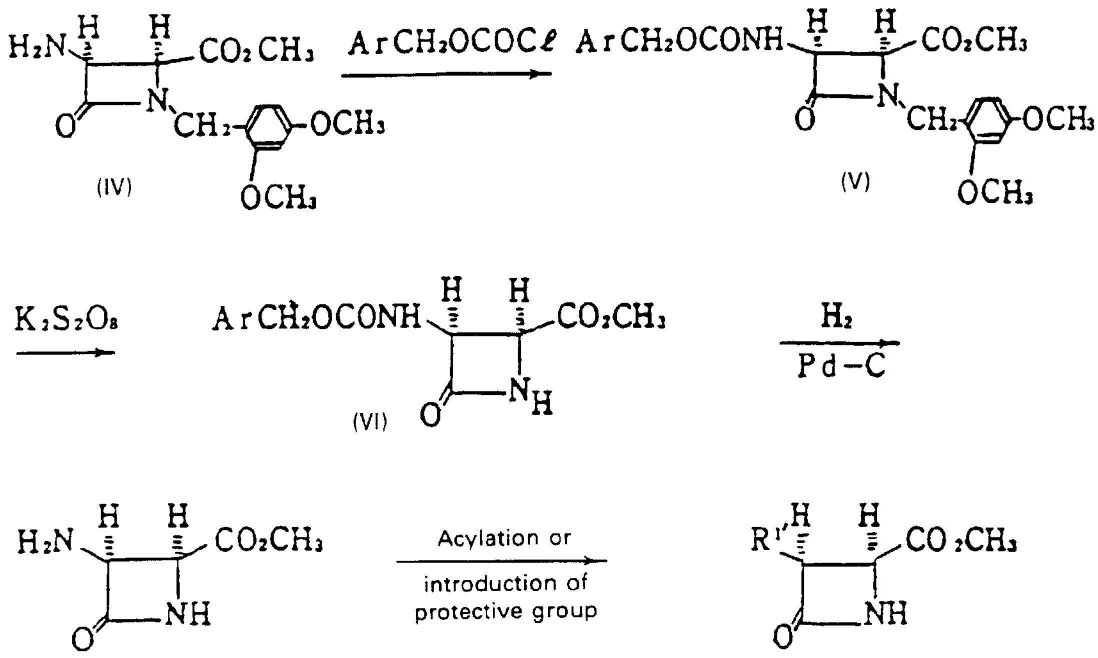


(IV) (V) (VI) (VII) (VIII)

Process 2)

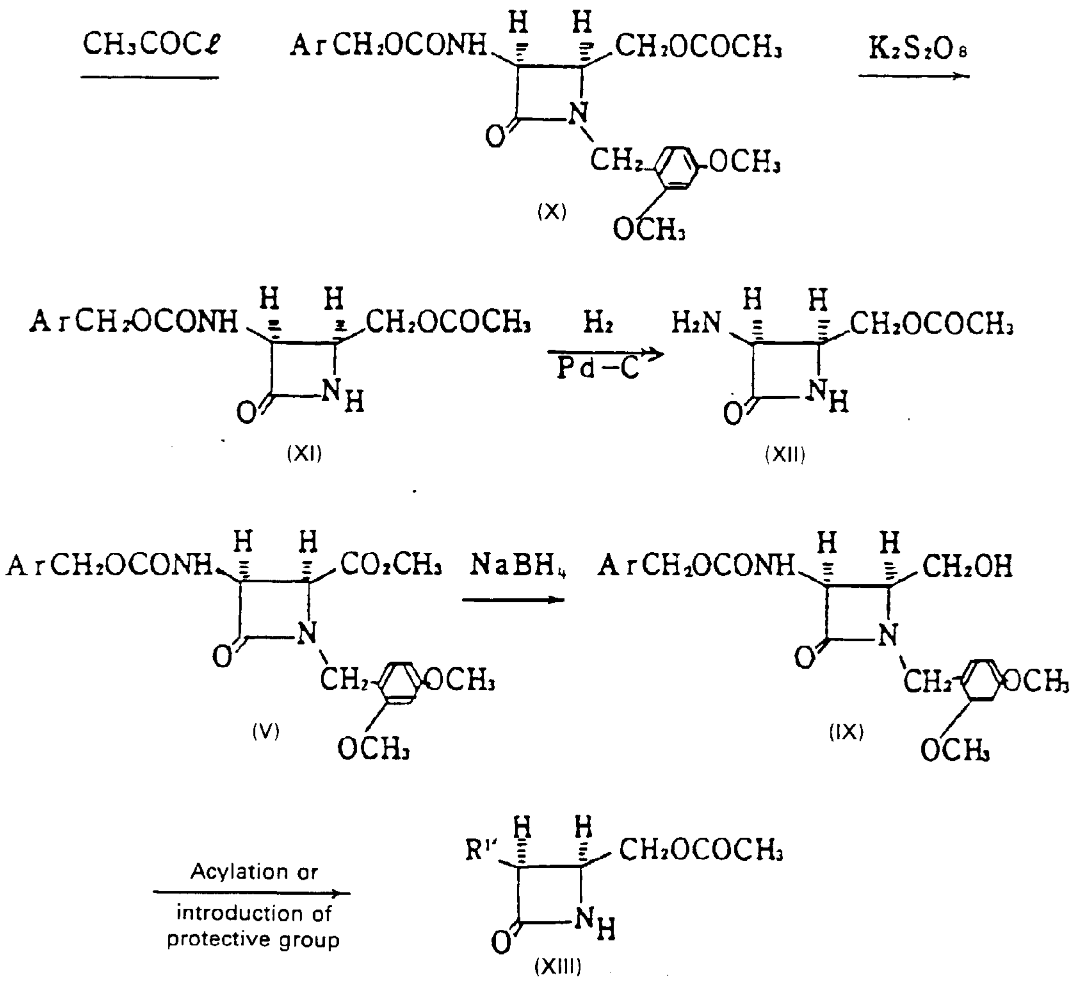


(X) (XI) (XII) (V) (IX) (XIII)

Process 3)

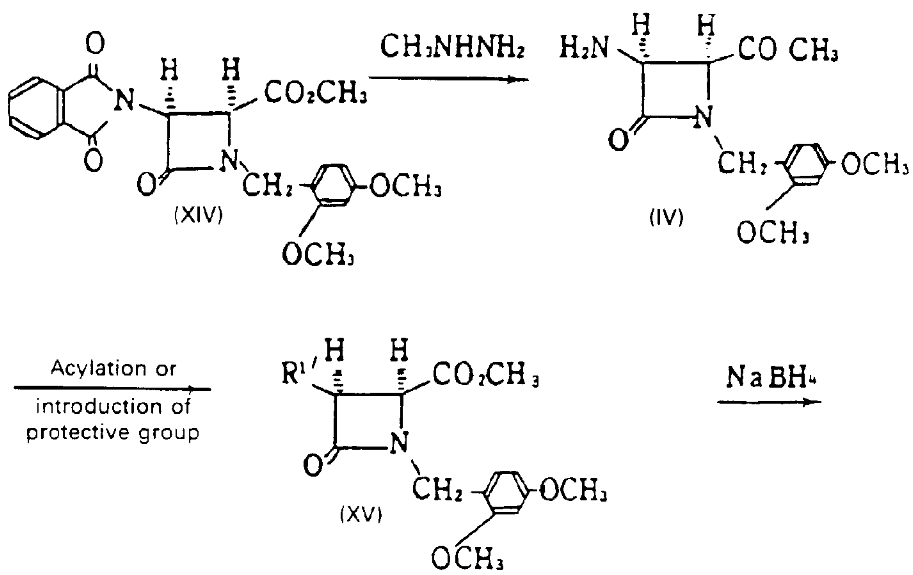


(XIV) (IV) (XV)

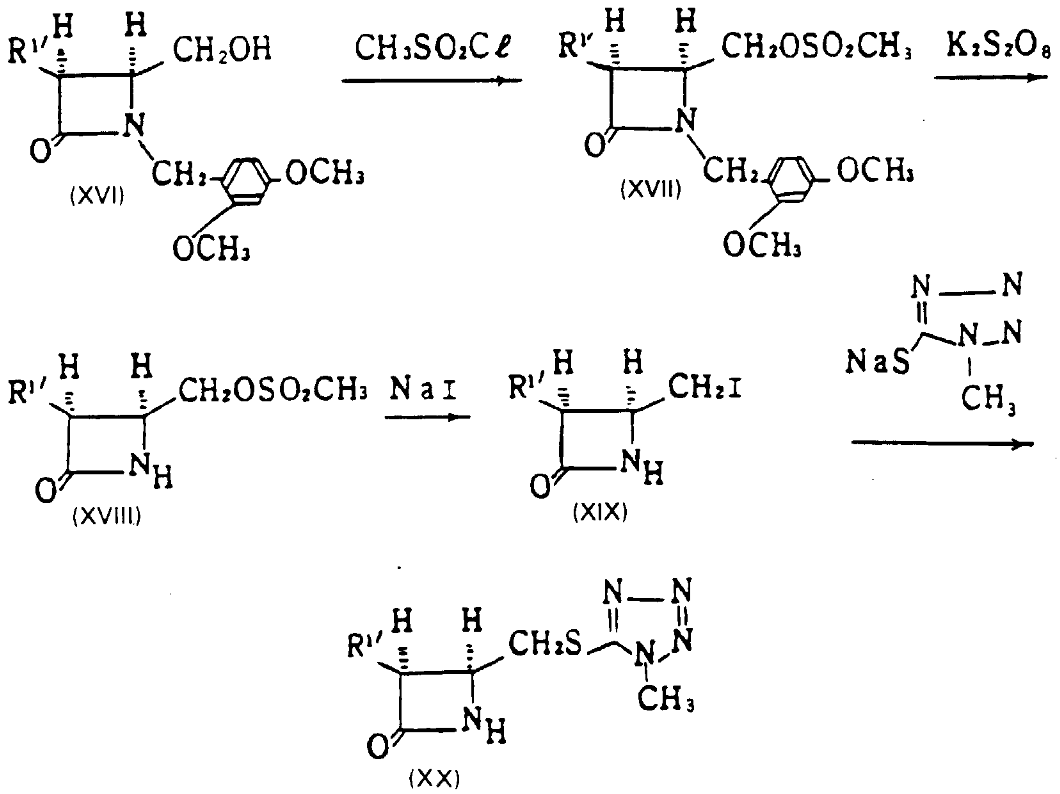


(XVI) (XVII) (XVIII) (XIX) (XX)

Process 4)

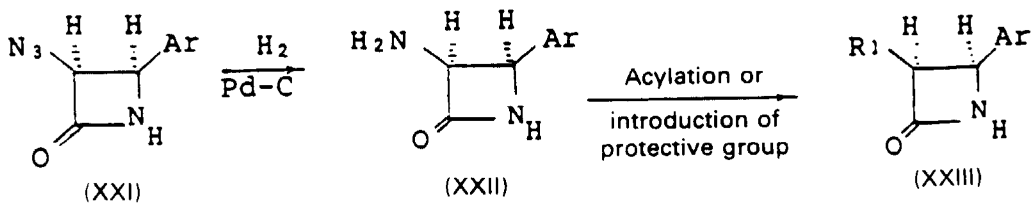


(XXI) (XXII) (XXIII)

Process 5)

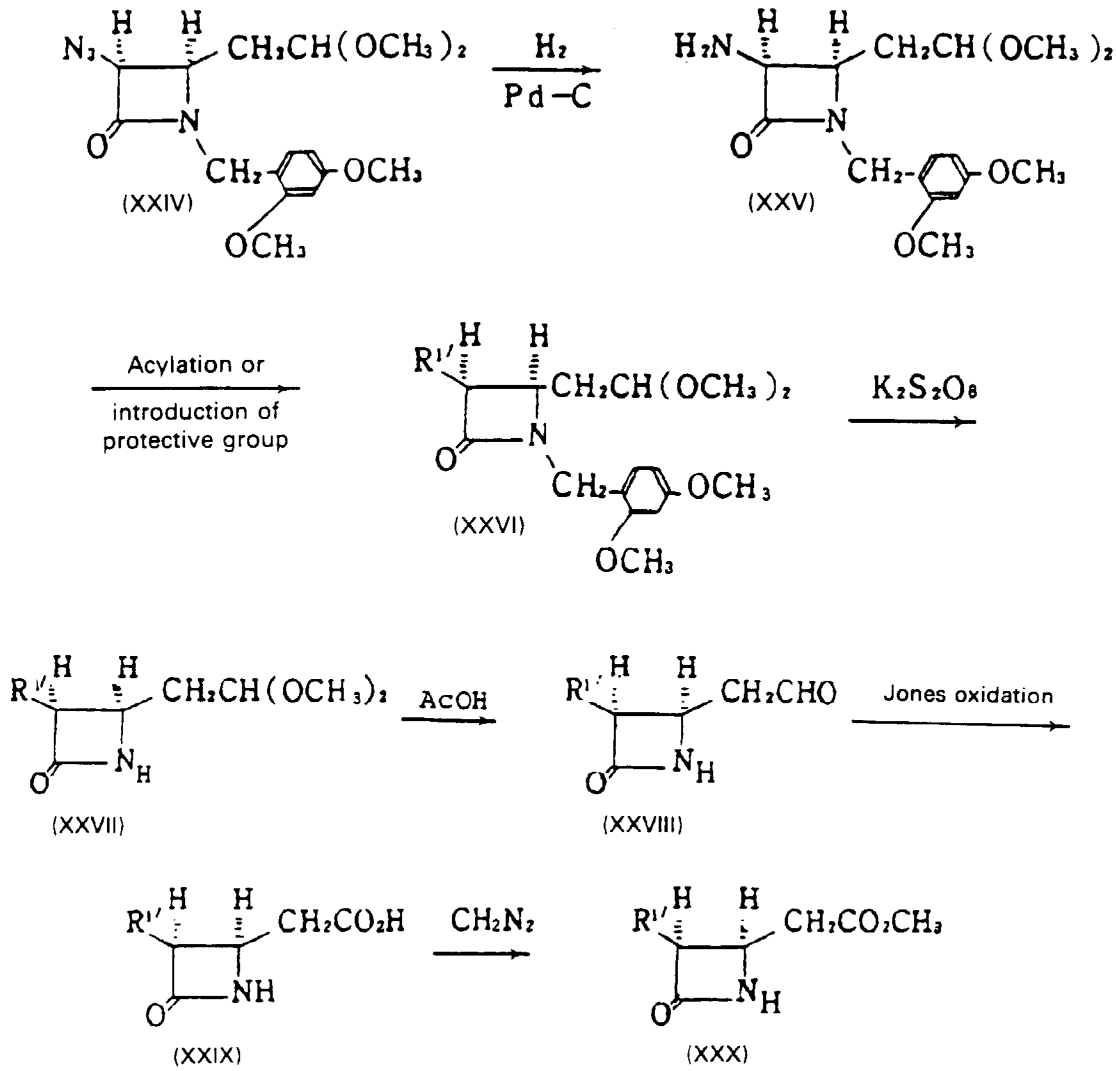


(XXIV) (XXV) (XXVI) (XXVII) (XXVIII) (XXIX) (XXX)

Process 6)

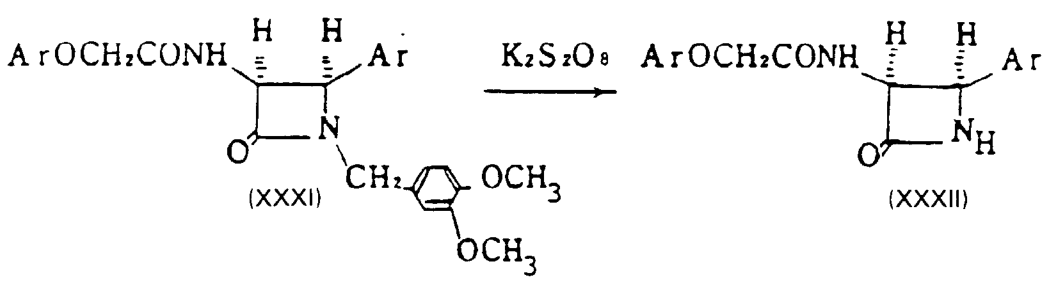


(XXXI) (XXXII)

Process 7)

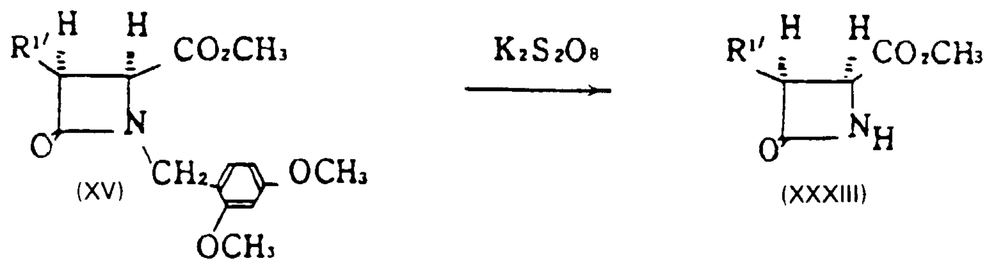


(XV) (XXXIII)

Process 8)

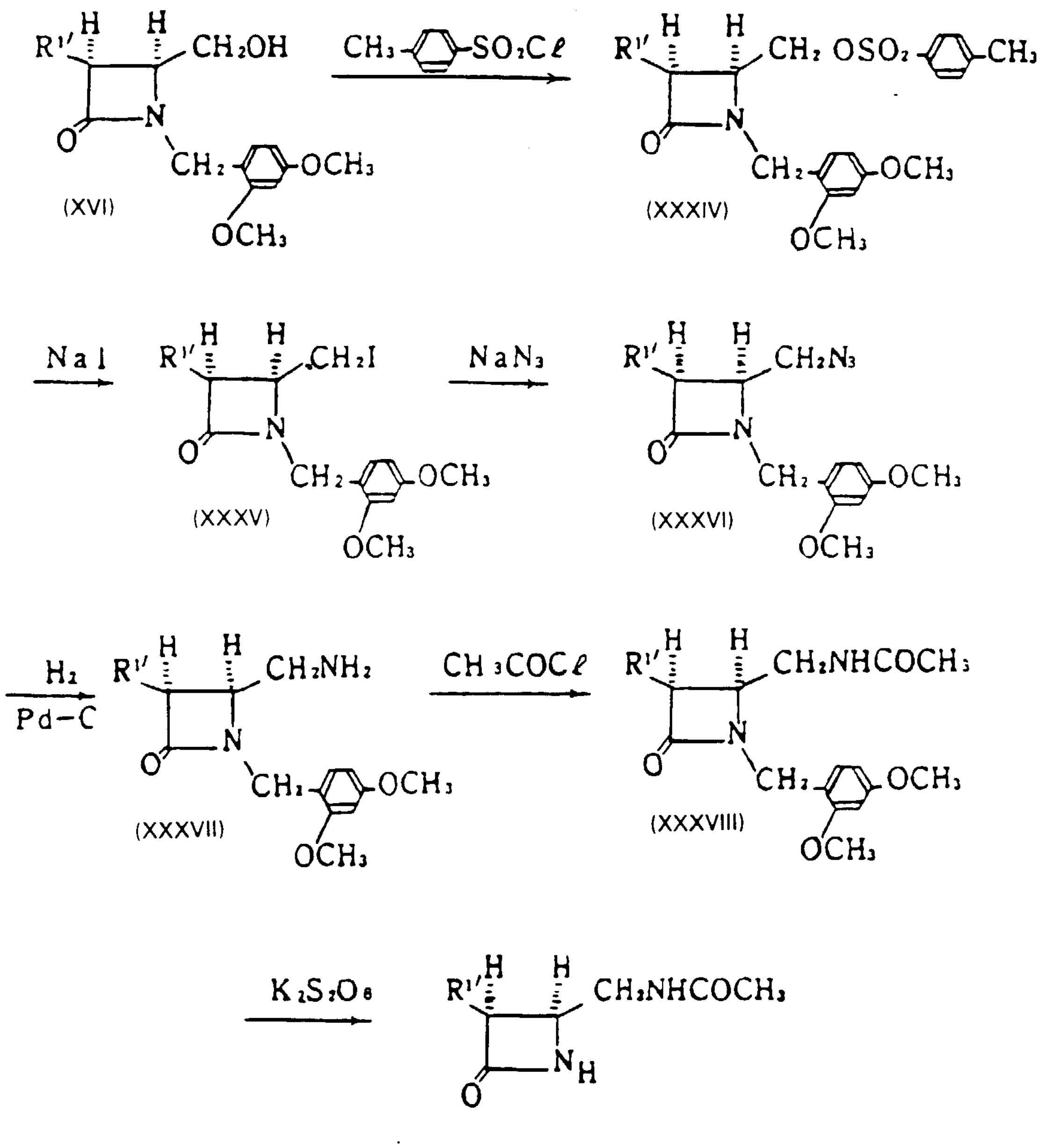


(XVI) (XXXIV) (XXXV) (XXXVI) (XXXVII) (XXXVIII) (XXXIX)

Process 9)

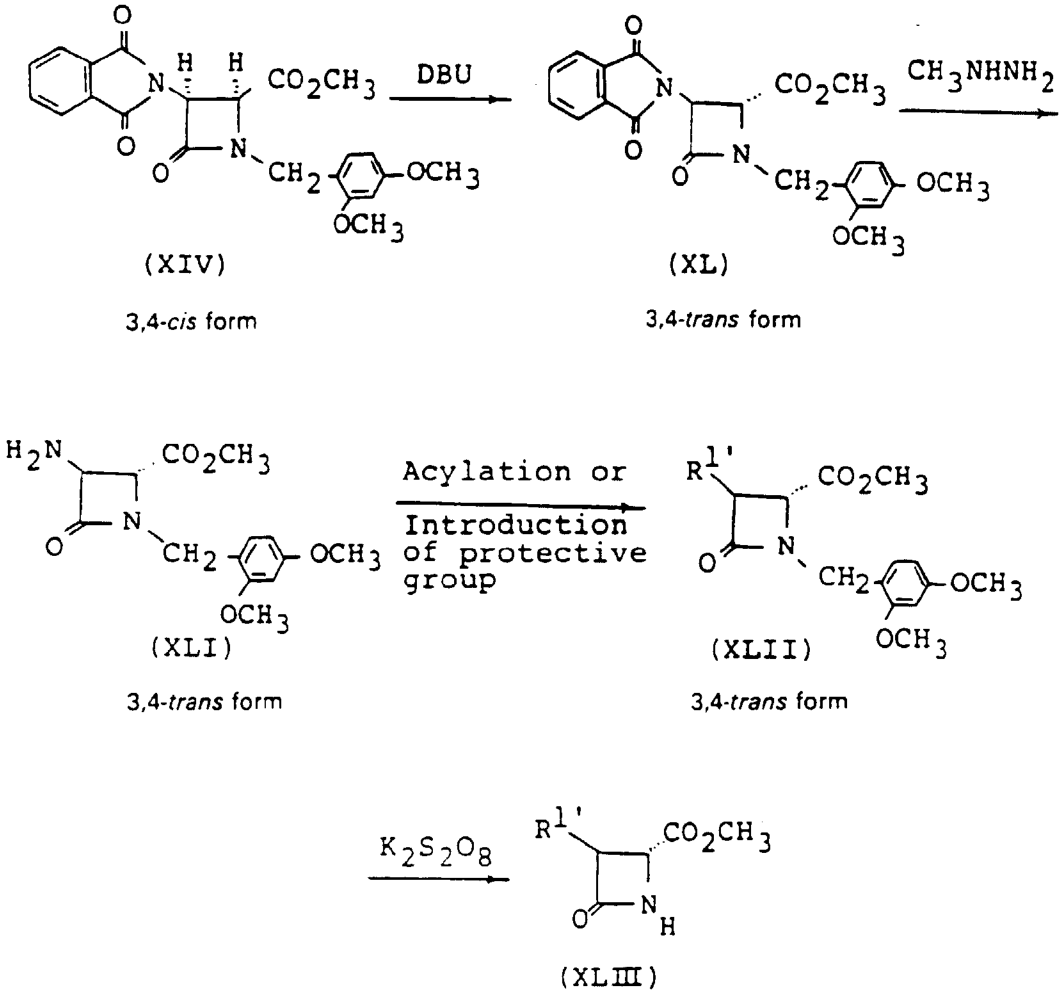


(XIV) 3,4-*cis* form

(XL) 3,4-*trans* form

(XLI) 3,4-*trans* form

(XLII) 3,4-*trans* form

(XLIII) 3,4-*trans* form

Process 10)

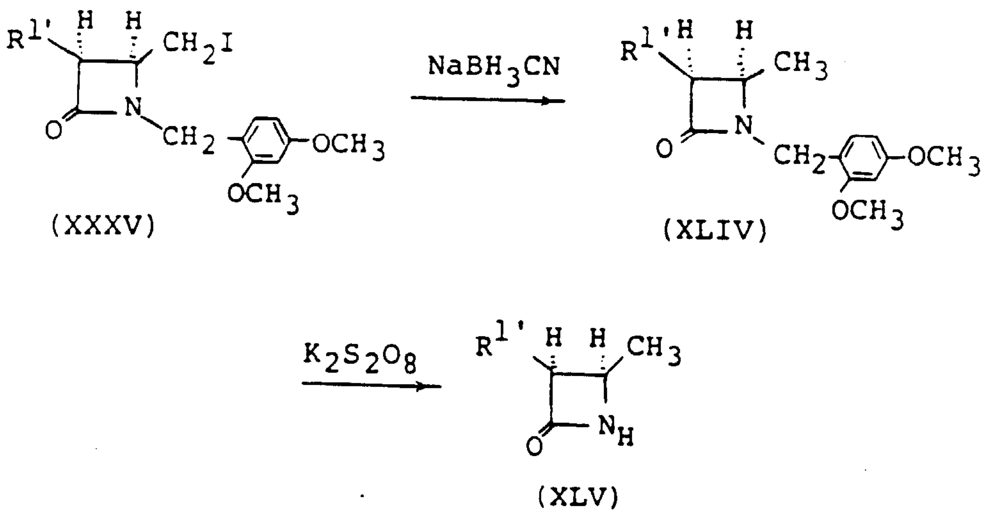


(XXXV)

(XLIV)

(XLV)

Process 11)

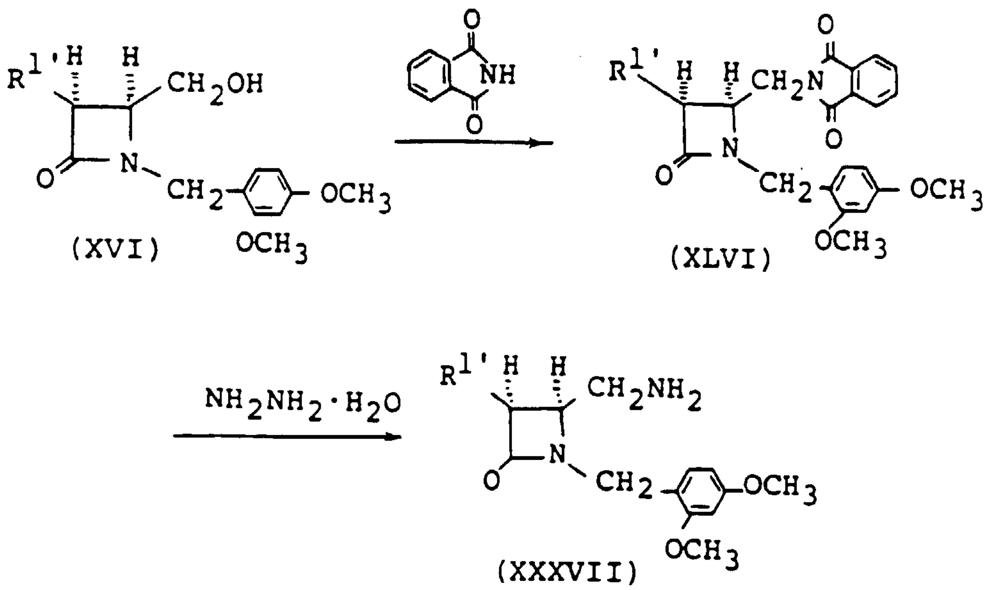


(XVI) (XLVI) (XXXVII)

Process 12)

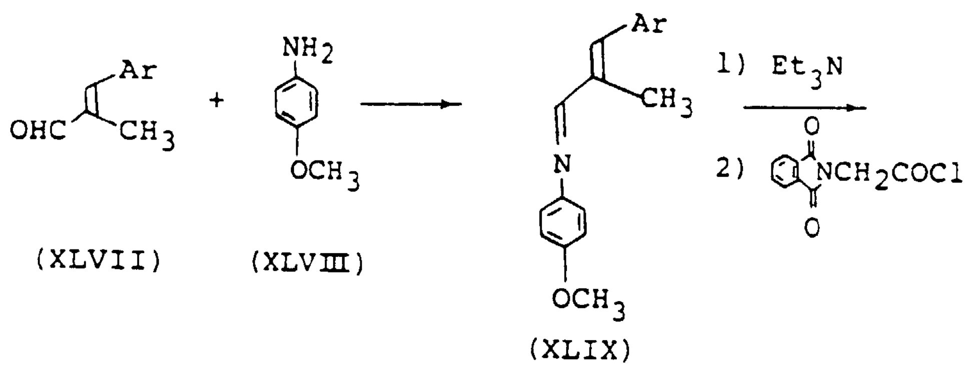


(XLVII) (XLVIII) (XLIX)

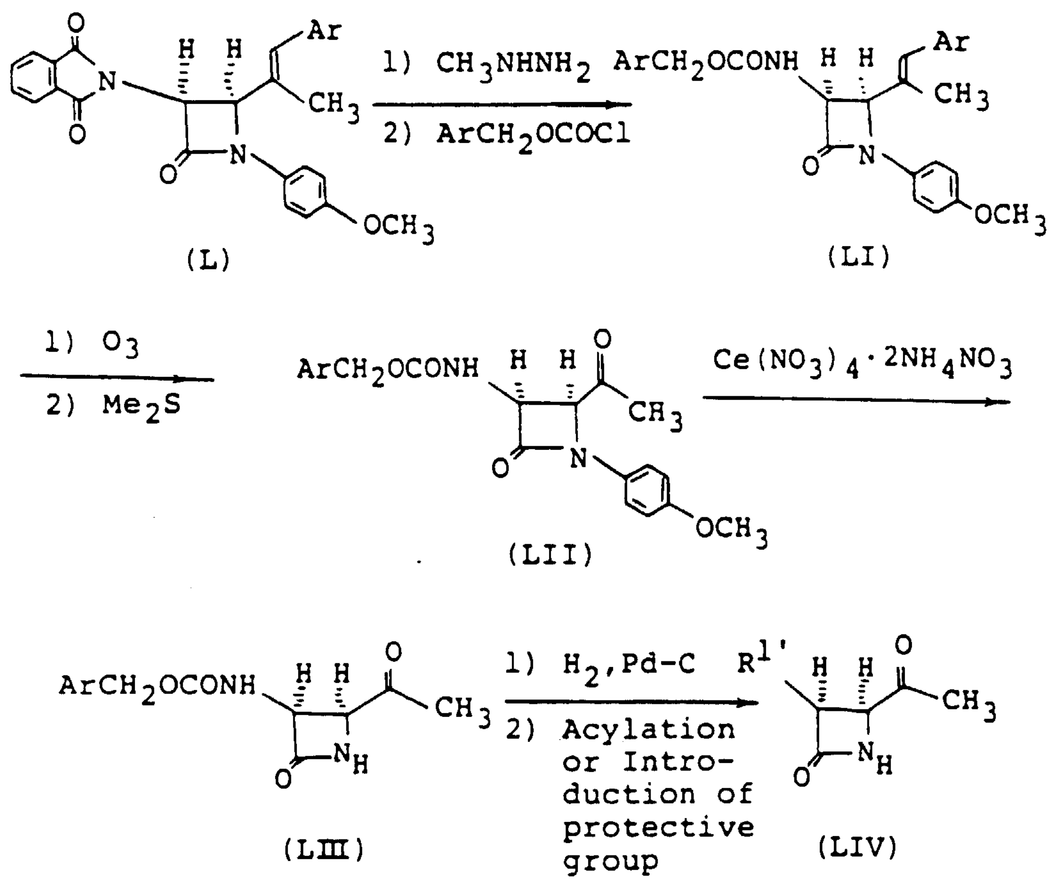


(L) (LI) (LII) (LIII) (LIV)

Process 13)

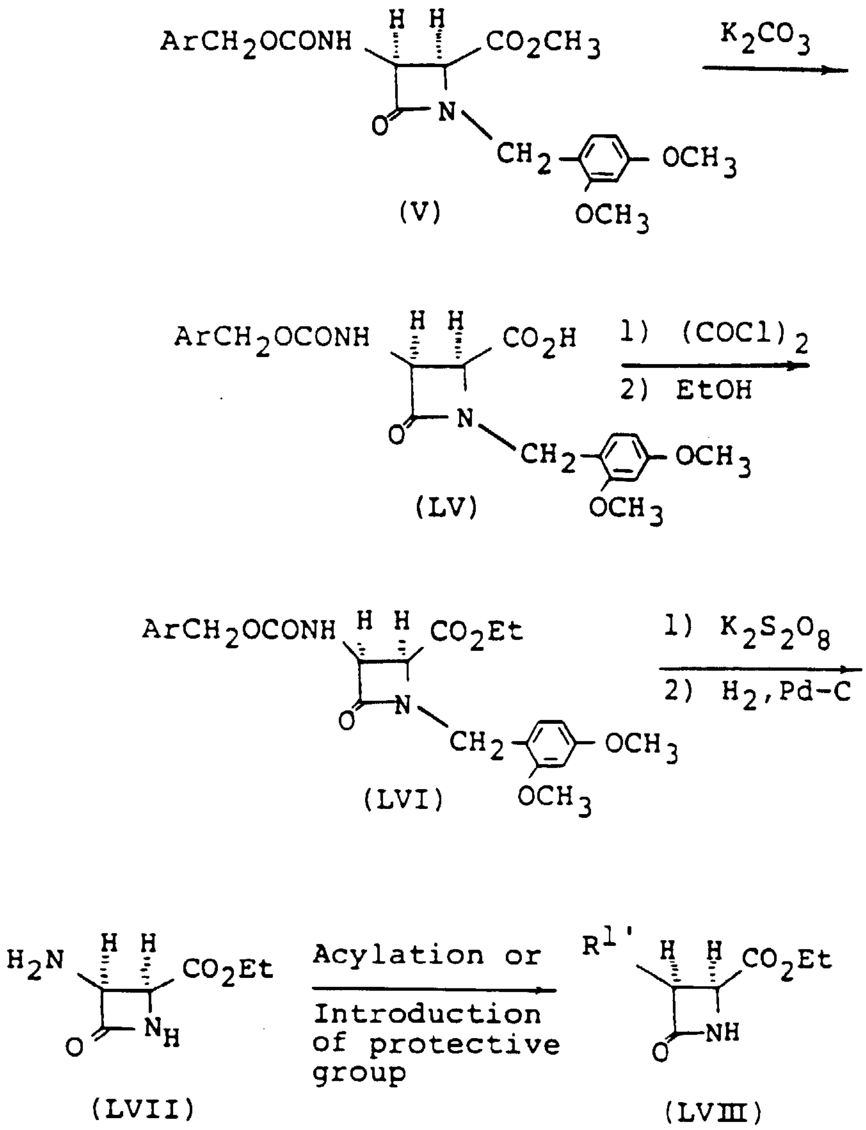


(V) (LV) (LVI) (LVII) (LVIII)

Process 14)

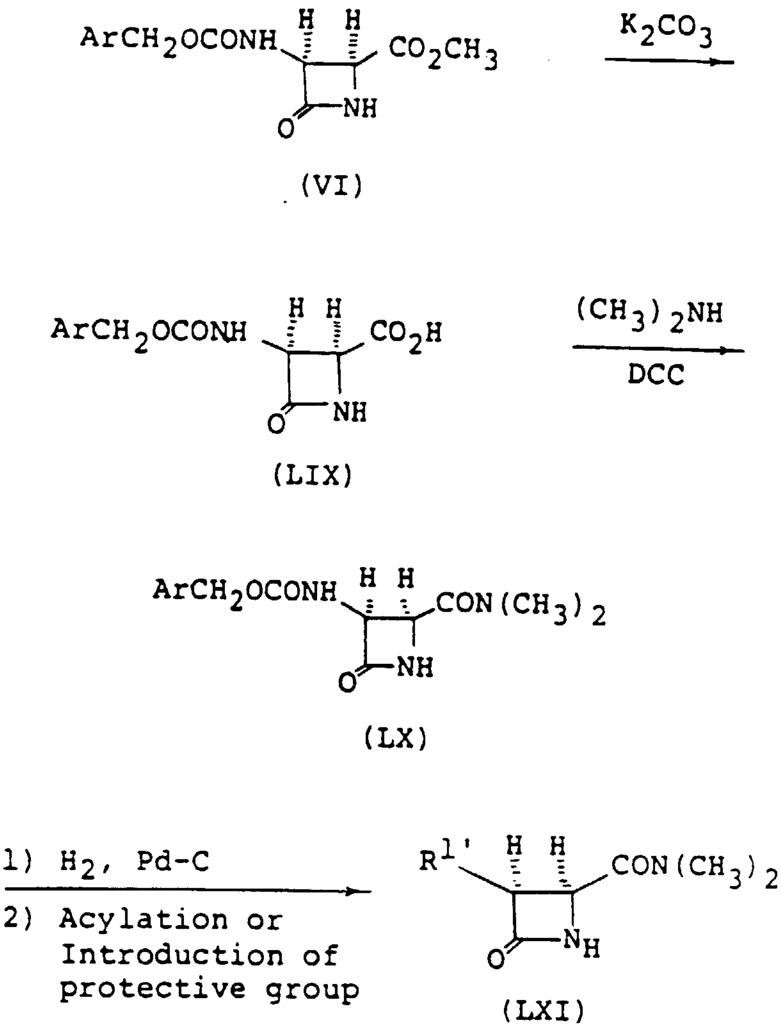


(VI)

(LIX)

(LX)

(LXI)

Process 15)

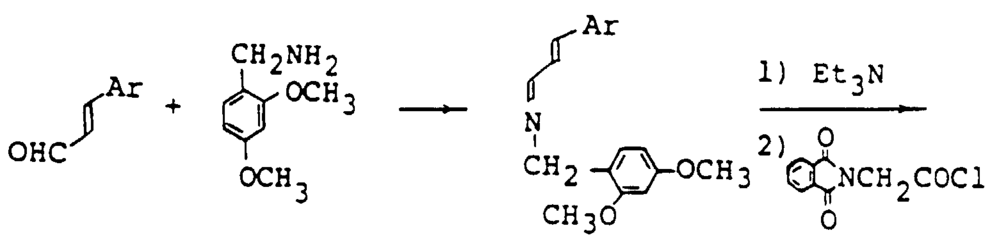


(LXII) (LXIII) (LXIV)

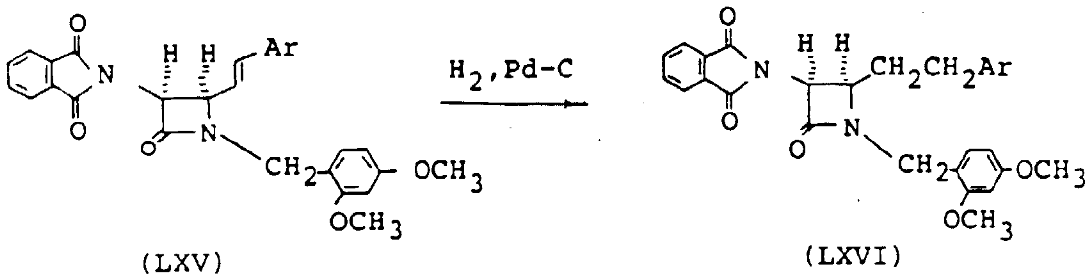


(LXV) (LXVI)

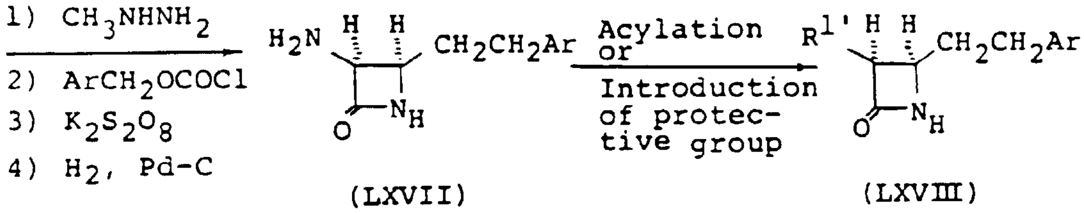


(LXVII) (LXVIII)

Process 16)

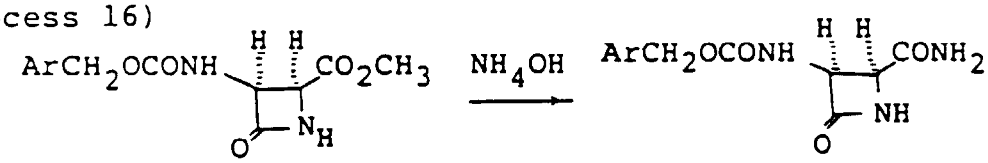


(VI) (LXIX)

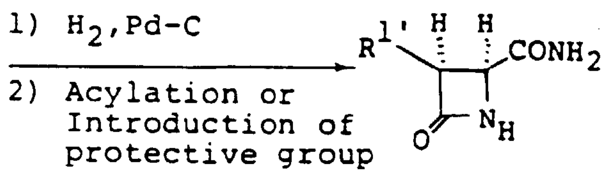


(LXX)

Process 17)

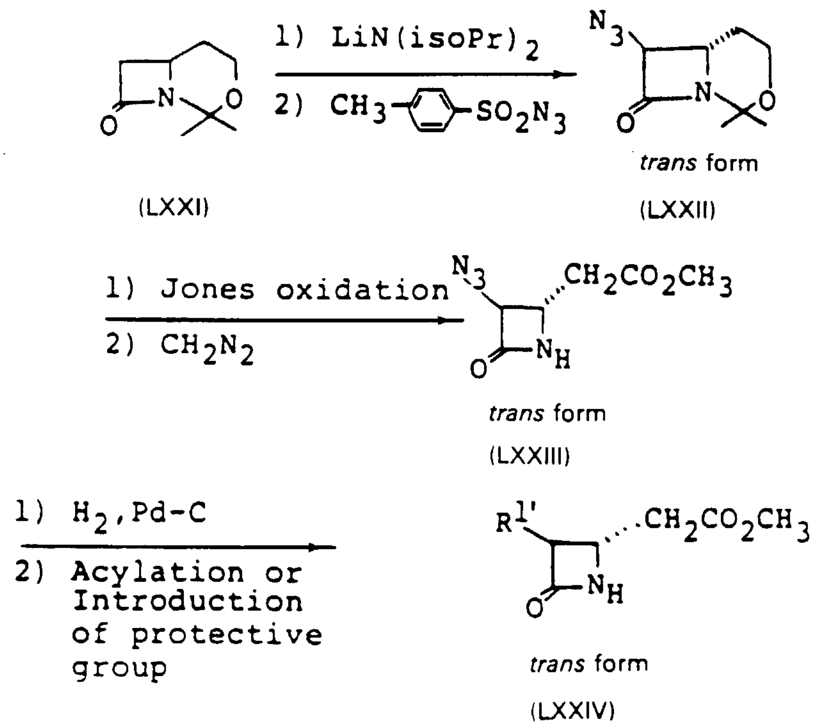


(LXXI)

*trans* form

(LXXII)

*trans* form

(LXXIII)

*trans* form

(LXXIV)

In the above formulas, Ac is acetyl, Et is ethyl, Me is methyl, iso-Pr is isopropyl, Ar is aryl, $R^{1'}$ is an acylated or protected amino group, DBU is 1,3-diazabicyclo[5,4,0]-7-undecene, and DCC is dicyclohexylcarbodiimide.

The starting materials used in the above processes 1) to 17) include, for example, *cis*-3-amino-1-(2,4-dimethoxybenzyl)-2-oxoazetidine-4-carboxylic acid methyl ester (IV) described in J. Am. Chem. Soc., *99*, 2352 (1977), *cis*-1-(2,4-dimethoxybenzyl)-3-phthalimido-2-oxoazetidine-4-carboxylic acid methyl ester (XIV) described in Japanese Patent Laid-Open Publication No. 136694/1976, *cis*-3-azido-4-phenyl-2-oxoazetidine (XXI) described in J. Org. Chem., *34*, 1477 (1969), *cis*-3-azido-1-(2,4-dimethoxybenzyl)-4-2,2-dimethoxyethyl)-2-oxoazetidine (XXIV) described in J. Am. Chem. Soc., *101,* 4730 (1979), *cis*-1-(3,4-dimethoxybenzyl)-3-phenoxyacetamido-4-phenyl-2-oxoazetidine (XXXI) described in Synthesis (1979), 543 and 2,2-dimethyl-1-aza-3-oxabicyclo[4,2,0]octan-8-one (LXXI) described in J. Am. Chem. Soc., *100,* 313 (1978). These examples are not intended to restrict the starting materials thereto, and any compound meeting the purpose of this invention can be used. Examples of such starting materials will be given in the following reference examples.

The compounds of the formula (I), wherein $R^1$ is an amino group which may optionally be protected and R is the group [A] wherein $R^4$ is carbamoyloxy group, are representable by the formula

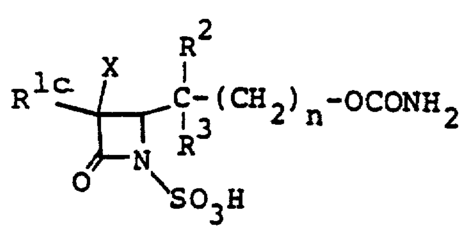

$(I^n)$

wherein $R^{1c}$ is an amino group which may optionally be protected, and $R^2$, $R^3$ and n are as defined above. The compound $(I^n)$, or a salt or ester thereof can also be used advantageously as the starting material for the production of other compounds (I) wherein R is a group of the formula

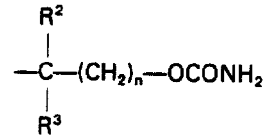

wherein all symbols are as defined above, or a salt or ester thereof. That is, the compound ($I^n$) wherein $R^{1c}$ is amino group, which may be produced by removing the protective group of the compound ($I^n$) wherein $R^{1c}$ is a protected amino group, can be used as a starting compound (III) wherein R is the group

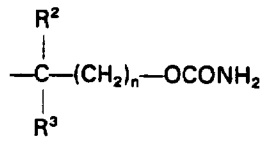

(all symbols are as defined above). The compound ($I^n$), or a salt or ester thereof can be produced by sulfonating a compound of the formula

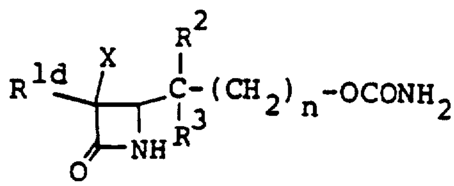

($II^a$)

wherein $R^{1d}$ is an amino group which may optionally be protected and $R^2$, $R^3$ and n are as defined above, or a salt or ester thereof, and if necessary, removing the protective group. The sulfonation reaction can be conducted in the same manner as the sulfonation reaction of the compound (II), and the removal of the protective group can be accomplished by the conventional methods as mentioned above.

In the above formulas ($I^n$) and ($II^a$), the protective group for the amino groups in $R^{1c}$ and $R^{1d}$ includes those exemplified hereinbefore for the group $R^1$. A preferable one of the group

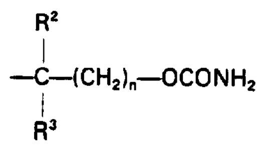

is a group of the formula $-(CH_2)_{n^a}-OCONH_2$
wherein $n^a$ is as defined below. Among them, the group wherein $n^a$ is 1 is more preferably employed as the 4-substituent of ($I^n$) and ($II^a$).

In particular, this invention relates to, among the above-mentioned compounds (I), 1-sulfo-2-oxoazetidine derivatives of formula ($I^a$)

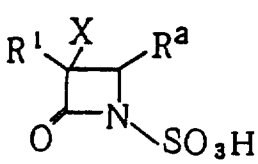

($I^a$)

wherein $R^a$ is $-COQ^0$ ($Q^0$ is an amino group which may optionally be protected or substituted), $-COQ^5$ ($Q^5$ is a hydroxy group which may optionally be protected), $-(CH_2)_{n^a}-R^{4a}$ ($n^a$ is an integer of 1 to 3; $R^{4a}$ is a hydrogen atom, halogen

atom or a carbamoylamino, N-sulfocarbamoylamino, carbamoyl, carbamoyloxy, N-sulfocarbamoyloxy, haloalkylcarbonylcarbamoyloxy, alkylsulfonyloxy, pyridinio, alkoxy, alkylsulfinyl, alkylsulfonyl, haloalkylcarbonyloxy, hydroxy, alkoxycarbonyl, acyloxy, 1-alkoxyiminoalkyl, alkylcarbonyl or acylamino group) or a nitrogen-containing heterocyclic group; $R^1$ is an amino group which may optionally be acylated or protected; X is a hydrogen atom or a methoxy group, or a salt or ester thereof and, more particularly, to compounds of the formula

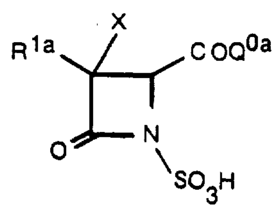

($I^b$)

wherein $Q^{0a}$ is a hydrazino, carbamoylhydrazino, lower alkoxycarbonylhydrazino, or carbamoyl($C_{1-4}$)alkylamino group; $R^{1a}$ is an esterified carboxylamino group or a group of the formula

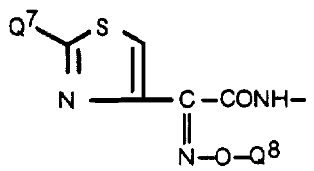

(all symbols are as defined above); and X is a hydrogen atom or a methoxy group, compounds of the formula

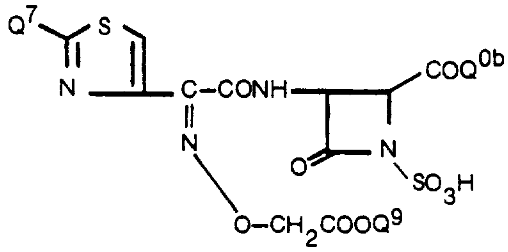

($I^c$)

wherein $Q^{0b}$ is a lower alkylamino group; $Q^7$ and $COOQ^9$ are as defined above, compounds of the formula

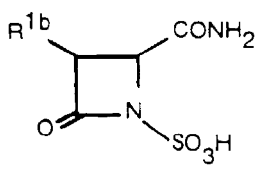

($I^d$)

wherein $R^{1b}$ is D-2-(6,7-dihydroxychromone-3-carboxamido)-2-(4-hydroxyphenyl)acetamido or a group of the formula

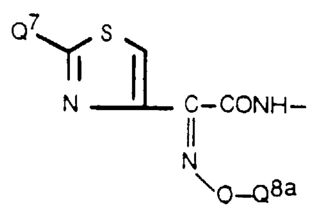

($Q^7$ is as defined above; $Q^{8a}$ is a lower alkyl group), compounds of the formula

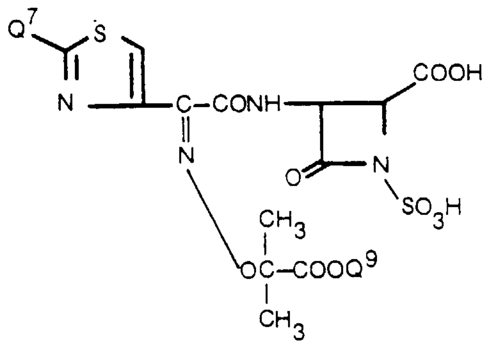

($I^e$)

wherein all symbols are as defined above, compounds of the formula

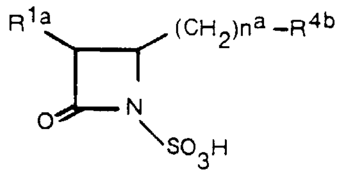

($I^f$)

wherein $R^{1a}$ and $n^a$ are as defined above; $R^{4b}$ is a carbamoylamino, (N-sulfocarbamoyl)amino, carbamoyl, pyridinio, lower alkoxy, lower alkylsulfinyl, lower alkylsulfonyl, 1-lower alkoxyimino-lower alkyl, lower alkylcarbonyl or formylamino group, compounds of the formula

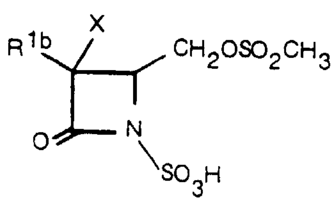

($I^g$)

wherein $R^{1b}$ is an esterified carboxylamino group; X is a hydrogen atom or a methoxy group, compounds of the formula

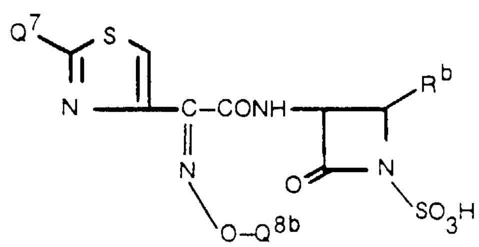

($I^h$)

wherein $Q^7$ is as defined above; $Q^{8b}$ is $-CH_2COOQ^9$ or

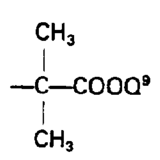

and $COOQ^9$ is as defined above); $R^b$ is a monochloroacetoxymethyl or hydroxymethyl group, compounds of the formula

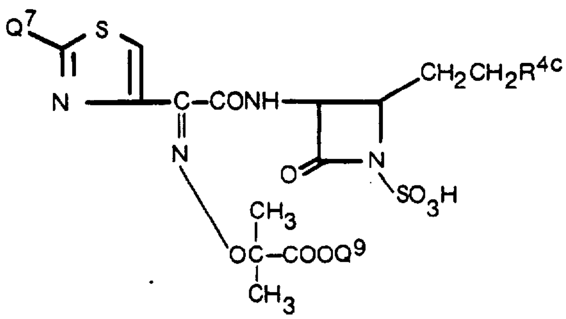

$(I^i)$

wherein $Q^7$ and $COOQ^9$ are as defined above; $R^{4c}$ is a lower alkoxycarbonyl group or a $C_{2-4}$ acyloxy group, compounds of the formula

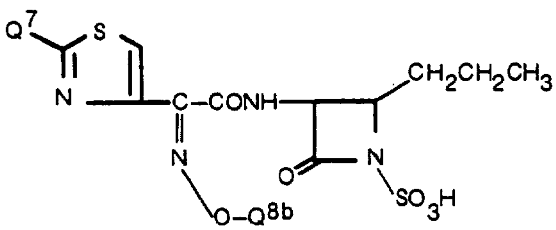

$(I^j)$

wherein all symbols are as defined above, compounds of the formula

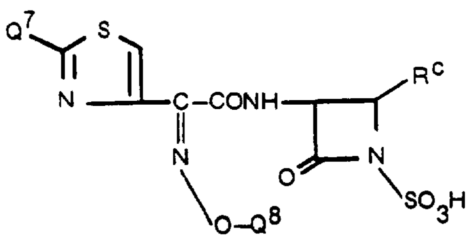

$(I^k)$

wherein $Q^7$ and $Q^8$ are as defined above; $R^c$ is a sulfaminocarbonyloxymethyl group or a haloalkylcarbonylcarbamoyloxymethyl group, compounds of the formula

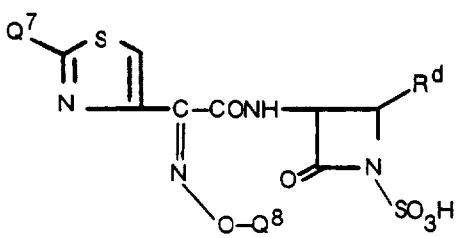

$(I^l)$

wherein $Q^7$ and $Q^8$ are as defined above; $R^d$ is a nitrogen-containing heterocyclic group which may optionally be substituted, and compounds of the formula

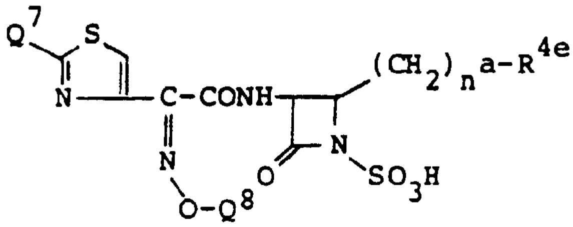

$(I^{o})$

wherein $Q^7$ and $Q^8$ are as defined above and $R^{4e}$ is a halogen atom, for instance. Preferred are compounds of the formula

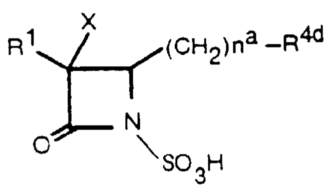

$(I^{m})$

wherein $R^1$, X and $n^a$ are as defined above; $R^{4d}$ is a lower alkoxy group, and particularly desirable are compounds of the same formula wherein $R^1$ is a group of the formula

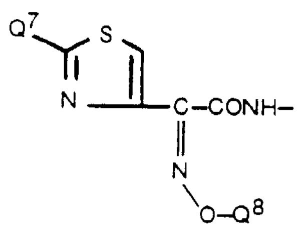

wherein all symbols are as defined above.

In the above formulas $(I^b)$, $(I^c)$, $(I^d)$, $(I^e)$, $(I^f)$, $(I^g)$, $(I^h)$, $(I^i)$, $(I^j)$, $(I^k)$, $(I^l)$ and $(I^o)$, the esterified carboxylamino group represented by $R^{1a}$ or $R^{1b}$ includes as a preferable one a $C_{1-4}$ alkoxycarbonylamino and substituted or unsubstituted benzyloxycarbonylamino group, the substituent being nitro or $C_{1-4}$ alkoxy. The protective group of the protected amino represented by $Q^7$ includes as a preferable one a halogeno-$C_{2-4}$ acyloxy group. The esterified carboxyl group represented by $COOQ^9$ includes as a preferable one a substituted or unsubstituted benzyloxycarbonyl group, the substituent being nitro or $C_{1-4}$ alkoxy. A preferable one of "the nitrogen-containing heterocyclic group which may optionally be substituted" represented by $R^d$ is a substituted or unsubstituted 5-membered heterocyclic group including two nitrogen atoms or nitrogen and oxygen atoms as hetero atoms, the substituent being $C_{1-4}$ alkyl or $C_{2-4}$ acyl group. Among the group represented by $R^a$, a group of the formula $-(CH_2)_na-R^{4a}$ (especially, $R^{4a}$ being carbamoyloxy and $n^a$ is 1) is preferable.

The compound $(I^a)$, or a salt or ester thereof may be produced by sulfonating the compound of the formula

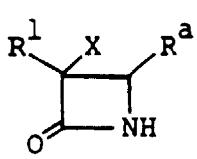

$(II^{b})$

wherein all symbols are as defined above, or a salt or ester thereof, and if necessary, removing the protective group, or by acylating a compound of the formula

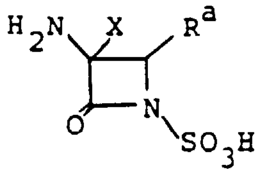

(III$^{a}$)

wherein all symbols are as defined above, or a salt or ester thereof, and if necessary, removing the protective group. The sulfonation and acylation reactions, and the removal of the protective group may be accomplished in the same manner as mentioned above. In the acylation reaction, as an acylating agent, use may be made of, for example, a compound of the formula

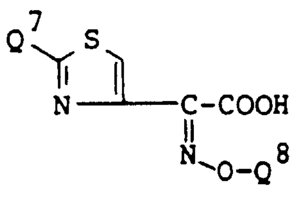 ,

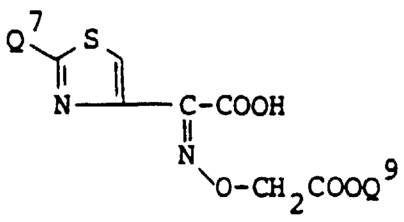 , .

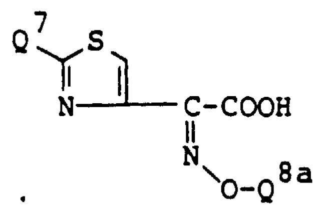 ,

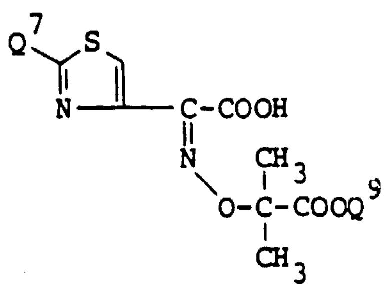 .

or

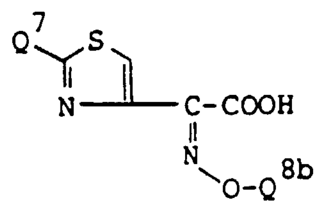

where all symbols are as defined above, or a reactive derivative thereof. The reactive derivative includes those as mentioned above in the acylation reaction of the compound (III), or as mentioned hereinafter in relation to a carboxylic acid [II]'.

The starting compounds for the intermediates (II) and (III) used in the synthesis of the objective compounds of (I$^{a}$) through (I$^{m}$), for instance, can be produced for example by the processes described in Japanese Patent Application No. 194311/1981, which are shown below, or processes analogous therewith, and the specific examples of production of the objective compounds and said intermediates (II) and (III) are described in the Reference Examples and the Examples, both of which appear hereinafter.

Process 1'

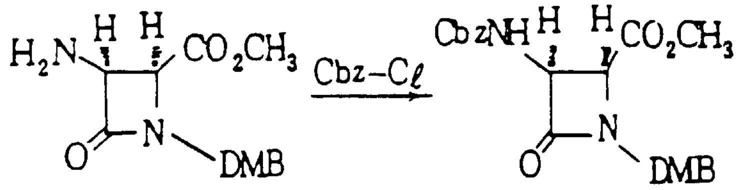


(IV) (V)

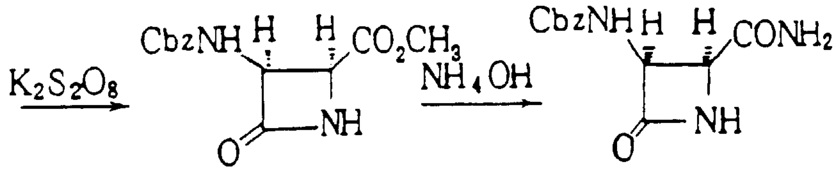


(VI) (VII)[a]

Process 2'

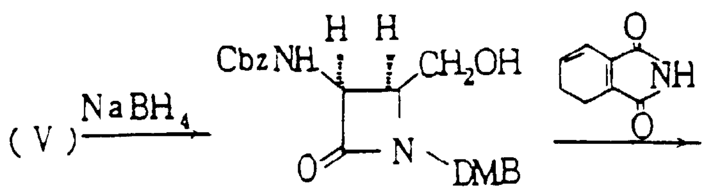


(VIII)[a]

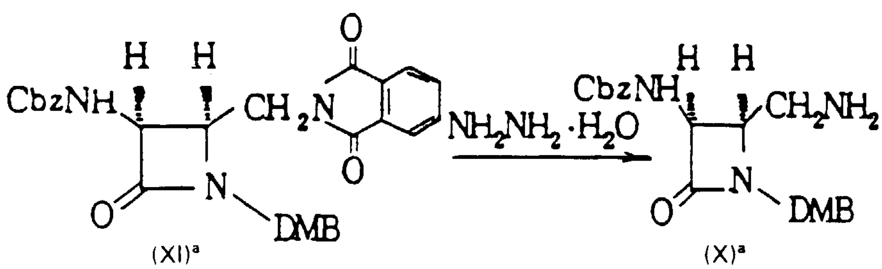


(XI)[a] (X)[a]

Process 3'

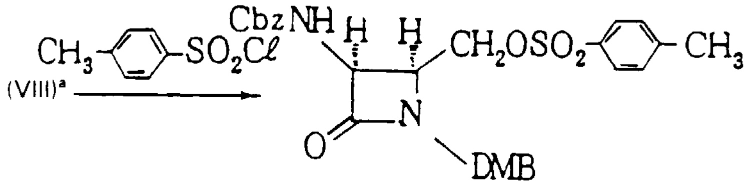


(XI)[a]

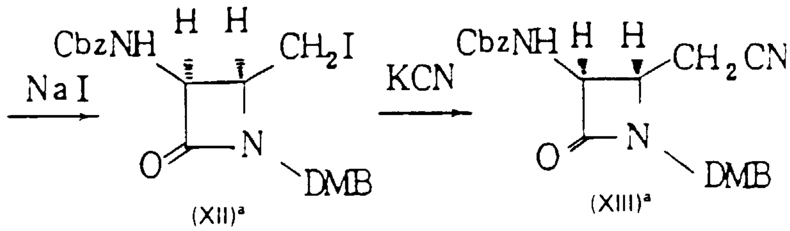


(XII)[a] (XIII)[a]

Process 4'

(VI)
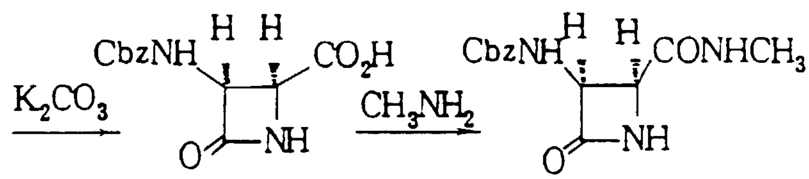


(XIV)[a] (XV)[a]

Process 5'

(VI)
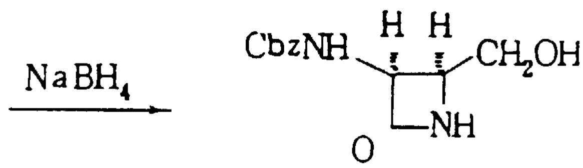


(XVI)[a]

Process 6'

(IV)
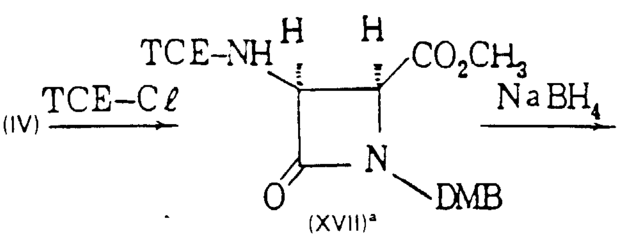


(XVII)[a]

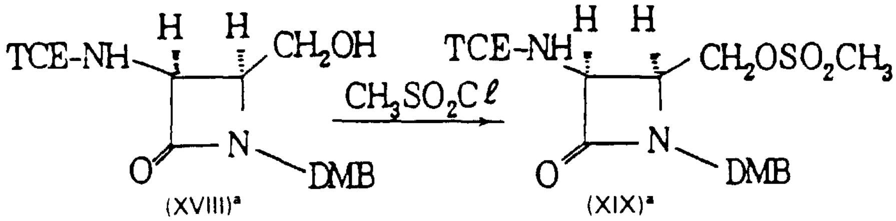


(XVIII)$^a$ (XIX)$^a$

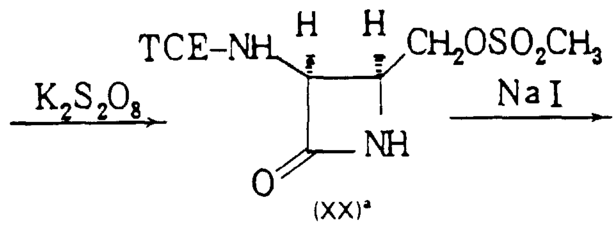


(XX)$^a$

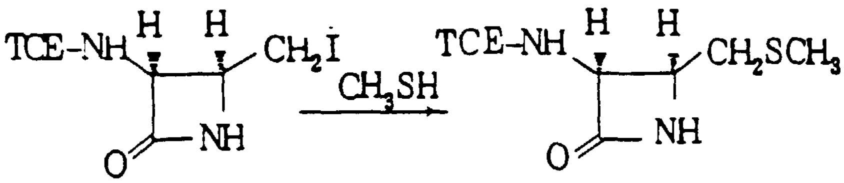


(XXI)$^a$ (XXII)$^a$

Process 7'

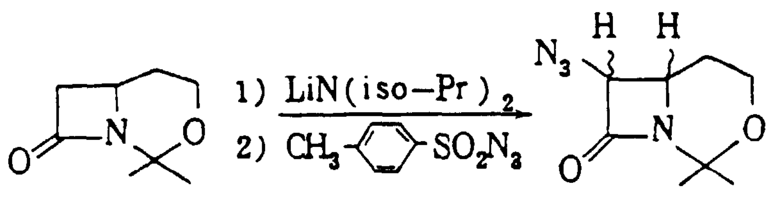


(XXIII)$^a$ (XXIV)$^a$

wherein DMB is a 2,4-dimethoxybenzyl group; Cbz is a benzyloxycarbonyl group; and TCE is a β,β,β-trichloroethoxycarbonyl group.

The starting compound (XXIII)$^a$ for the above process 7') is a known compound described in Journal of the American Chemical Society *100,* 313 (1978) and can be easily prepared. The above examples are not intended to delimit the starting materials for the present invention but any other compounds that are suited to the practice of the present invention can be successfully employed. By way of example, the compounds described in Japanese Patent Application No. 182148/1981 and No. 011965/1982 may also be employed with advantage. The acylating agent used in accordance with this invention can be prepared for example by the processes described in Japanese Patent Application No. 194311/1981 and No. 011965/1981 or European Journal of the Medical Chemistry *16,* 307 (1981), or by processes analogous thereto.

Throughout the specification, both the terms "2-oxo-azetidine" and "2-azetidinone" represent the same chemical structure.

In a particularly important aspect, the present inventors, as a result of their intensive research for the purpose of obtaining novel and useful 1-sulfo-2-azetidinone derivatives, have found that either sulfonation of a compound of the formula

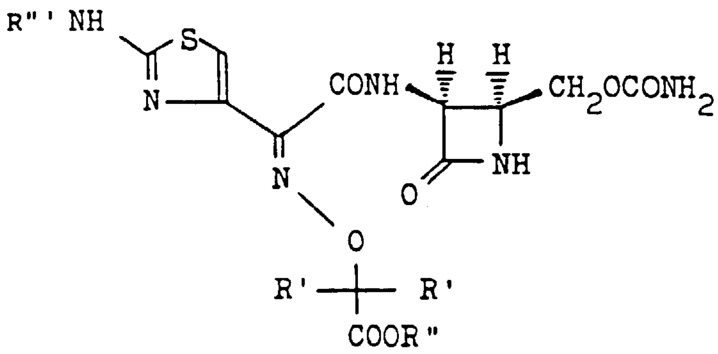

(I)'

wherein R' is a hydrogen atom or a lower alkyl group, R" is a hydrogen atom or an ester residue and R"' is an amino-protecting group, or a salt thereof, followed by removal of the protective group and, if necessary, the ester residue, or reaction of (3S,4S)-cis-3-amino-4-carbamoyloxymethyl-2-azetidinone-1-sulfonic acid (hereinafter called "compound [A']") or a salt or ester thereof with a carboxylic acid of the formula

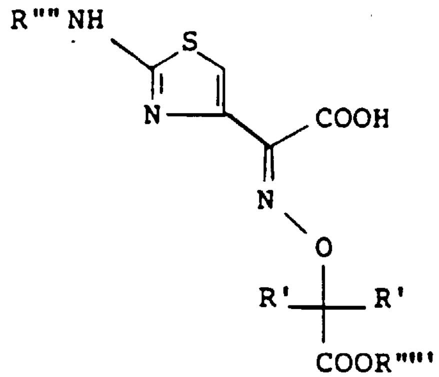

[II]'

wherein R' is as defined above, R"" is a hydrogen atom or an amino-protecting group and R""' is an ester residue, or a functional derivative thereof, followed by removal of the protective group and, if necessary, the ester residue gives a 1-sulfo-2-azetidinone derivative of the formula

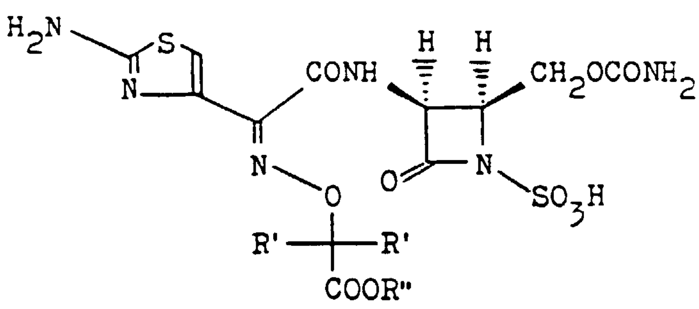

(III)'

wherein the symbols are as defined hereinabove, or a salt or ester thereof, which derivatives have the (3S,4S)-configuration and that the thus-obtained compound [III]' or a salt or ester thereof exhibit strong antibacterial activity against gram-negative bacteria including *Pseudomonas aeruginosa* and are very stable against β-lactamases produced by microorganisms and have a good distribution to body tissues. These findings have led to this aspect of the present invention.

Thus, this particular aspect of the invention provides:

(1) A compound [III]' or a salt or ester thereof;

(2) A method of producing compound (III)' or a salt or ester thereof which comprises sulfonating compound (I)' or a salt thereof and then removing the protective group and, if necessary, the ester residue; and

(3) A method of producing compound (III)' or a salt or ester thereof which comprises reacting compound (A') or a salt or ester thereof with carboxylic acid (II)' or a functional derivative thereof and then removing the protective group and, if necessary, the ester residue.

Referring to the above formulas [I]', [II]' and [III]', the lower alkyl group represented by R', which preferably contains 1-4 carbon atoms, is, for example, methyl, ethyl, n-propyl, n-butyl, isopropyl or isobutyl.

The ester residue represented by R" in the above formulas [I]' and [III]' or by R""' in formula [II]' includes those biologically active ester residues that are capable of increasing the blood concentration and the duration of efficacy, among others, such as, for example, α-($C_{1-4}$)alkoxy($C_{1-4}$)alkyl groups (e.g. methoxymethyl, ethoxymethyl, isopropoxymethyl, α-methoxyethyl, α-ethoxyethyl), ($C_{1-4}$)alkylthiomethyl groups (e.g. methylthiomethyl, ethylthiomethyl, isopropylthiomethyl), α-($C_{2-6}$)acyloxy($C_{1-4}$)alkyl groups (e.g. pivaloyloxymethyl, α-acetoxyethyl) and α-($C_{1-4}$)alkoxycarbonyloxy($C_{1-4}$)alkyl groups (e.g. ethoxycarbonyloxymethyl, α-ethoxycarbonyloxyethyl) as well as those ester residues that are commonly used as carboxyl-protecting groups, such as, for example, tert-butyl, benzhydryl, benzyl, p-nitrobenzyl, p-methoxybenzyl p-nitrophenyl, β-trimethylsilylethyl, β,β,β-trichloroethyl and trialkylsilyl (e.g. tert-butyl-dimethylsilyl, isopropyldimethylsilyl). As the ester residues serving as carboxyl-protecting groups, tert-butyl, benzhydryl and p-nitrobenzyl, for instance, are especially preferable.

The amino-protecting group represented by R"' and R"" in the above formulas [I]' and [II]', respectively, is conveniently selected from among those which are commonly used for the same purpose in the synthesis of antimicrobial β-lactam compounds. Thus, for example, such amino-protecting groups as acyl groups (e.g. formyl, acetyl, monochloroacetyl, dichloroacetyl, trichloroacetyl, trifluoroacetyl), esterified carboxyl groups (e.g. tert-butoxycarbonyl, 2-cyanoethoxycarbonyl, β,β,β-trichloroethoxycarbonyl, β-trimethylsilylethoxycarbonyl, benzyloxycarbonyl, p-nitrobenzyloxycarbonyl, p-methoxybenzyloxycarbonyl, diphenylmethyloxycarbonyll and further trityl and trialkylsilyl. Especially preferable among these are formyl, monochloroacetyl and trityl, for instance.

The desired compounds according to this aspect of the invention, namely compounds [III]', may be used either in the free acid form with respect to the sulfa and carboxyl groups or, in the conventional manner, in the form of a salt with a nontoxic cation such as sodium or potassium or with a basic amino acid (e.g. arginine, ornithine, lysine, histidine) or a polyhydroxyalkylamine (e.g. N-methylglucamine, diethanolamine, triethanolamine, tris(hydroxymethyl)aminomethane), for instance. Also the amino group in the 2-position of the thiazole ring may be used either in the free amino form or, in the conventional manner, in the form of a salt with an organic acid (e.g. acetic, tartaric or methanesulfonic acid) or an inorganic acid (e.g. hydrochloric, hydrobromic, sulfuric or phosphoric acid) or further an acidic amino acid (e.g. aspartic or glutamic acid). Furthermore, the carboxyl group may be used in the form of an ester with such biologically active ester residue as mentioned above for R" and R""'. Although the compounds of this aspect of the invention, [III]', may be used in the form of a racemic mixture, it is the optically active form having the (3S,4S)-configuration that has an essential antimicrobial activity. Therefore, the present aspect of invention provides compounds [III] ' having the (3S,4S)-configuration, salts and esters thereof and methods of producing them.

The objective compounds (III)' or salts or esters thereof are valuable antibiotics active against gram-negative bacteria, among others. They are used as drugs for humans and domestic animals. More particularly, they are safely used as antimicrobial agents for treating a variety of bacterial infections. The compounds of this aspect of the invention [III]' or salts or esters thereof are added as bactericides to feed to be given to animals for the preservation thereof. Furthermore, they may be used as bactericides for destroying and inhibiting the growth of hazardous bacteria on, for instance, medical and dental devices and as industrial microbiocides for inhibiting the growth of hazardous bacteria in water-based paints, paper mill white water and other aqueous compositions in concentrations of 0.1-100 parts of compounds [III]' or salts or esters thereof per million parts of the aqueous compositions.

Compounds [III]' of this aspect of the invention or salts or esters may be used in various pharmaceutical compositions either alone or in combination with other active ingredients. The pharmaceutical compositions may take the form of capsules, tablets, powders, solutions, suspensions or elixirs. They may be administered orally, intravenously or intramuscularly.

The tablets for oral administration may contain usual vehicles such as binders (e.g. syrup, gum arabic, gelatin, sorbitol, gum tragacanth, polyvinylpyrrolidone), fillers (e.g. lactose, other saccharides, corn starch, calcium phosphate, sorbitol, glycine), lubricants (e.g. magnesium stearate, talc, polyethylene glycol, silica), disintegrants (e.g. potato starch) and available humectants (e.g. sodium lauryl sulfate). The tablets may be coated by the methods well known in the art. The liquid preparations for oral use may take the form of aqueous or oleaginous suspensions, solutions, emulsions, syrups, elixirs, etc., or may be dried products to be dissolved in water or other appropriate solvents prior to use. Such liquid preparations may contain suspending agents (e.g. sorbitol syrup, methylcellulose, glucose/saccharide syrup, gelatin, hydroxyethylcellulose, carboxymethylcellulose, aluminum stearate), hydrogenated edible oils (e.g. almond oil, fractionated coconut oil, oily esters), propylene glycol or ethyl alcohol, preservatives (e.g. methyl or propyl p-hydroxybenzoate, sorbic acid), etc. As the suppository bases, there may be used, for instance, cacao butter and other glycerides.

The compositions for injection may be supplied in the unit dosage form such as an ampule or a container with a preservative added. Said compositions may be in the form of suspensions, solutions or emulsions in oleaginous or aqueous solvents and may contain adequate auxiliaries such as suspending agents, stabilizers and/or dispersing agents. The active ingredients may also be formulated in the powder form so that the compositions may be reconstructed with an appropriate solvent, for example, sterilized pyrogen-free water, prior to use.

Furthermore, adequate forms for absorption through the nasal and laryngeal mucosa or the bronchial tissue may also be formulated, for instance, powders, liquid sprays or inhalants, lozenges and throat paints. For the eye or ear treatment, the active ingredients may be used in the liquid or semisolid form as capsules or drops. They may further be formulated in compositions for external use using hydrophobic or hydrophilic ointment, cream, lotion, paint, powder or other bases.

Furthermore, the pharmaceutical compositions may contain other ingredients than vehicles, such as, for example, stabilizers, binders, antioxidants, preservatives, lubricants, suspending agents, viscosity modifiers and flavoring agents. For broader antibacterial spectra, the compositions may also contain other active ingredients.

For use in domestic animals, the active ingredients may be formulated in intrammary compositions in bases adequate for prolonged action or rapid release.

Compounds [III]' of this aspect of the invention or salts or esters thereof may be used as the therapeutic agents for bacterial infections in the treatment of, for example, respiratory tract infections, urinary tract infections, suppurative diseases, bile tract infections, intestinal infections, gyneocological infections and surgical infections in mammals. The daily dose depends on condition of the patient to be treated, body weight of the host, route of administration (parenteral administration being suited for treating general infections and oral administration for treating intestinal infections) and frequency of administration, among others. Generally, the daily oral dose is about 15-300 mg of the active ingredient per kilogram of body weight of the patient in single or multiple application. The adequate daily dose for an adult human is about 10 to about 150 mg of the active ingredient per kg of body weight, preferably in 2 to 4 divided doses with each single dose of about 2.5 to about 75 mg/kg, and parenteral administration is adequate.

The compositions containing compounds (III)' or salts or esters thereof may be administered in several solid or liquid unit dosage forms administrable orally. The liquid or solid unit dosage form compositions contain the active substance in concentrations of 0.5-99%, preferably about 13-60%. The compositions generally contain about 15-1500 mg of the active ingredient. However, doses within the range of about 250-1000 mg are generally suited.

The desired compounds [III]' of this aspect of the invention or salts or esters thereof can be produced by sulfonating compounds [I]' or salts thereof followed by removing the protective group and, if necessary, the ester residue.

The starting compounds [I]' are used in the free form or in the form of salts such as mentioned for compounds (III)'. The sulfonation reaction involves the introduction of a sulfa group into the 1-position of compounds [I]' or salts thereof by reacting compounds [I]' or salts thereof with sulfuric anhydride (sulfur trioxide) or a functional derivative thereof, for instance. The functional derivative of sulfuric anhydride includes, for example, such adducts as sulfuric anhydride-pyridine, sulfuric anhydride-picoline, sulfuric anhydride-lutidine, sulfuric anhydride-N,N-dimethylformamide, sulfuric anhydride-dioxane, sulfuric anhydride-trimethylamine and sulfuric anhydride-chlorosulfonic acid as well as such mixtures as sulfuric acid-acetic anhydride. In the above sulfonation reaction, sulfuric anhydride or a functional derivative thereof is used in an amount of about 1-10 moles, preferably about 1-5 moles per mole of compound [I]'. The reaction temperature is about -20°C to about 80°C, preferably 0-60°C. The use of a solvent is generally preferred and the solvent includes water and commonly used organic solvents such as ethers (e.g. dioxane, tetrahydrofuran, diethyl ether), esters (e.g. ethyl acetate, ethyl formate), halogenated hydrocarbons (e.g. chloroform, dichloromethane), hydrocarbons (e.g. benzene, toluene, n-hexane) and amides (e.g. N,N-dimethylformamide, N,N-dimethylacetamide), either alone or in admixture. Depending on the kind of starting material [I]', sulfonating agent, reaction temperature and kind of solvent, the reaction generally proceeds to completion in a few scores of minutes to a few scores of hours. In some cases, several days may be required for completion of the reaction. After the reaction, the reaction product can be recovered by any of purification and separation procedures known *per se,* for example, solvent extraction, recrystallization and chromatography, in any desired purity. It is also possible to use the reaction mixture itself as the starting material in the next step.

The thus-obtained sulfonation products, upon removal of the protective group and, if necessary, the ester residue, give the desired products. The ester residue to be removed if necessary includes those residues generally used as the carboxyl-protecting groups and easily removable after the sulfonation reaction and may be removed simultaneously with the removal of the amino-protecting group R"' or before or after the removal of said amino-protecting group. Some of the above-mentioned amino-protecting and carboxyl-protecting groups, depending on the kind thereof, may be removed under the above-mentioned sulfonation reaction conditions, and, in such a case, no separate protective group removal step is required. The removal of the amino-protecting and carboxyl-protecting groups can be effected, for example, with an acid or base, or by reduction, depending on the protective groups species. When an acid is used, the acid which should be selected depending on the protective group species and other factors is, for

example, such an inorganic acid as hydrochloric, sulfuric or phosphoric acid, such an organic acid as formic, acetic, trifluoroacetic, propionic, benzenesulfonic or p-toluenesulfonic acid, or an acid-form ion exchange resin. When a base is used, the base which should be selected depending on the protective group species and other factors is, for example, such an inorganic base as a hydroxide or carbonate of an alkali metal (e.g. sodium, potassium) or an alkaline earth metal (e.g. calcium, magnesium), such an organic base as a metal alkoxide, an organic amine or a quaternary ammonium salt, or a base-form ion exchange resin. When a solvent is used for the protective group removal, the solvent is in most cases a hydrophilic organic solvent, water or a mixture thereof. When the removal is effected by reduction, the reduction is carried out, depending on the protective group species and other factors, by using such a metal as tin or zinc or such a metal compound as chromium dichloride or chromium acetate together with such an organic or inorganic acid as acetic, propionic or hydrochloric acid, or catalytically in the presence of a metal catalyst for catalytic reduction. The catalyst for use in the catalytic reduction includes among others platinum catalysts such as platinum wire, platinum sponge, platinum black, platinum oxide and colloidal platinum, palladium catalysts such as palladium sponge, palladium black, palladium oxide, palladium-on-barium sulfate, palladium-on-barium carbonate, palladium-on-carbon, palladium-on-silica gel and colloidal palladium, and nickel catalysts such as reduced nickel, nickel oxide, Raney nickel and Urushibara nickel. When a combination of a metal and an acid is used, a compound of such a metal as iron or chromium on one hand and such an inorganic acid as hydrochloric acid or such an organic acid as formic, acetic or propionic acid on the other are used. The reductive method is generally carried out in a solvent. In catalytic reduction, for instance, alcohols such as methanol, ethanol, propyl alcohol and isopropyl alcohol as well as ethyl acetate or the like are used frequently. In performing the method using a metal and an acid, the solvent is most frequently water or acetone or the like, and, when the acid is a liquid, the acid itself may be used as the solvent. The reaction in the acid-treatment, base-treatment or reduction procedure is generally carried out with or without cooling or warming. For removing a silyl-containing protective group, a fluoride ion-containing compound such as tetrabutylammonium fluoride or potassium fluoride may also be used. When the amino-protecting group is monochloroacetyl, removal thereof can easily be performed by using, for example, thiourea or sodium N-methyl-dithiocarbamate.

The desired compounds of this aspect of the invention can also be produced by reacting compound [A'] or a salt or ester thereof with carboxylic acid [II]' or a functional derivative thereof followed by removing the protective group and, if necessary, the ester residue.

Compounds [A'] may be used either in the free form or in the form of salts or esters such as mentioned for compounds [III]'. Carboxylic acids [II]' are used either in the free acid form or in the form of functional derivatives, with respect to the carboxyl group. The functional derivatives of carboxylic acids (II)' are, for example, acid halides, acid anhydrides, active amides, active esters and active thioesters. Examples of these functional derivatives are given below:

1) Acid halides:

Such acid halides as acid chlorides and acid bromides are used.

2) Acid anhydrides:

Such acid anhydrides as mixed acid anhydrides with, for example, monoalkyl carbonic acids, aliphatic carboxylic acids (e.g. acetic, pivalic, valeric, isovaleric, trichloroacetic acid) or aromatic carboxylic acids (e.g. benzoic acid) as well as symmetric acid anhydrides.

3) Active amides:

Amides with pyrazole, imidazole, 4-substituted imidazole, dimethylpyrazole and benzotriazole, for instance, are used.

4) Active esters:

Such active esters as methyl ester, ethyl ester, methoxy-methyl ester, propargyl ester, 4-nitrophenyl ester, 2,4-dinitrophenyl ester, trichlorophenyl ester, pentachlorophenyl ester, mesylphenyl ester and esters with 1-hydroxy-1H-2-pyridone, N-hydroxysuccinimide and N-hydroxyphthalimide, among others, are used.

5) Active thioesters:

Thioesters, for example, with thiols such as heterocycle thiols (e.g. 2-pyridinethiol, 2-benzothiazolylthiol, etc.)
Preferable one of said thioesters is a compound of the formula

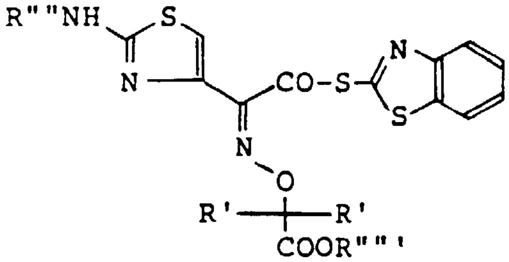

[II]"

wherein the symbols are as defined hereinabove. Said thioesters may be produced by reacting the compound [II]' with such a heterocycle thiol as mentioned above. A heterocycle thiole is used in an amount of 1 to 4 moles per mole of [II]'. The reaction may be conducted in an inactive organic solvent having no hydroxy group in its molecule, in the presence of a phosphine or phosphite. As such a phosphine, use is made of aryl phosphines such as triphenyl phosphine, and as such a phosphite, use is made of tri-lower alkyl phosphites such as trimethyl phosphite or triethyl phosphite. The phosphine or phosphite is preferably used in an amount of 1 to 2 moles per 1 mole of [II]'. The inactive organic solvent for use in this reaction includes halogenated hydrocarbons such as dichloromethane, chloroform, etc., nitriles such as acetonitrile, propionitrile, etc., esters such as ethyl acetate, isopropyl acetate, etc. Among them, nitriles such as acetonitrile, for instance, are especially preferable. The used amount of the solvent may be 10~50 times (weight) of that of the compound [II]'. In order to dissolve [II]', a base may be added to the solvent. For example, an organic base such as pyridine, N-methylmorpholine, triethylamine, etc. may be used as the base. The used amount of the base is 1~2.5 moles per 1 mole of [II]'. The reaction temperature is normally -30°C~50°C, preferably -20°C~25°C, more preferably -5°C~5°C. The reaction time is usually about 1~20 hours. Generally, thus obtained active thioester of the compound [II]' forms precipitation and so may be isolated by filtration. If necessary, before the filtration, putting the obtained reaction mixture into water, extracting the aqueous solution with such an organic solvent as mentioned above and then adding n-hexane, etc. to the extract in this order may be conducted to get the active thioesther of the compound [II]' as precipitates.

A functional derivative adequate for each specific instance is selected from among the above-mentioned derivatives depending on the kinds of R', R"" and R""' in carboxylic acid [II]'.

In practicing the above method, compound (A') or a salt or ester thereof is first reacted with carboxylic acid [II]' or a functional derivative thereof in a proportion of 1 mole of the former to at least 1 mole, preferably 1-4 moles of the latter. The reaction is generally carried out in a solvent. The solvent includes water, acetone, dioxane, acetonitrile, methylene chloride, chloroform, dichloroethane, tetrahydrofuran, ethyl acetate, N,N-dimethylformamide, N,N-dimethylacetamide, pyridine and other common organic solvents inert to the reaction. Hydrophilic solvents may be used in admixture with water. When carboxylic acid (II)' is used in the free form, the reaction is preferably carried out in the presence of a condensing agent, such as, for example, N,N'-dicyclohexylcarbodiimide, N-cyclohexyl-N'-morpholinoethylcarbodiimide, N-cyclohexyl-N'-(4-diethylaminocyclohexyl)carbodiimide or N-ethyl-N'-(3-dimethylaminopropyl) carbodiimide. The reaction may also be conducted in the presence of such a base as an alkali metal carbonate, a trialkylamine (e.g. trimethylamine, triethylamine, tributylamine, N-methylmorpholine, N-methylpiperidine), N,N-dialkylaniline, N,N-dialkylbenzylamine, pyridine, picoline, lutidine, 1,5-diazabicyclo(4,3,O)non-5-ene, 1,4-diazabicyclo(2,2,2) octane or 1,8-diazabicyclo(5,4,4)undecene-7. When the base or condensing agent is a liquid, it may also serve as the solvent. The reaction temperature is not critical but, generally, the reaction is carried out in many cases with cooling or at room temperature. The reaction is complete in several minutes to a few scores or hours. Especially, when an active thioester of the compound [II]' is used, the used amount thereof is preferably 1~2 moles per 1 mole of the compound [A']. In the use of the active thioester, a preferable solvent is a halogenated alkane such as methylene chloride, etc., and as a base, for example, an trialkylamine such as trimethylamine, etc. is advantageously used. The reaction temperature and time are 0~40°C and a few minutes to several hours, respectively, to gain a good result in this case. The reaction product can be recovered and purified by *per se* known methods, such as concentration, pH adjustment, phase transfer, solvent extraction, crystallization, recrystallization, fractional distillation and chromatography. The reaction product may also be used as the starting material in the next step in the form of a reaction mixutre, without isolating said product.

The product yielded by the above acylation reaction is then used as the starting material in the step of removing the protective group and, if necessary, the ester residue. This removal step is carried out in the same manner as the previously mentioned step of removing the protective group and, if necessary, the ester residue following the sulfonation.

Furthermore, the compounds of this aspect of the present invention can also be produced, for example, by reacting compound [A'], for example a compound of the formula

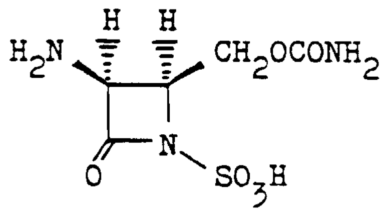

(IV)'

or a salt or ester thereof with a compound of the formula

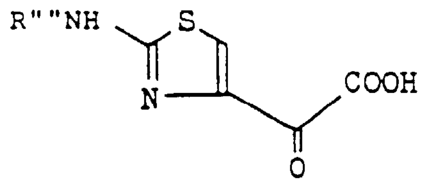

(V)'

wherein R"" is as defined above or a functional derivative thereof, subjecting the resulting compound of the formula

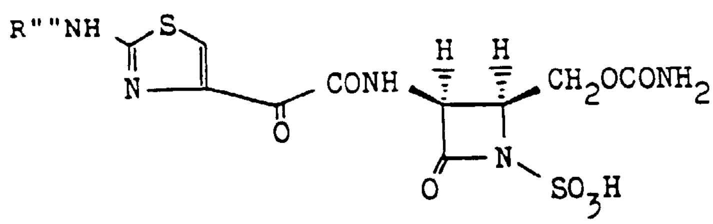

(VI)'

wherein R"" is as defined above or a salt or ester thereof to dehydration condensation with a compound of the formula

$$H_2N\text{-}OCH_2COOR'' \qquad \text{(VII)'}$$

wherein R" is as defined above, or a salt thereof, and removing the protective group and, if necessary, the ester residue. The reaction of compound (IV)' with compound [V]' can be carried out in the same manner as the reaction of compound [A'] with carboxylic acid [III', and the dehydration condensation reaction of compound [VI]' with compound [VII]' can be carried out by the method substantially disclosed in Japanese Published unexamined patent application No. 125190/1977, for instance. The removal of the protective group and the ester residue is advantageously carried out in the same manner as mentioned above.

The final products of this aspect of the present invention, namely compounds [III]', obtained after the removal of the protective group and, if necessary, the ester residue are isolated and purified by *per se* known methods such as mentioned above. Compounds [III]', having a sulfa group and a carboxyl group as well as an amino group, can generally form salts with bases and acids. Therefore, compounds [III]' may be recovered in the form of salts, and the salts may be converted to free forms or different salts. Furthermore, compounds [III]' obtained in the free form may be converted to salts. For converting salts of compounds [III]' with bases to free forms, acids, for instance may be used. The acid to be used in a specific instance depends on the protective group species and other factors and includes, among others, such inorganic acids as hydrochloric, sulfuric and phosphoric acid and such organic acids as formic, acetic and p-toluenesulfonic acid, which are frequently used. Furthermore, acid-form ion exchange resins and the like are usable. In many cases, a hydrophilic organic solvent (e.g. acetone, tetrahydrofuran, methanol, ethanol, dioxane), water or a mixture thereof is used as the solvent. This procedure is generally carried out at room temperature but may be carried out with cooling or heating. The reaction time depends on the kind of acid, the kind of solvent and the temperature. In any case, however, a shorter reaction period is preferred. The thus-produced free forms of compounds [III]' can be isolated by known methods such as mentioned above. Furthermore, compounds (III)' obtained in the form of free acids or salts may be converted to esters thereof by conventional methods.

Among the salts of the compound [III]', a salt with a nontoxic cation, especially mono- or di-sodium salt is

desirable. Di-sodium salt of the compound [III]' can be unexpectedly recovered in crystalline form which is highly stable in storage and has high water solubility. Crystalline di-sodium salt of the compound [III]' may be prepared, for example, by bringing non-crystalline di-sodium salt of the compound [III]' into contact with water vapor until 0.2~0.7 time (weight) of water to the salt has been absorbed, by condensing an aqueous solution of di-sodium salt of the compound [III]' until the water content has become 0.2~0.7 time (weight) to the salt, by adding an organic solvent to an aqueous solution of di-sodium salt of the compound [III]', or the like. As an organic solvent, use is made of a water-miscible solvent, e.g. alcohols such as methanol, ethanol, n-propanol, isopropanol or n-butanol, ketones such as acetone or methyl ethyl ketone, nitriles such as acetonitrile or propionitrile, ethers such as diethylether, tetrahydrofuran or dioxane, or a mixture thereof, etc. The volume to be used of solvent is not limited as far as the purpose is attained. Usually, it is 1 to 100 parts per 1 part of the aqueous solution. The preparation of said crystals is normally conducted at the temperature in the range of 0~40°C, preferably 10~35°C, more preferably 15~30°C. At a lower temperature than 0°C, the crystals grow too slowly, while at a higher temperature than 40°C, the starting material or/and the product decompose, and the recovery yield of crystallization decreases. The time required to form crystals varies according to the purity of the starting material, solvent, method for preparing crystals, temperature and so on. Generally, the preparation is accomplished within 3.0 minutes to 10 hours at the temperature of 15~30°C. Thus-obtained crystals, after, if necessary, being washed with use of an organic solvent as mentioned above or/and being subjected to dehydration, are isolated by filtration or centrifugation, for instance. The starting material, non-crystalline di-sodium salt of [III]' may be obtained according to *per se* known methods. For example, to a solution or suspension of the compound [III]' in water or mixture of water and organic solvent is added carbonic acid sodium salt such as sodium hydroxide, sodium carbonate or sodium hydrogen carbonate, aliphatic acid sodium salt such as sodium acetate, sodium propionate or sodium 2-ethylhexanate, aromatic carboxylic acid sodium salt such as sodium benzonate, in the proportion of two molecular equivalents or a slightly excess to the compound [III]' to give an aqueous solution of di-sodium salt thereof. Lyophilization of the said aqueous solution may give the non-crystalline salt.

The starting compounds [I]', [A'] and [II]' be used in practicing the invention can be prepared, for example, by the methods shown below or modifications thereof.

Method 1)

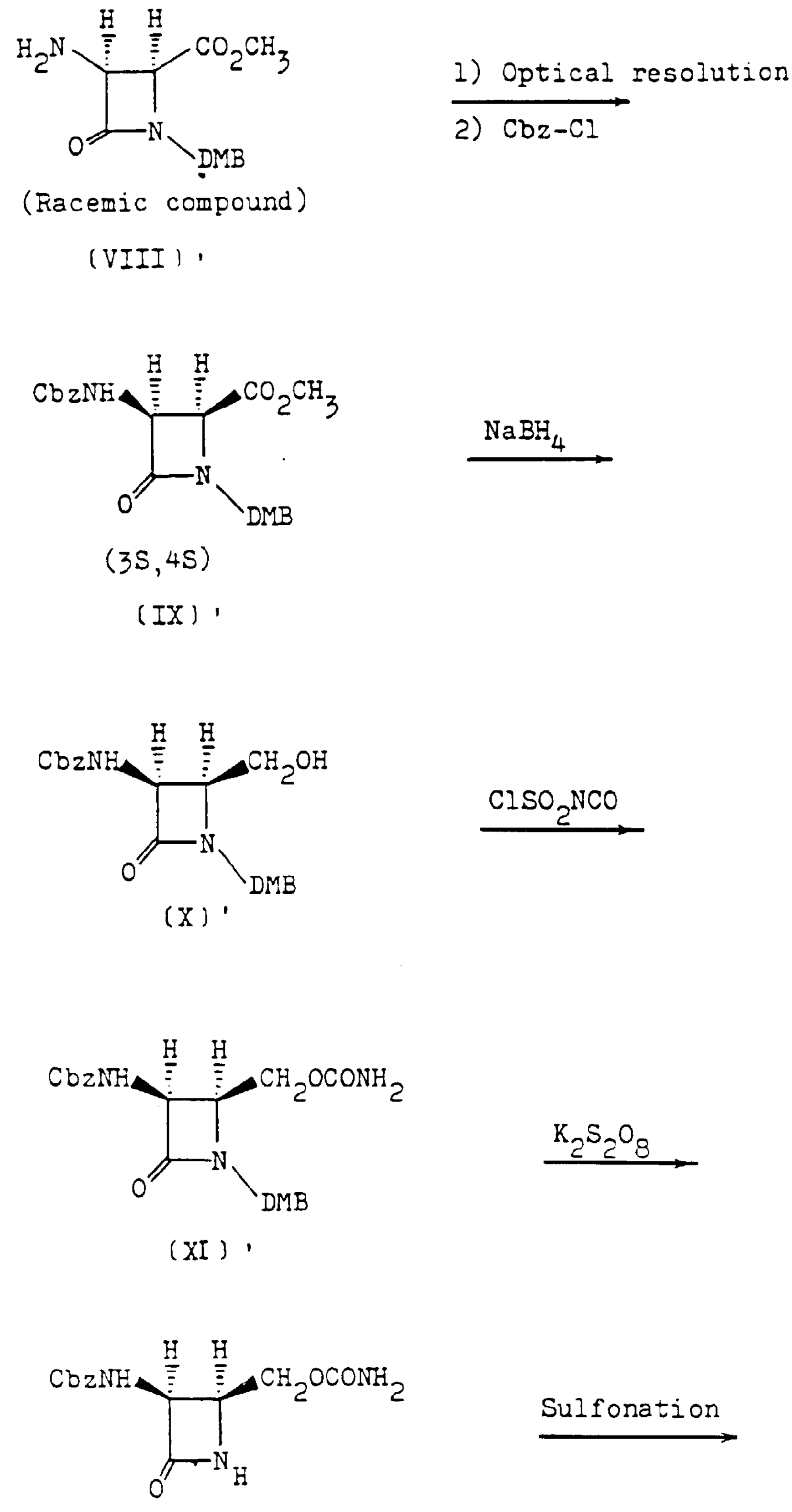


(Racemic compound)

(VIII)'

(3S,4S)

(IX)'

(X)'

(XI)'

(XII)'

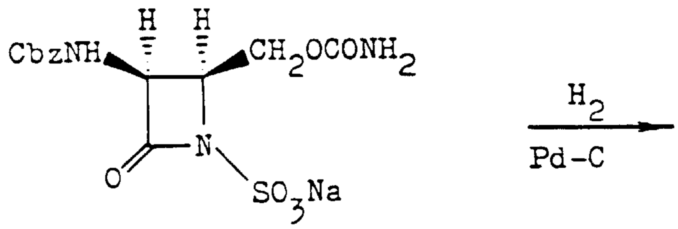


(XIII)'

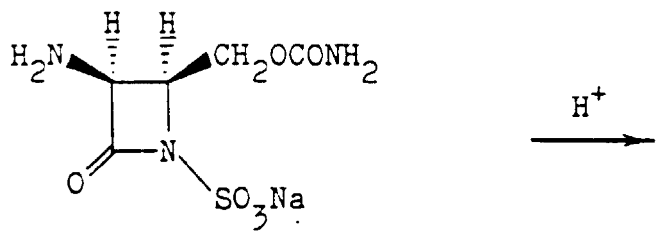


[A']

(XIV)'

Method 2)

[XII]'

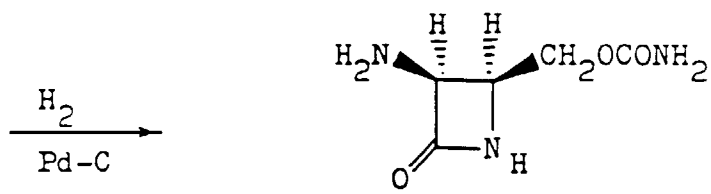


(XV)'

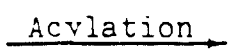


(I)'

Method 3)

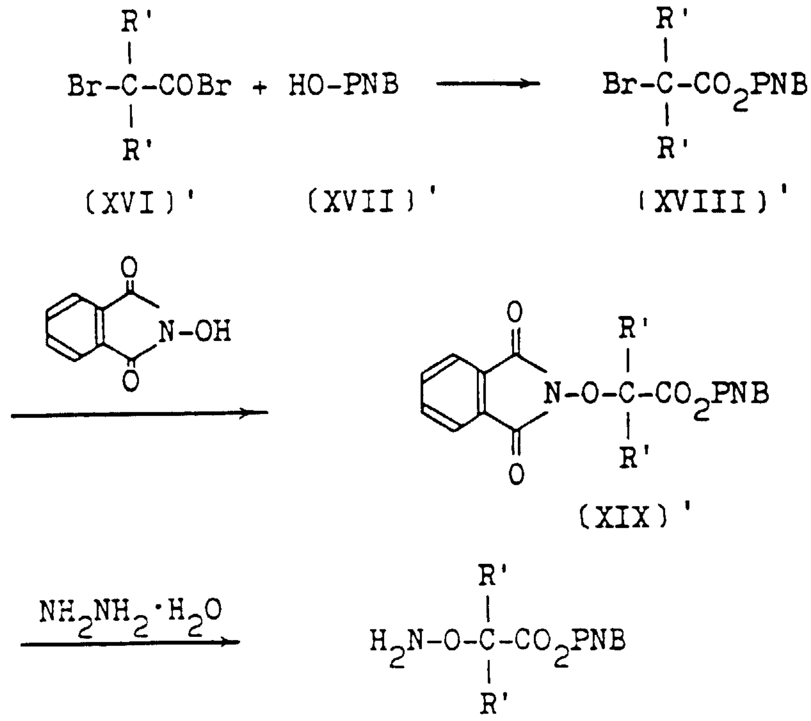


(XVI)' (XVII)' (XVIII)'

(XIX)'

(XX)'

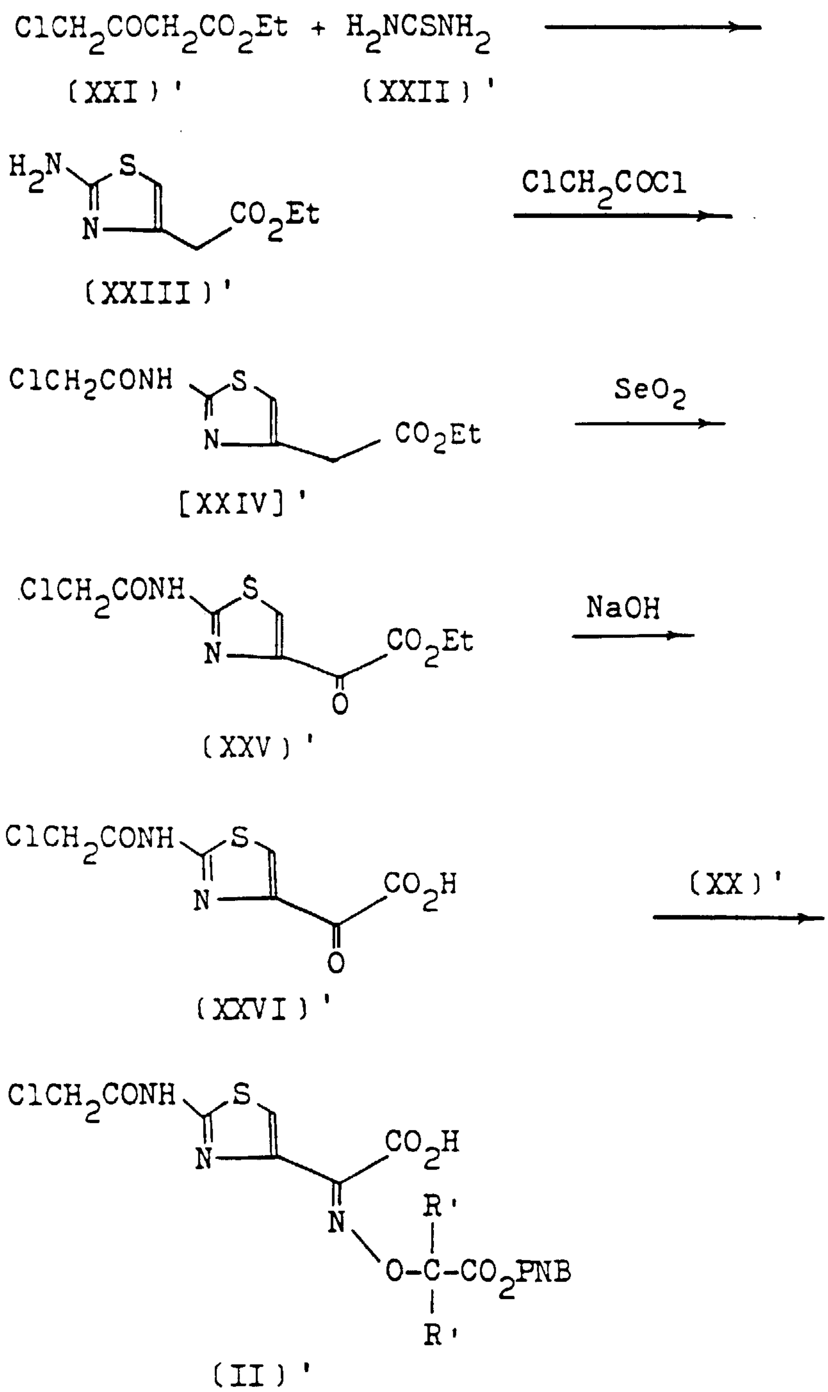


(R'''' = $ClCH_2CO$, R''''' = PNB)

In the above formulas, DMB stands for 2,4-dimethoxybenzyl, Cbz for benzyloxycarbonyl, Et for ethyl, and PNB for p-nitrobenzyl, and R' is as defined above.

The starting material for method 1) shown above, namely cis-3-amino-4-methoxycarbonyl-1-(2,4-di-methoxybenzyl)-2-azetidinone (VIII)', is a known compound described, for example, in the Journal of the American Chemical Society, vol. 99, page 2352 (1977) and can easily be prepared. Compound (II)' can be prepared, for example, by method 3) shown above or a modification thereof. Detailed procedures are disclosed, for example, in Japanese Patent Application Nos. 194311/1981 and 011965/1982.

The thus-obtained starting materials [I]', [A'] and [II]' may be fed to the reaction step in accordance with this aspect of the present invention either after isolation and purification by such conventional methods as mentioned above

or in the form of a reaction mixture.

The present invention will be described hereinafter in further detail by way of experimental examples, reference examples and working examples. It should, however, be understood that these examples are merely illustrative of the present invention and should by no means be construed as limiting the scope of the invention and that changes and modifications may be made by one skilled in the art without departing from the spirit of the present invention.

Referring to the column chromatographic procedures used in Examples and Reference Examples, elution was carried out in conjunction with a TLC (thin layer chromatography) assay unless otherwise noted. The TLC assay was performed using Merck 60 $F_{254}$ as the TLC plate, the solvent used as an eluent in column chromatography as the developing solvent, and a UV detector for detection. As an adjunct detection method, the spot on the TLC plate was sprayed with 48% HBr, hydrolyzed by heating, sprayed with ninhydrin reagent and reheated to see if the spot would turn red to red-purple. The fractions thus confirmed to contain the objective compound were pooled. When two different developing solvents are indicated, it means, unless otherwise noted, that the byproducts were eluted with the solvent first mentioned and the objective compound with the second-mentioned solvent. As to the purification procedures using Amberlite® or Sephadex® columns, water was run first and, then, aqueous ethanol was passed with an increasing concentration gradient unless specifically noted otherwise in the respective examples or reference examples. Unless otherwise indicated, all drying procedures employed anhydrous sodium sulfate as a desiccant.

Amberlite®, Dowex® and Sephadex® are the products of Rohm & Haas Co., U.S.A., Dow Chemical Co., and Pharmacia Fine Chemicals, respectively. The NMR spectra were measured using tetramethylsilane as an internal or external reference and an EM390 (90 MHz) or $T_{60}$ (60 MHz) spectrometer. All δ values are in ppm. The symbols used in Examples and Reference Examples have the following meanings.

| | |
|---|---|
| s : | singlet |
| d : | doublet |
| t : | triplet |
| q : | quartet |
| ABq : | AB type quartet |
| dd : | double doublet |
| m : | multiplet |
| sh : | shoulder |
| br : | broad |
| J : | coupling constant |
| mg : | milligram |
| g : | gram |
| ml : | milliliter |
| 1 : | liter |
| ppm : | part per million |
| Hz : | Herz |
| Ph : | phenyl |
| Me : | methyl |
| Et : | ethyl |
| DMSO : | dimethyl sulfoxide |
| $D_2O$ : | heavy water |
| m.p. or M.P. : | melting point |

In Examples and Reference Examples, 1-sulfo-2-oxoazetidine derivatives are sometimes referred to as 1-sulfo-2-azetidinone derivatives but these different designations are solely due to different systems of nomenclature and represent exactly the identical structures.

Experimental Example

The MIC (mcg/ml) values of some of the objective compounds according to the Examples were measured by the following procedure and are shown below.

Assay procedure

The MIC of each test compound was determined by the agar dilution method. Thus, a stock aqueous solution of each test compound is serially diluted and the resultant dilution series are put in petri dishes. Then, 9.0 ml of Trypticase soy agar per dish is added and mixed. Each of the agar plates is smeared with a suspension of the test organism

(about $10^8$ CFU/ml). After overnight culture at 37°C, the minimum concentration of the test compound that caused a complete growth inhibition of the organism is taken as the MIC (minimal inhibitory concentration) value.

Test organisms

(1) *Enterobacter cloacae* IFO 12937
(2) *Klebsiella pneumoniae* TN 1711

Results

| | | (mcg/ml) |
|---|---|---|
| Test compounds | Test organisms | |
| | (1) | (2) |
| Sodium *cis*-3-[2-(2-amino-4-thiazolyl)-2-(Z)-methoxyiminoacetamido]-4-carbamoyl-2-azetidinone-1-sulfonate | 1.56 | 0.39 |
| *cis*-3-[2-(2-Amino-4-thiazolyl)-2-(Z)-(1-carboxy-1-methylethoxyimino)acetamido]-4-ureidomethyl-2-azetidinone-1-sulfonic acid | 12.5 | 3.13 |
| Monosodium *cis*-3-[2-(2-amino-4-thiazolyl)-2-(Z)-(1-carboxylato-1-methylethoxyimino) acetamido]-4-pyridiniomethyl-2-azetidinone-1-sulfonate | 3.13 | 12.5 |
| *cis*-3-[2-(2-Amino-4-thiazolyl)-2-(Z)-(1-carboxy-1-methylethoxyimino)acetamido]-4-methoxymethyl-2-azetidinone-1-sulfonic acid | 1.56 | 0.2 |
| *cis*-3-[2-(2-Amino-4-thiazolyl)-2-(Z)-(1-carboxy-1-methylethoxyimino]acetamido]-4-methylsulfinyl-methyl-2-azetidinone-1-sulfonic acid | 3.13 | 0.78 |
| *cis*-3-[2-(2-Amino-4-thiazolyl)-2-(Z)-(1-carboxy-1-methylethoxyimino)acetamido]-4-methylsulfonyl-methyl-2-azetidinone-1-sulfonic acid | 12.5 | 1.56 |
| *cis*-3-[2-(2-Amino-4-thiazolyl)-2-(Z)-(1-carboxy-1-methylethoxyamino)acetamido]-4-(2-methoxy-carbonylethyl)-2-azetidinone-1-sulfonic acid | 6.25 | 3.13 |
| Sodium [3S, 4S]-*trans*-3-[2-(2-aminothiazol-4-yl]-2-(Z)-methoxyimino]acetamido]-4-(2-oxopropyl)-2-azetidinone-1-sulfonate | 6.25 | - |
| Sodium [3S,4S]-3-[(2-(2-aminothiazol-4-yl)-2-(Z)-(1-carboxy-1-methylethoxyimino)acetamido]-4-propyl-2-azetidinone-1-sulfonate | 12.5 | 0.78 |
| *cis*-3-[2-(2-Amino-4-thiazolyl)-2-(Z)-carboxy-methoxyiminoacetamido]-4-methoxymethyl-2-azetidinone-1-sulfonic acid | 3.13 | 0.1 |
| Sodium [3S,4S]-3-[(2-(2-aminothiazol-4-yl)-2-(Z)-(1-carboxy-1-methylethoxyimino]acetamido]-4-(2-oxopropyl)-2-azetidinone-1-sulfonate | 12.5 | 3.13 |
| Sodium [3S,4S]-*cis*-3-[(2-(2-aminothiazol-4-yl)-2-(Z)-methoxyimino]acetamido]-4-(2-oxopropyl)-2-azetidinone-1-sulfonate | 3.13 | 3.13 |

Reference Example 1

*cis*-3-Benzyloxycarboxamido-4-methylcarbamoyl-2-azetidinone (831 mg) is dissolved in a mixture of tetrahydrofuran (50 ml) and methanol (50 ml), 5% palladium-on-carbon (830 mg) is added, and the mixture is stirred in a hydrogen atmosphere at room temperature for an hour. The catalyst is filtered off and the filtrate is concentrated under reduced pressure, whereupon the residue crystallize. Yield 410 mg. This product (286 mg) is dissolved in a mixture of tetrahydrofuran (6 ml) and water (6 ml), and sodium bicarbonate (370 mg) is added. Under ice-cooling and stirring, 1.125 g of 2-(2-chloroacetamido-4-thiazolyl)-2-(Z)-(p-nitrobenzyloxycarbonylmethoxyimino)acetyl chloride hydrochloride is added and the mixture is stirred for an hour. The solvent is then distilled off under reduced pressure, followed

by addition of water. The resulting crystalline precipitate is collected by filtration, washed with aqueous sodium bicarbonate, water, ethanol and ether in that order, and air-dried to give 960 mg of cis-3-[2-(2-chloroacetamido-4-thiazolyl)-2-(Z)-(p-nitrobenzyloxycarbonylmethoxyimino)acetamido]-4-methylcarbamoyl-2-azetidinone as colorless crystals.

NMR ($d_6$-DMSO) δ: 2.58 (3H, d, J=5Hz, $CH_3$), 7.50 (1H, s proton at 5-position of the thiazole ring).

Reference Example 2

*cis*-4-Aminomethyl-3-benzyloxycarboxamido-1-(2,4-dimethoxybenzy)-2-azetidinone (3.2 g) is suspended in 150 ml of water and dissolved by warming at the pH adjusted to 2 with 10% hydrochloric acid. A small amount of insoluble matter is filtered off, 3.2 g of potassium cyanate is added, and the mixture is stirred at 70-80°C for 20 minutes. After cooling, the crystalline precipitate is collected by filtration, washed with water and ether and dried to give 3.3 g of cis-benzyloxycarboxamido-1-(2,4-dimethoxybenzyl)-4-ureidomethyl-2-azetidinone as colorless crystals.

M.p. 202-204°C

IR (KBr) $cm^{-1}$: 3440, 3280, 2990, 1730, 1680, 1660.

NMR ($d_6$-DMSO) δ: 3.0~3.4 (2H, m, $C_4$-$CH_2$), 3.4~3.7 (1H, m, $C_4$-H), 3.77 (3H, s, $OCH_3$), 3.80 (3H, s, $OCH_3$), 4.23 (2H, ABq, $N_1$-$CH_2$), 4.82 (1H, d, d, J=6, 9Hz, $C_3$-H), 5.07 (2H, s, ph*$CH_2$*), 7.39 (5H, s, ph), 8.03 (1H, d, J=9Hz, $C_3$-NH).

| Elemental analysis for $C_{22}H_{26}N_4O_6$: | | | |
|---|---|---|---|
| | C(%) | H(%) | N(%) |
| Calcd. | 59.72 | 5.92 | 12.66 |
| Found | 59.29 | 5.67 | 12.51 |

Reference Example 3

*cis*-3-Benzyloxycarboxamido-1-(2,4-dimethoxybenzyl)-4-ureidomethyl-2-azetidinone (3.2 g) is suspended in a mixture of acetonitrile (185 ml) and water (71 ml), 2.74 g of potassium persulfate and 1.64 g of dipotassium phosphate are added, and the mixture is stirred at 90°C for 1.5 hours. The reaction mixture is concentrated to 100 ml under reduced pressure and 200 ml of water is added to the concentrate. The resulting solution is washed with 100 ml of ethyl acetate and the ethyl acetate layer is extracted with 200 ml of water. The aqueous layers are combined, washed with 50 ml of ethyl acetate and concentrated to 100 ml under reduced pressure. The crystalline precipitate is collected by filtration and recrystallized from 500 ml of ethanol to give 1.0 g of cis-3-benzyloxycarboxamido-4-ureidomethyl-2-azetidinone as colorless crystals.

M.p. 218-220°C

IR (KBr) $cm^{-1}$: 3480, 3370, 3220, 3070, 2940, 1740, 1650.

NMR ($d_6$-DMSO) δ: 3.05~3.3 (2H, m, $C_4$-$CH_2$), 3.55~3.8 (1H, m, $C_4$-H), 4.87 (1H, d.d, J =6,9Hz, $C_3$-H), 5.09 (2H, s, ph*$CH_2$*), 7.40 (5H, s, ph), 8.01 (1H, d, J=9Hz, $C_3$-NH), 8.16 (1H, s, $N_1$-H).

| Elemental analysis for $C_{13}H_{16}N_4O_4$: | | | |
|---|---|---|---|
| | C (%) | H (%) | N (%) |
| Calcd. | 53.42 | 5.52 | 19.17 |
| Found: | 53.11 | 5.37 | 19.00 |

Reference Example 4

*cis*-3-Benzyloxycarboxamido-4-cyanomethyl-1-(2,4-dimethoxybenzyl-2-azetidinone (3.5 g) is dissolved in 100 ml of acetone and, under ice-cooling and stirring, 30 ml of 30% aqueous hydrogen peroxide is added. Then, 11 ml of 1 N sodium hydroxide is added and the mixture is stirred at room temperature for 12 hours, adjusted to pH 8 with 3 N hydrochloric acid and concentrated under reduced pressure. To the residual aqueous solution is added sodium chloride and the mixture is extracted with ethyl acetate-tetrahydrofuran (3:1). The extract is washed with 3 N hydrochloric acid, saturated aqueous sodium bicarbonate and aqueous sodium chloride in that order, dried over anhydrous magnesium sulfate and concentrated under reduced pressure. The residue is subjected to column chromatography using silica gel (140 g) and the column is treated with chloroform-ethyl acetate (1:1), followed by elution with chloroform-ethyl acetate-methanol (4:4:1) to give 1.45 g of *cis*-3-benzyloxycarboxamido-4-carbamoylmethyl-1-(2,4-dimethoxybenzyl)-2-acetidinone.

IR (KBr) $cm^{-1}$: 3280, 1755, 1685, 1640.

NMR ($d_6$-DMSO) δ: 2.38 (2H, d, J=6Hz, $C_4$-$CH_2$), 3.75 (3H, s, $OCH_3$), 3.78 (3H, s, $OCH_3$), 4.20 (2H, ABq, $N_1$-$CH_2$), 4.87 (1H, d.d, J=5.9 Hz, $C_3$-NH), 5.05(2H, s, ph*CH₂*), 7.38 (5H, s, ph), 8.00(1H, d, J-9Hz, $C_3$-NH).

Reference Example 5

*cis*-3-Benzyloxycarboxamido-2-azetidinone4-carboxylic acid (3 g) is dissolved in a mixture of tetrahydrofuran (80 ml) and water (40 ml) and, under ice-cooling and stirring, 1.05 g of sodium bicarbonate is added. Then, 1.38 g of glycine amide hydrochloride and 2.61 g of 1-ethyl-3-(3-(dimethylaminopropyl)-carbodiimide hydrochloride are added and the mixture is stirred at room temperature for 40 hours and concentrated to 10 ml under reduced pressure, followed by addition of 20 ml of water. The resulting crystalline precipitate is collected by filtration and washed with water, ether and hexane in that order to give 1.4 g of cis-3-benzyloxycarboxamido-4-carbamoylmethylaminocarbonyl-2-azetidinone.

IR (KBr) $cm^{-1}$: 3280, 1770, 1690, 1665.

NMR ($d_6$-DMSO) δ: 4.25 (1H, d, J=6Hz, $C_4$-H), 5.05 (2H, s, $CH_2$ph), 5.12 (1H, d.d, J=6.9 Hz, $C_3$-H), 7.36 (5H, s, ph), 7.76 (1H, d, J=9Hz, $C_3$-NH).

Reference Example 6

D-(-)-p-Hydroxyphenylglycine (112.8 mg) is suspended in methylene chloride (3 ml) and 219.9 mg of trimethylchlorosilane is added. Then, 204.9 mg of triethylamine is added under ice-cooling and the mixture is stirred at room temperature for 2 hours. Under ice-cooing, a suspension of 6,7-dihydroxychromone-3-carbonyl chloride (162.5 mg) in methylene chloride (7.5 ml) is added to the above mixture over 20 minutes, and the whole mixture is further stirred at room temperature for 2 hours. The solvent is then distilled off under reduced pressure and 10 ml of water is added to the residue. The resulting crystalline precipitate is collected by filtration and dried to give 210 mg of D-(2-6,7-dihydroxychromone-3-carboxamido)-2-(4-hydroxyphenyl)acetic acid.

M.P. 190-200°C (decompn.) $[\alpha]_D^{20}$+ 16.9° (C=0.3, DMSO).

IR (KBr) $cm^{-1}$ 3200, 1720, 1655, 1610.

Reference Example 7

*cis*-3-Amino-1-(2,4-dimethoxybenzy)-4-methoxycarbonyl-2-azetidinone (10 g) is dissoved in dry tetrahydrofuran (50 ml), 8.2 g of di-t-butyl dicarbonate is added, and the mixture is refluxed for 3 hours and concentrated under reduced pressure. The residue is dissolved in a mixture of 160 ml of tetrahydrofuran and 80 ml of water, 3.21 g of sodium borohydride is added under ice-cooling, and the whole mixture is stirred at room temperature for 8 hours and extracted with ethyl acetate. The extract is washed with aqueous monopotassium phosphate, dried over anhydrous magnesium sulfate and concentrated under reduced pressure. The residue is subjected to column chromatography using silica gel (100 g). The column is treated with a 1:1 mixture of hexane and ethyl acetate, followed by elution with a 1:2 mixture of hexane and ethyl acetate. The solvent is then distilled off under reduced pressure to give 11.5 g of *cis*-3-(t-butoxycarboxamido)-1-(2,4-dimethyoxybenzyl)-4-hyroxymethyl-2-azetidinone as colourless crystals.

M.p. 135-136°C

IR (KBr) $cm^{-1}$ : 3420, 3300, 2975, 2930, 1735, 1705.

| Elemental analysis for $C_{18}H_{26}O_6N_2$: | | | |
|---|---|---|---|
| | C(%) | H(%) | N(%) |
| Calcd. | 59.00 | 7.15 | 7.65 |
| Found | 59.03 | 7.20 | 7.38 |

Reference Example 8

*cis*-3-(t-Butoxycarboxamido)-1-(2,4-dimethoxybenzyl)-4-hydroxymethyl-2-azetidinone (7.33 g) is dissolved in pyridine (60 ml) and 2.32 ml of methylsulfonyl coloride is added dropwise under ice-cooling and stirring. The mixture is stirred at room temperature for 30 minutes, 100 ml of ethyl acetate, 100 ml of tetrahydrofuran and 100 ml of water are added, and the whole mixture is adjusted to pH 2 with 3 N hydrochloric acid under ice-cooling and stirring. The organic solvent layer is separated and the aqueous layer is extracted with ethyl acetate-tetrahydrofuran. The organic solvent layers are combined, washed with water, aqueous sodium bicarbonate and aqueous sodium chloride in that order, and dried over anhydrous magnesium sulfate. The solvent is then distilled off under reduced pressure and ether

is added to the solid residue. The solid substance is collected by filtration to give 7.98 g of *cis*-3-(t-butoxycarboxamido)-1-(2,4-dimethoxybenzyl)-4-methylsulfonyloxymethyl-2-azetidinone as colorless crystals.

M.p. 158-159°C

IR (KBr) $cm^{-1}$ : 3300, 1760, 1685.

NMR ($d_6$-DMSO) δ: 1.38 (9H, s, t-butyl)-3.07 (3H, s, $SO_2CH_3$),4.98 (1H, d, d, J=5, 9Hz, $C_3$-H), 7.58 (1H, d, J=9Hz, $C_3$-NH).

| Elemental analysis for $C_{19}H_{28}H_2O_8S$: | | | |
|---|---|---|---|
| | C (%) | H (%) | N (%) |
| Calcd. | 51.34 | 6.35 | 6.30 |
| Found | 51.30 | 6.44 | 6.28 |

Reference Example 9

*cis*-3-Amino-4-methoxycarbonyl-1-(2,4-dimethoxybenzyl)-2-azetidinone (23.54 g) and di-(p-toluoyl)-D-tartaric acid monohydrate (16.17 g) are added to 600 ml of acetonitrile, and the mixture is warmed for dissolution, filtered and allowed to cool. The resulting crystalline precipitate is collected by filtration and washed with cold acetonitrile to give the salt (20.3 g). Recrystallization from 300 ml of acetonitrile gave 16.3 g of the salt.

M.p. 165-168°C

$[\alpha]_D^{22}$ +71.9° (c=0.985, MeOH)

The above salt is dissolved in a mixture of 100 ml of water and 300 ml of tetrahydrofuran, followed by addition of 6.1 g of sodium hydrogen carbonate. Then, under ice-cooling and stirring, 4.2 ml of carbobenzoxy chloride is added dropwise. The mixture is stirred under ice-cooling for an hour and then at room temperature for an hour, and the tetrahydrofuran is distilled off under reduced pressure on a water bath at not higher than 30°C. The residue is shaken with 400 ml of ethyl acetate and 200 ml of water and the aqueous layer is reextracted with 200 ml of ethyl acetate. These extracts are combined and washed twice with 2% aqueous solution of sodium bicarbonate, then with aqueous sodium chloride, 1 N hydrochloric acid and aqueous sodium chloride in that order, and dried. The solvent is then distilled off under reduced pressure and 30 ml of ether is added. The resulting crystalline precipitate is collected by filtration and dissolved in 50 ml of ethyl acetate by warming. After filtration, 50 ml of hexane is added to the filtrate and the mixture is allowed to cool. The colorless crystalline precpitate is collected by filtration to give 6.45 g (37.5%) of (3S, 4S)-*cis*-3-benzyloxycarboxamido-4-methoxycarbonyl-1-(2,4-dimethoxybenzyl)-2-azetidinone.

M.p. 120-121°C

$[\alpha]_{22}^{D}$ + 24.4° (c=1.08, $CHCl_3$)

IR $\nu_{max}^{Nujol}$ $cm^{-1}$ : 3300, 1770, 1745, 1695.

| Elemental analysis for $C_{22}H_{24}N_2O_7$: | | | |
|---|---|---|---|
| | C(%) | H(%) | N(%) |
| Calcd. | 61.67 | 5.65 | 6.54 |
| Found | 61.50 | 5.59 | 6.37 |

Reference Example 10

(3S,4S)-*cis*-3-Benzyloxycarboxamido-1-(2,4-dimethoxybenzyl)-4-hydroxymethyl-2-azetidinone (2.0 g) is dissolved in 40 ml of methylene chloride and, under ice-cooling and stirring, 0.52 ml of chlorosulfonyl isocyanate is added. The mixture is stirred under ice-cooling for 30 minutes and 0.35 ml of chlorosulfonyl isocyanoate is added. The mixture is further stirred for 10 minutes and, under ice-cooling, a solution of 1.26 g of sodium sulfite in 30 ml of water is added. The whole mixture is stirred at room temperature for an hour. The methylene chloride is then distilled off under reduced pressure and the residue is extracted with chloroform. The extract is washed with aqueous sodium chloride and dried over anhydrous magnesium sulfate. The solvent is distilled off under reduced pressure,.followed by addition of ether and filtration to give 2.46 g of crude crystals. Recrystallization from ethyl acetate-hexane gives 1.72 g (77.7%) of (3S, 4S)-*cis*-3-benzyloxycarboxamido-4-carbamoyloxymethyl-1-(2,4-dimethoxybenzyl)-2-azetidinone as colorless crystals.

M.p. 173-180°C

$[\alpha]_D^{24.5}$ + 34.5° (c=0.8, DMSO).

IR $\nu_{max}^{KBr}$ $cm^{-1}$: 3410, 3300, 1760, 1710.

NMR ($d_6$-DMSO) δ: 3.74 (3H, s, $OCH_3$), 3.76 (3H, s, $OCH_3$), 3.7~4.3 (3H, m, $C_4$-H, $C_4$-$CH_2$), 4.20 (2H, ABq, J=15Hz, $N_1$-$CH_2$), 4.92 (1H, d.d, J=5, 10 Hz, $C_3$-H), 5.05 (2H, s, *$CH_2$*ph), 7.35 (5H, s, ph), 7.87 (1H, d, J=10Hz, $C_3$-NH).

| Elemental analysis for $C_{22}H_{25}N_3O_7$: | | | |
|---|---|---|---|
| | C(%) | H(%) | N(%) |
| Calcd. | 59.59 | 5.68 | 9.48 |
| Found | 59.30 | 5.70 | 9.57 |

Reference Example 11

*cis*-3-Benzyloxycarboxamido-4-(2-methoxycarbonylethenyl)-1-(2,4-dimethoxybenzyl)-2-azetidinone (908 mg) is dissolved in 50 ml of methanol, 908 mg of 5% palladium-on-carbon is added, and the mixture is stirred in a hydrogen atmosphere at room temperature for an hour. The catalyst is filtered off and the filtrate is concentrated under reduced pressure. The oily residue is dissolved in 5 ml of methylene chloride and 870 mg of propyene oxide is added. Then, under ice-cooling and stirring, 410 mg of carbobenzoxy chloride is added dropwise and the mixture is stirred under ice-cooling for an hour. The solvent is then distilled off under reduced pressure and ether is added to the residue. The resulting crystalline precipitate is collected by filtration to give 529 mg of *cis*-3-benzyloxycarboxamido-4-(2-methoxycarbonylethyl)-1-(2,4-dimethoxybenzyl)-2-azetidinone.

IR (KBr) $cm^{-1}$ : 3300, 1760, 1730, 1680.

NMR($CDCl_3$) δ: 3.60 (3H, s, $CO_2CH_3$), 3.75(6H, s, 2×$OCH_3$), 5.85(1H, d, J=9Hz, $C_3$-NH), 7.20 (5H, s, ph).

Reference Example 12

In 20 ml of ethyl acetate is dissolved 940 mg of 2,2-dimethyl-7-azido-1-aza-3-oxabicyclo[4,2,0]octan-8-one (1:4 cis-trans mixture) and catalytic reduction is carried out using 300 mg of 10% palladium-on-carbon (wet). The catalyst is then filtered off and washed with ethyl acetate. The filtrate and washings are combined and concentrated to about 20 ml. Then, 4 ml of propylene oxide and 0.9 ml of carbobenzoxy chloride are added and the mixture is stirred at room temperature for 1.5 hours and concentrated. The residue is subjected to column chromatography using silica gel (60 g), elution being carried out with chloroform-ethyl acetate (6:4) to give 388 mg of *cis*-(2,2-dimethyl-7-benzyloxyformamido-1-aza-3-oxa-bicyclo[4,2,0]octan-8-one) as the first product.

M.p. 129-131°C (crystallized from isopropyl ether)

IR (KBr) $cm^{-1}$ : 3280, 1770, 1725, 1550, 1250.

NMR ($CDCl_3$) δ: 1.35 (3H, s, $CH_3$), 1.60 (2H, *m*, $CH_2$), 1.70 (3H, s, $CH_3$), 3.83 (3H, *m*, $CH_2$ and $C_6$-H), 4.95 (1H, dd, $J_1$=5Hz, $J_2$=8Hz, $C_7$-H), 5.05 (2H, s, $CH_2$), 6.27 (1H, d, J=8Hz, NH), 7.25 (5H, s, arom H).

| Elemental analysis for $C_{16}H_{20}N_2O_4$: | | | |
|---|---|---|---|
| | C (%) | H (%) | N (%) |
| Calcd. | 63.14 | 6.22 | 9.21 |
| Found | 63.31 | 6.55 | 8.77 |

Further elution gives *trans*-(2,2-dimethyl-7-benzyloxyformamide-1-aza-3-oxabicyclo[4,2,0]octan-8-one. Yield 324 mg.

M.p. 141-143°C (crystallized from isopropyl ether)

IR (KBr) $cm^{-1}$ : 3270, 1720, 1540, 1270.

NMR ($CDCl_3$) δ: 1.40 (3H, s, $CH_3$), 1.72 (3H, s, $CH_3$), 1.86 (2H, *m*, $CH_2$), 3.52 (1H, *m*, $C_6$-H), 3.80 (2H, *m*, $CH_2$), 4.38 (1H, dd, $J_1$ =2Hz, $J_2$=8Hz, $C_7$-H), 5.08 (2H, s, $Ch_2$), 6.80 (1H, d, J=8Hz, NH), 7.30 (5H, s, arom H).

| Elemental analysis for $C_{16}H_{20}N_2O_4$: | | | |
|---|---|---|---|
| | C(%) | H(%) | N(%) |
| Calcd. | 63.14 | 6.22 | 9.21 |
| Found | 63.04 | 6.38 | 9.05 |

The above cis-compound (456.5 mg) is dissolved in 45 ml of acetone, 2.1 ml of 8 N Jones reagent is added under ice-cooling, and the mixture is stirred for 3 hours. After completion of the reaction, acetone and then isopropyl

alcohol (4 ml) are added and the mixture is stirred for 10 minutes. The resulting insoluble matter is filtered off and the filtrate is concentrated to dryness. The residue is extracted with a mixture of 30 ml of tetrahydrofuran and 50 ml of ethyl acetate and the extract is washed with a small amount of saturated aqueous sodium chloride, dried over sodium sulfate and concentrated to about 50 ml. The concentrate is methylated with a diazomethane-ether solution and the reaction mixture is concentrated to dryness. The residue is subjected to column chromatography using silica gel (30 g), elution being carried out with chloroform then with ethyl acetate to give 109 mg of *cis*--3-benzyloxyformamido-4-methoxycarbonylmethyl-2-azetidinone.

IR (KBr) $cm^{-1}$ : 3300, 1775, 1740, 1695, 1540, 1260.

NMR($CDCl_3$)δ: 2.59(2H, *m*, $CH_2$), 3.65 (3H, s, $OCH_3$), 4.10 (1H, *m*, $C_4$-H), 5.00 (1H, *m*, $C_3$-H) 5.10 (2H, s, $CH_2$), 5.90 (1H, d, J=8Hz, NH), *m*, 6.38 (1H, s, NH), 7.30 (5H, s, arom H).

The above product (109 mg) is dissolved in 10 ml of methyl alcohol and catalytic reduction is carried out using 60 mg of 10% palladium-on-carbon (wet). The catalyst is then filtered off and the filtrate is concentrated to dryness. The residue is dissolved in 15 ml of tetrahydrofuran and, under ice-cooling, 0.14 ml of triethylamine and then 116 mg of 2-(2-chloroacetamido-4-thiazolyl)-2-(Z)-(methoxyimino)acetyl chloride hydrochloride are added. The mixture is stirred for 15 minutes and further stirred at room temperature for 1.5 hours, followed by addition of 100 ml of ethyl acetate. The mixture is washed with saturated aqueous sodium chloride, dried over sodium sulfate and concentrated to dryness to give 190 mg of *cis*-3-[2-(2-chloroacetamido-4-thiazolyl)-2-(Z)-(methoxyimino)acetamido]-4-methoxycarbonylmethyl-2-azetidinone.

IR (KBr) $cm^{-1}$ : 3420, 3275, 1750, 1740, 1665, 1550.

Reference Example 13

*cis*-3-Benzyloxycarboxamido-4-hydroxymethyl-2-azetidinone (2.5 g) is dissolved in dry methylene chloride (400 ml) and, under ice-cooling, 142 mg of a boron trifluoride ether complex is added. Then, solution of diazomethane in ether is added in excess and the mixture is stirred under ice-cooling for 4 hours, followed by addition of water and ethyl acetate. The organic layer is separated, washed with aqueous sodium chloride, dried over anhydrous magnesium sulfate and concentrated under reduced pressure. The residue is subjected to column chromatography using silica gel (80 g). The column is treated with ethyl acetate, followed by elution with ethanol-ethyl acetate (1:9). The solvent is then distilled off to give 280 mg of *cis*-3-benzyloxycarboxamido-4-methoxymethyl-2-azetidinone.

IR (KBr) $cm^{-1}$ : 3310, 3200, 1780, 1720, 1690, 1665.

NMR ($CDCl_3$ + $d_6$-DMSO) δ: 3.33 (3H, s, $OCH_3$), 5.10 (2H, s, $CH_2$Ph), 7.34 (5H, s, Ph).

Reference Example 14

*cis*-4-Methylthiomethyl-3-(2,2,2-trichloroethoxycarboxamido)-2-azetidinone (600 mg) is dissolved in a mixture of 100 ml of tetrahydrofuran and 40 ml of 1N ammonium acetate, 10 mg of zinc is added and the mixture is stirred at room temperature for 4 hours. The mixture is filtered and the solid matter on the filter is washed with tetrahydrofuran. The filtrate and washings are combined and the tetrahydrofuran is distilled off under reduced pressure. To the residual aqueous solution is added 40 ml of tetrahydrofuran and, under ice-cooling and stirring, 784 mg of sodium bicarbonate is added. Then, 985 mg of 2-(2-chloroacetamido-4-thiazolyl)-2-(Z)-(1-benzhydryloxycarbonyl-1-methylethoxyimino) acetyl chloride hydrochloride is added and the mixture is stirred at room temperature for an hour and extracted with ethyl acetate-tetrahydrofuran (2:1). The extract is washed with 0.1 N sodium hydroxide and aqueous sodium chloride in that order, dried over anhydrous magnesium sulfate, and concentrated under reduced pressure. The residue is dissolved in a mixture of 100 ml of methanol and 40 ml of water and, at room temperature, 1.2 g of sodium metaperiodate is added. The mixture is stirred for 30 minutes and concentrated under reduced pressure. The crystalline precipitate is collected by filtration and washed with ether to give 855 mg of *cis*-3-[2-(2-chloroacetamido-4-thiazolyl)-2-(Z)-(1-benzhydryloxycarbonyl-1-methylethoxyimino)acetamido]-4-methylsulfinylmethyl-2-azetidinone.

IR (KBr) $cm^{-1}$ : 3170, 2960, 1740 (br) 1670 (br)

NMR ($d_6$-DMSO) δ: 2.52 (3H, s, $SOCH_3$).

Reference Example 15

Dimethyl sulfoxide (4.65 g) is dissolved in 47 ml of methylene chloride and, under cooling at -78°C in an argon atmosphere, 8.4 g of trifluoroacetic anhydride is added dropwise. The mixture is stirred for 15 minutes. Then, a solution of 8 g of *cis*-3-benzyloxycarboxamido-1-(2,4-dimethoxybenzyl)-4-hydroxymethyl-2-azetidinone in 80 ml of methylene chloride is added dropwise and the mixture is stirred for an hour, followed by dropwise addition of 16 g of triethylamine. The mixture is stirred for an hour, a solution of 6.7 g of methoxycarbonylmethylenetriphenylphosphorane in a mixture of 40 ml of methylene chloride and 40 ml of dimethyl sulfoxide is added dropwise, and the whole mixture is stirred for

an hour. (The above reactions are conducted under cooling at -78°C.) The solvent is then distilled off under reduced pressure and the residue is shaken with water and ethyl acetate. The organic solvent layer is separated, washed with water and dried over anhydrous sodium sulfate. The solvent is distilled off under reduced pressure and the residue is subjected to column chromatography using silica gel (240 g), elution being carried out with ethyl acetate-hexane (1: 1). The solvent is distilled off under reduced pressure and the residue is collected by addition of ether and filtration to give 7.5 g of *cis*-3-benzyloxycarboxamido-4-(2-methoxycarbonylethenyl)-1-(2,4-dimethoxybenzyl)-2-azetidinone as colorless crystals.

M.p. 116-117°C

IR (Nujol) $cm^{-1}$ : 3270, 1770, 1730, 1680.

NMR ($CDCl_3$) δ: 3.67, 3.72, 3.73 (3H, 3×s, 3×$OCH_3$), 7.23 (5H, s, ph).

| Elemental analysis for $C_{24}H_{26}N_2O_7$: | | | |
|---|---|---|---|
| | C(%) | H(%) | N(%) |
| Calcd. | 63.42 | 5.76 | 6.16 |
| Found | 63.14 | 5.57 | 6.08 |

(1) In the same method as Reference Example 1, the following compounds are produced;

*cis*-3-[2-(2-chloroacetamido-4-thiazolyl)-2-(Z)-[1-methyl-1-(p-nitrobenzyloxycarbonyl)ethoxyimino]acetamido]-4-ureidomethyl-2-azetidinone.

M.p. 145-150°C

*cis*-3-[2-(2-chloroacetamido-4-thiazolyl)-2-(Z)-[1-benzhydryloxycarbonyl-1-methylethoxy)acetamido]-4-(t-butoxycarbonylcarbazoyl)-2-azetidinone.

*cis*-3-[2-(2-chloroacetamido4-thiazolyl )-2-(Z)-[1-methyl-1-(p-nitrobenzyloxycarbonyl)ethoxyimino]-acetamido]-4-carbamoylmethylaminocarbonyl-2-azetidinone.

*cis*-3-[2-(2-chloroacetamido-4-thiazolyl)-2-(Z)-[1-methyl-1-(p-nitrobenzyloxycarbonyl)ethoxyimino]acetamido (-4-methoxymethyl-2-azetidinone.

*cis*-3-[2-(2-chloroacetamido-4-thiazolyl)-methyl-1-(p-nitrobenzyloxycarbonyl)ethoxyimino]acetamido]-4-hydroxymethyl-2-azetidinone.

M.p. 183-186°C

*cis*-3-[2-(2-chloroacetamido-4-thiazolyl)-2-(Z)-(1-methyl-1-(p-nitrobenzyloxycarbonyl)ethoxyimino]acetamido]-4-(2-methoxycarbonylethyl)-2-azetidinone.

(2) In the same method as Reference Example 3, the following compounds are produced :

*cis*-3-benzyloxycarboxamido-4-carbamoylmethyl-2-azetidinone.

*cis*-3-(t-butoxycarboxamidol-4-methylsulfonyloxymethyl-2-azetidinone.

M.p. 147-149°C (decompn.)

(3S,4S)-*cis*-3-benzyloxycarboxamido-4-carbamoyloxymethyl-2-azetidinone.

M.p. 191-192°C

*cis*-3-benzyloxycarboxamido-4-(2-methoxycarbonylethyl)-2-acetidinone.

M.p. 106.5-108°C

(3) In the same method as Reference Example 7, the following compound is produced;

(3S,4S)-*cis*-3-benzyloxycarboxamido-1-(2,4-dimethoxybenzyl)-4-hydroxymethyl-2-azetidinone.

M.p. 137-138°C

(4) In the same method as Reference Example 8, the following compound is produced ;

4-acetoxyethyl-3-azido-2-azetidinone.

(5) In the same method as Reference Example 10, the following compound is produced;

*cis*-3-[2-(2-chloroacetamido-4-thiazolyl)1-methyl-1-(p-nitrobenzyloxycarbonyl)ethoxyimino]-acetamido]-4-chloroacetamidocarbonyloxymethyl-2-azetidinone.

M.p. 203-209°C (decompn.)

Example 1

(3S,4S)-*cis*-3-Benzyloxycarboxamido-4-carbamoyloxymethyl-2-azetidinone (293 mg) is dissolved in 10 ml of dioxane, 477 mg of sulfuric anhydride-pyridine complex is added, and the mixture is stirred at room temperature for 14 hours. The dioxane is distilled off under reduced pressure and the residue is stirred with 20 ml of water and 20 ml of Dowex 50W (Na) at room temperature for an hour. The resin is then filtered off and the filtrate is concentrated under reduced pressure. The residue is subjected to chromatography on an Amberlite® XAD-2 column and elution is carried out with water, 5% ethanol and 10% ethanol in that order. The fractions containing the desired product are combined, concentrated under reduced pressure and lyophilized to give 270 mg (64%) of sodium (3S,4S)-*cis*-3-benzyloxycarboxamido-4-carbamoyloxymethyl-2-azetidinone-1-sulfonate as a colorless powder.

$[\alpha]_D^{25}$ + 29.4° (c=0.715, $H_2O$)

IR $\nu_{max}^{KBr}$ $cm^{-1}$: 3500, 3370, 3320, 1795, 1760, 1730, 1690.

NMR ($d_6$-DMSO) δ: 3.85~4.40 (3H, $C_4$-H, $C_4$-$CH_2$), 4.92 (1H, d, d, J=5, 10Hz, $C_3$-H), 6.10~6.65 (1H, $CONH_2$), 7.35 (5H, s, Ph), 7.98 (1H, d, J=10Hz, $C_3$-NH).

| Elemental analysis for $C_{13}H_{14}N_3NaO_8S \cdot 1½H_2O$: | | | |
|---|---|---|---|
| | C(%) | H(%) | N(%) |
| Calcd. | 36.97 | 4.06 | 9.95 |
| Found | 37.24 | 4.13 | 10.02 |

The above product (236 mg) is dissolved in 6.7 ml of water, 0.56 ml of 1N hydrochloric acid and 236 mg of 10% palladium-on-carbon are added thereto, and the mixture is stirred in a hydrogen atmosphere at room temperature for 40 minutes. The catalyst is then filtered off and washed with 16 ml of water. The filtrate and washings are combined and concentrated to 4 ml under reduced pressure, followed by addition of 2.24 ml of 1N hydrochloric acid. The resulting mixture is further concentrated to 1 ml and then allowed to stand in a refrigerator at 4°C overnight. The resulting colorless crystals are collected by filtration, washed with 1 ml of cold water and dried over phosphorus pentoxide under reduced pressure to give 80 mg (60%) of (3S,4S)-*cis*-3-amino-3-carbamoyloxymethyl-2-azetidinone-1-sulfonic acid.

M.p. 207-210°C (decompn.)

$[\alpha]_D^{24}$ - 62.9° (C=0.49, DMSO)

IR (KBr) $cm^{-1}$ : 3440, 3350, 3180, 1795, 1775, 1735, 1720.

NMR ($d_6$-DMSO) δ: 4.67 (1H, d, J=5Hz, $C_3$-H).

| Elemental analysis for $C_5H_9N_3O_6S$: | | | |
|---|---|---|---|
| | C(%) | H(%) | N(%) |
| Calcd. | 25.10 | 3.79 | 17.57 |
| Found | 25.02 | 3.72 | 17.73 |

Example 2

*cis*-3-Benzyloxycarboxamido-1-(2,4-dimethoxybenzyl)-4-hydroxymethyl-2-azetidinone (racemic form) is worked up in the same manner as Reference Examples 10 and 3 to give *cis*-3-benzyloxycarboxamido-4-carbamoyloxymethyl-2-azetidinone (racemic form), which, on recrystallization from acetone-ethyl acetate, melts at 210-211°C.

This product (1.45 g) is suspended in 87 ml of dioxane, 1.753 g of sulfuric anhydride-pyridine complex is added, and the mixture is stirred at room temperature for 7 hours. The dioxane is distilled off under reduced pressure and the residue is stirred with 100 ml of water and 45 ml of Dowex® 50 W (Na) for an hour. The resin is then filtered off and the filtrate is concentrated to 20 ml under reduced pressure to give a crystalline precipitate. After cooling, the crystals are collected by filtration and dried to give 1.52 g (74.5%) of sodium *cis*-3-benzyloxycarboxamido-4-carbamoyloxymethyl-2-azetidinone-1-sulfonate.

M.p. 167-169°C (decompn.)

IR (KBr) $cm^{-1}$ : 3500, 3350, 1790, 1690.

NMR ($d_6$-DMSO) δ: 4.93 (1H, d.d, J=6, 9Hz, $C_3$-H), 5.07 (2H, s, $CH_2$ph), 7.39 (5H, s, ph), 7.99 (1H, d, J=9Hz), $C_3$NH).

| Elemental analysis for $C_{13}H_{14}N_3NaO_8S \cdot H_2O$: | | | |
|---|---|---|---|
| | C(%) | H(%) | N(%) |
| Calcd. | 37.78 | 3.90 | 10.17 |
| Found | 37.81 | 3.98 | 10.07 |

Example 3

*cis*-3-[2-(2-Amino-4-thiazolyl)-2-(1-carboxy-1-methylethoxyimino)acetamido]-4-carbazoyl-2-azetidinone-1-sulfonic acid (100 mg) is dissolved in 2 ml of water and 47 mg of potassium cyanate is added at room temperature. The mixture is stirred at room temperature for an hour, then adjusted to pH 1 with 0.6 ml of 1 N hydrochloric acid under ice-cooling, and stirred further under ice-cooling for 30 minutes. The colorless crystalline precipitate is collected by filtration, washed with water, and dried over anhydrous calcium chloride under reduced pressure to give 50 mg of *cis*-3-[2-(2-amino-4-thiazolyl)-2-(Z)-(1-carboxy-1-methylethoxyimino)acetamido]-4-semicarbazidocarbonyl-2-azetidinone-1-sulfonic acid.

M.p. 218-227°C (decompn.)

IR (KBr) $cm^{-1}$ : 3280, 1770, 1710, 1680, 1635.

NMR ($d_6$-DMSO) δ: 1.50 (6H, s, 2×$CH_3$), 4.50 (1H, d, J=6Hz, $C_4$-H), 5.40 (1H, d.d, J=6, 9Hz, $C_3$-H), 7.05 (1H, s, proton at 5-position of the thiazole ring), 9.10 (1H, d, J=9Hz, $C_3$-NH).

| Elemental analysis for $C_{14}H_{18}N_8O_{10}S_2 \cdot 2H_2O$: | | | |
|---|---|---|---|
| | C(%) | H(%) | N(%) |
| Calcd. | 30.11 | 3.97 | 20.07 |
| Found | 30.29 | 3.69 | 20.09 |

Example 4

Sodium *cis*-3-benzyloxycarboxamido-4-(t-butoxycarbonylcarbazoyl)-2-azetidinone-1-sulfonate (1.1 g) is dissolved in a mixture of 40 ml of water and 20 ml of tetrahydrofuran, 600 mg of 10% palladium-on-carbon is added, and the mixture is stirred in a hydrogen atmosphere for 30 minutes. The catalyst is filtered off and the filtrate is concentrated to 10 ml under reduced pressure. Tetrahydrofuran (13 ml) is added and then 480 mg of sodium bicarbonate and 1.29 g of 2-(2-chloroacetamido-4-thiazolyl)-2-(Z)-(p-nitrobenzyloxycarbonylmethoxyimino)acetyl chloride hydrochloride are added under ice-cooling. The mixture is stirred under ice-cooling for an hour, 591 mg of sodium N-methyldithiocarbamate is added and the whole mixture is stirred at room temperature for 2 hours, washed with two 50-ml portions of ether and concentrated under reduced pressure. The residue is subjected to column chromatography on Amberlite® XAD-2 (180 ml), elution being carried out with water, 5% ethanol, 10% ethanol and 20% ethanol in that order. The fractions containing the desired product are combined and lyophilized to give 950 mg of a powder. This product is dissolved in a mixture of 20 ml of water and 20 ml of tetrahydrofuran, 950 mg of 10% palladium-on-carbon is added and the mixture is stirred in a hydrogen atmosphere at room temperature for 2 hours. The catalyst is then filtered off, 120 mg of sodium bicarbonate is added and the mixture is washed with ethyl acetate. To the aqueous layer is added 4 ml of 1 N hydrochloric acid and the mixture is concentrated under reduced pressure. The residue is subjected to column chromatography on Amberlite® XAD-2 (180 ml), elution being carried out with water, 5% ethanol and 10% ethanol in that order. The fractions containing the desired product are combined and lyophilized to give 190 mg of *cis*-3-[2-(2-amino-4-thiazolyl)-2-(Z)-carboxymethoxyiminoacetamido]-4-(t-butoxycarbonylcarbazoyl)-2-azetidinone-1-sulfonic acid.

IR (KBr) $cm^{-1}$ : 3280, 1780, 1720, 1690, 1630.

NMR ($d_6$-DMSO+$D_2O$) δ: 1.54 (9H, s, t-butyl), 4.75 (1H, d, J=6Hz, $C_4$-H, 4.90 (2H, s, $OCH_2$), 5.70 (1H, d, J=6Hz, $C_3$-H), 7.27 (1H, s, proton at 5 position of the thiazole ring).

Example 5

Sodium *cis*-3-(t-butoxycarboxamido)-4-methylsulfonyloxymethyl-2-azetidinone-1-sulfonate (396 mg) is dissolved in 15 ml of pyridine and the mixture is heated in an argon atmosphere at 110-120°C. After 2 hours, the pyridine is distilled off under reduced pressure, ether is added and the resulting precipitate is collected by filtration. This product is dissolved in water and subjected to column chromatography on Amberlite® XAD-2 (80 ml), elution being carried out

with water and 10% ethanol. The fractions containing the desired product are combined, concentrated under reduced pressure and lyophilized to give 192 mg of *cis*-3-(t-butoxycarboxamido)-4-pyridiniomethyl-2-azetidinone-1-sulfonate as a colorless powder.

IR (KBr) $cm^{-1}$: 1775, 1710, 1640.

NMR ($d_6$-DMSO) δ: 1.38 (9H, s, t-butyl), 4.83 (2H, d, J=6Hz, $C_4$-$CH_2$), 5.02 (1H, d, d, J=6, 9Hz, $C_3$-H), 7.75 (1H, d, J=9Hz, $C_3$-N H), 7.95~9.05 (5H, m, proton at 5 position of the thiazole ring).

| Elemental analysis for $C_{14}H_{19}N_3O_6S \cdot H_2O$ | | | |
|---|---|---|---|
| | C(%) | H(%) | N(%) |
| Calcd. | 44.79 | 5.64 | 11.19 |
| Found | 44.62 | 5.41 | 11.45 |

Example 6

*cis*-3-(t-Butoxycarboxamido)-4-pyridiniomethyl-2-azetidinone-1-sulfonate (179 mg) is suspended in a mixture of 1 ml of methylene chloride and 1 ml of anisole and, under ice-cooling, 5 ml of trifluoroacetic acid is added.

The mixture is stirred at the same temperature for 30 minutes and concentrated under reduced pressure, followed by addition of benzene and reconcentration. To the residue is added 7 ml of acetone and the mixture is adjusted to pH 7 with 5% aqueous sodium bicarbonate. Under ice-cooling and stirring, 400 mg of 2-(2-chloroacetamido-4-thiazolyll-2-(Z)-(1-benzhydryloxycarbonyl-1-methylethoxyimino)acetyl chloride hydrochloride is added and the mixture is stirred under ice-cooling for an hour with the pH maintained at 6-7 with aqueous sodium bicarbonate. The acetone is then distilled off under reduced pressure, whereupon a syrupy substance separates out. This product is dissolved by addition of 7 ml of acetone, followed by addition of 97 mg of sodium N-methyldithiocarbamate. The mixture is stirred at room temperature for 3 hours, with further addition of 97 mg portions of sodium N-methyldithiocarbamate at hour 1 and hour 2. The acetone is distilled off under reduced pressure to give a syrupy substance. Then, ethyl acetate is added and the aqueous layer is separated. The aqueous layer and the syrupy substance are combined, concentrated under reduced pressure and lyophilized. The resulting powder is suspended in a mixture of 5 ml of methylene chloride and 1 ml of anisole and 10 ml of trifluoroacetic acid is added dropwise under cooling at -20°C. The mixture is stirred at -20 to -10°C for an hour and concentrated under reduced pressure. The residue is adjusted to pH 5 with 5% aqueous sodium bicarbonate under ice-cooling and then shaken with water and ethyl acetate. The aqueous layer is separated and concentrated under reduced pressure, and the residue is subjected to column chromatography on Amberlite® XAD-2 (70 ml). After development with water, elution is carried out with 10% ethanol, and the eluate is concentrated under reduced pressure and lyophilized to give 99 mg of a pale yellow powder. This product (85 mg) is dissolved in a small amount of water, adjusted to pH 5 with aqueous sodium bicarbonate and subjected to column chromatography on Amberlite® XAD-2 (70 ml), elution being carried out with water. The fractions containing the desired product are combined and lyophilized to give 70 mg of monosodium *cis*-3-[2-(2-amino-4-thiazolyl)-2-(Z)-(1-carboxylato-1-methylethoxyimino)acetamido]-4-pyridiniomethyl-2-azetidinone-1-sulfonate as a pale yellow powder.

IR (KBr) $cm^{-1}$: 1770, 1660, 1635.

NMR ($d_6$-DMSO) δ: 1.35 (3H, s, $CH_3$), 1.44 (3H, s, $CH_3$), 6.71 (1H, s, proton at 5 position of the thiazole ring), 7.85~9.35 (5H, m, proton on the pyridine ring).

| Elemental analysis for: $C_{18}H_{19}N_6NaO_8S_2 \cdot 4H_2O$ | | | | |
|---|---|---|---|---|
| | C(%) | H(%) | N(%) | Na(%) |
| Calcd. | 35.64 | 4.49 | 13.86 | 3.79 |
| Found | 35.73 | 4.21 | 13.69 | 3.7 |

(Sodium content is to be determined by the atomic absorption method.)

Example 7

*cis*-4-Methylthiomethyl-3-(2,2,2trichloroethoxycarboxamido)-2-azetidinone (643 mg) is dissolved in a mixture of 50 ml of tetrahydrofuran and 25 ml of 1 N ammonium acetate, 5 g of zinc is added and the mixture is stirred at room temperature for 4 hours and then filtered. The filtrate is concentrated under reduced pressure, followed by addition of 400 ml of chloroform. The chloroform solution is dried over anhydrous magnesium sulfate and concentrated under reduced pressure. The residue is dissolved in a mixture of 10 ml of tetrahydrofuran and 10 ml of water, and 672 mg of

sodium bicarbonate is added under ice-cooling. Then, 1.19 g of 2-(2-chloroacetamido-4-thiazolyl)-2-(Z)-[1-methyl-1-(p-nitrobenzyloxycarbonyl)ethoxyimino(acetyl chloride hydrochloride is added and the whole mixture is stirred at room temperature for 3 hours and extracted with ethyl acetate. The extract is washed with 0.1 N sodium hydroxide and aqueous sodium chloride, dried over anhydrous magnesium sulfate and concentrated under reduced pressure. The residue is dissolved in 3 ml of N,N-dimethylformamide, 3.8 ml of a sulfuric anhydride-N,N-dimethylformamide complex solution (1.56 M) is added under ice-cooling, and the mixture is allowed to stand in a refrigerator at 4°C overnight. Then, 1 ml of pyridine is added, followed by addition of 100 ml of ether, whereupon crystals separate out. The supernatant is discarded and the crystals are washed with two 2-ml portions of ether and dissolved in 20 ml of water, followed by addition of 20 ml of Dowex® 50 W (Na). The mixture is stirred at room temperature for an hour and filtered, and the filtrate is concentrated under reduced pressure. The residue is subjected to column chromatography on Amberlite® XAD-2 (80 ml). After development with water, elution is carried out with 50% ethanol and the eluate is lyophilized. The resulting powder is dissolved in a mixture of 5 ml of acetonitrile and 5 ml of water, 321 mg of m-chloroperbenzoic acid is added at room temperature, and the mixture is stirred for 3 hours. The acetoniotrile is distilled off under reduced pressure and the concentrate is filtered. The filtrate is subjected to column chromatography using Amberlite® XAD-2 (60 ml). After development with water, elution is carried out with 40% ethanol and the eluate is concentrated under reduced pressure and lyophilized to give 310 mg of sodium cis-3-[2-(2-chloroacetamido-4-thiazolyl)-2-(Z)-[1-methyl-1-(p-nitrobenzyloxycarbonyl)ethoxyimino]acetamido]-4-methylsulfonylmethyl-2-azetidinone-1-sulfonate.

IR (KBr) $cm^{-1}$ : 3450, 1770, 1740, 1680.

NMR ($d_6$-DMSO) δ: 1.55 (6H, s, 2 × $CH_3$), 3.00 (3H, s, $SO_2CH_3$), 4.35(2H, s, $ClCH_2$), 5.31 (2H, s, $CO_2$ $CH_2$), 7.40 (1H, proton at 5 position of the thiazole ring), 9.40 (1H, d, J=10Hz, $C_3$-NH).

Example 8

*cis*-3-Benzyloxycarboxamido-4-carbazoyl-2-azetidinone-1-sulfonic acid (140 mg) is suspended in 30 ml of water and 125 mg of potassium cyanate is added at room temperature. The mixture is stirred at room temperature for 2 hours and concentrated under reduced pressure, and the residue is subjected to column chromatography using Amberlite® XAD-2 (60 ml), elution being carried out with water and 5% ethanol. The fractions containing the desired product are lyophilized to give 213 mg of potassium *cis*-3-benzyloxycarboxamido-4-carbamoylcarbazoyl-2-azetidinone-1-sulfonate.

IR (KBr) $cm^{-1}$ : 3300, 1770, 1695 (br.)

NMR ($d_6$-DMSO) δ: 4.45 (1H, d, J=6Hz, $C_4$-H), 7.38 (5H, s, ph), 7.60 (1H, d, J=9Hz, $C_3$-NH).

| Elemental analysis for: $C_{13}H_{15}KN_5O_8S \cdot ½H_2O$ | | | |
|---|---|---|---|
| | C(%) | H(%) | N(%) |
| Calcd. | 34.74 | 3.58 | 15.58 |
| Found | 35.08 | 3.71 | 15.40 |

Example 9

Sodium 4-acetoxyethyl-3-azido-2-azetidinone-1-sulfonate (150 mg, 1:4 cis-trans mixture) as obtained in Reference Example 27 is dissolved in 5 ml of dried N,N-dimethylformamide and catalytic reduction is carried out using 100 mg of 10% palladium-on-carbon (wet). The catalyst is then filtered off, 203 mg of 2-(2-chloroacetamido-4-thiazolyl)-2-(Z)-(1-t-butyloxycarbonyl-1-methylethoxyimino)acetic acid, 77 mg of hydroxybenzotriazole and 103 mg of dicyclohexylcarbodiimide are added under ice-cooling, and the mixture is stirred for 1.5 hours. Stirring is then contionued at room temperature for 18 hours, followed by addition of 20 ml of water. The insoluble matter is filtered off and washed with 15 ml of water. The filtrate and washings are combined, 260 mg of sodium N-methyldithiocarbamate is added thereto, and the mixture is stirred at room temperature for 2.5 hours. The reaction mixture is subjected to column chromatography using Amberlite® XAD-2 (ϕ 4 × 22 cm) and elution is carried out with water, 10% ethanol and 30% ethanol in that order. The fractions containing the desired product are lyophilized to give 66.5 mg of sodium 3-[2-(2-amino-4-thiazolyl)-2-(Z)-(1-t-butyloxycarbonyl-1-methylethoxyiminolacetamido]-4-acetoxyethyl-2-azetidinone-1-sulfonate as a colorless powder.

IR (KBr) $cm^{-1}$ :3400,1750,1725,1660,1620,1530,1240, 1050.

The above product (66.5 mg) is dissolved in 2 ml of trifluoroacetic acid and the solution is stirred under ice-cooling for 3 hours and concentrated to dryness. The concentrate is dissolved in 5 ml of water and the solution is stirred with 6 ml of Dowex® 50 W (H-form) at room temperature for 15 minutes. The resin is filtered off and washed with two 5-ml portions of water. The filtrate and washings are combined, concentrated to about 10 ml under reduced pressure

and subjected to column chromatography using Amberlite® XAD-2 (ϕ 3 × 28 cm), elution being carried out with water and 10% ethanol in that order. The fractions containing the desired product are lyophilized to give 32 mg of 3-[2-amino-4-thiazolyl)-2-(Z)-(1-carboxy-1-methylethoxyimino)acetamido(-4-acetoxyethyl-2-azetidinone-1-sulfonic acid disodium salt (1:4 *cis-trans* mixture) as a colorless powder.

IR (KBr) $cm^{-1}$ : 3400, 1760, 1720, 1600, 1240, 1050.

NMR ($D_2O$) δ: 1.69 (6H, s, 2 × $CH_3$), 2.20 (3H, s, $COCH_3$), 4.43 (3H, m, $CH_2O$ + $C_4$-H), 5.05 (4/5 H, d, J=3Hz, $C_3$-H, 5.53 ($^1/_5$H, d, J=6Hz, $C_3$-H), 7.05 ($^1/_5$H, s, thiazol 5-H), 7.10 ($^4/_5$H, s, thiazol 5-H).

| Elemental analysis for: $C_{16}H_{19}N_5Na_2O_{10}S_2 \cdot 4.5H_2O$ | | | |
|---|---|---|---|
| | C(%) | H(%) | N(%) |
| Calcd. | 30.38 | 4.46 | 11.09 |
| Found | 30.44 | 4.25 | 10.45 |

Example 10

Production of sodium [3S, 4S]-trans-3-[[2-(2-aminothiazol-4-yl)-2-(Z)-methoxyimino]acetamido]-4-[3,5-dimethylisoxazol-4-yl)-2-azetidinone-1-sulfonate

[3S, 4S]-trans-4-(1-Acetyl-2-oxopropyl)-3-phthalimido-2-azetidinone (7.0 g) is dissolved in a mixture of 5 ml of dimethylformamide and 20 ml of acetic acid, 3.2 g of hydroxylamine hydrochloride is added, and the mixture is stirred at room temperature for 4 hours. Then, an additional 3.2 g of hydroxylamine hydrochloride is added and the whole mixture is stirred for 2 hours. To the reaction mixture is added 500 ml of water and the resulting mixture is extracted with 500 ml of methylene chloride. The extract is washed with water, dried over anhydrous magnesium sulfate and concentrated. The residue is subjected to silica gel column chromatography using ethyl acetate-methylene chloride (1:1) as the developing solvent. The fractions containing the desired product are concentrated to dryness to give 3.5 g of (3S, 4S]-trans-4-(3,5-dimethylisoxazol-4-yl)-3-phthalimido-2-azetidinone.

M.p. 218-220°C

$[\alpha]_D^{23}$= - 123.9° (c=0.875, chloroform).

b) [3S, 4S]-trans-4-(3,5-Dimethylisoxazol-4-yl)-3-phthalimido-2-azetidinone (3.1 g) is dissolved in 20 ml of dimethylformamide, 4.6 g of t-butyl-chlorodimethylsilane is added, and the mixture is stirred under ice-cooling. Thereto is added dropwise 4 ml of triethylamine and the whole mixture is stirred for an hour, and poured into 200 ml of ice water. The resulting mixture is extracted with 200 ml of ethyl ether and the extract is washed with water, dried over anhydrous magnesium sulfate, and concentrated to dryness under reduced pressure. The residue is recrystallized from ethyl ether-petroleum ether (1:1) to give 3.8 g of [3S,4S]-1 -(t-butyl-dimethyl)-silyl-4-(3,5-dimethylisoxazol-4-yl)-3-phthalimido-2-azetidinone.

M.p. 149-151°C

$[\alpha]_D^{23}$ = -163.4· (c=0.905, chloroform).

c) [3S, 4S]-*trans*-4-(3,5-Dimethylisoxazol-4-yl)-3-phthalimido-2-azetidinone (2.0 g) is dissolved in 20 ml of 1 N methylhydrazine-in-dimethoxyethane, and the solution is stirred at room temperature for an hour and concentrated to dryness under reduced pressure. The residue is dissolved in 20 ml of methylene chloride, and the solution is stirred at room temperature for 5 hours and then allowed to stand in a refrigerator overnight. The insoluble matter is filtered off and the filtrate is concentrated to dryness under reduced pressure. The residue is dissolved in a mixture of 20 ml of tetrahydrofuran and 20 ml of water and the solution is stirred under ice-cooling. To this solution are added 1.65 of sodium hydrogen carbonate and 2.4 g of 2-(2-chloroacetamidothiazol-4-yl)-2-(Z)-methoxyiminoacetyl chloride hydrochloride, and the mixture is stirred for 20 minutes and then extracted with 200 ml of ethyl acetate. The organic layer is dried over anhydrous magnesium sulfate and concentrated. The residue is subjected to silica gel column chromatography using ethyl acetate as the developing solvent. The collected fractions are concentrated to dryness to give 1.5 g of [3S,4S]-*trans*-1-(t-butyl-dimethyl)silyl-3-[[2-(2-chloroacetamidothiazol-4-yl)-2-(Z)-methoxyimino]acetamido]-4-(3,5-dimethylisoxazol-4-yl)-2-azetidinone.

M.p. 145-147°C (decompn.)

$[\alpha]_D^{23}$ = -24.6° (c=1, chloroform)

d) [3S,4S]-*trans*-1-(t-Butyl-dimethyl)silyl-3-[[2-(2-chloroacetamidothiazol-4-yl)-2-(Z)-methoxyimino]-acetamido)-4-(3,5-dimethylisoxazol-4-yl)-2-azetidinone (1.2 g) is dissolved in 30 ml of methanol, 160 mg of potassium fluoride is added, and the mixture is stirred at room temperature for an hour. The reaction mixture is concentrated to about half of its original volume under reduced pressure, followed by addition 30 ml of water. The resulting crystalline precipitate is collected by filtration to give 0.5 g of [3S,4S]-*trans*-3-[(2 - (2 - chloroacetamidothiazol - 4 - yl) - 2 - (Z) - methoxyimino]

acetamido - 4 - (3,5 - dimethylisoxazol - 4 - yl) - 2 - azetidinone.

M.p. 258-260°C

e) A solution of 430 mg of [3S-4S)-*trans*-3-[[2-(2-chloroacetamidothiazol-4-yl)-2-(Z)-methoxyimino]-acetamido-4-(3,5-dimethoxylisoxazol-4-yl)-2-azetidinone in 2 ml of dimethylformamide is stirred at -70°C, and 8 ml of 1.25 M sulfuric anhydride-dimethylformamide complex-in-dimethylformamide is added thereto.

The reaction is allowed to proceed at 0°C for 2 hours and then in a refrigerator for 4 hours. The reaction mixture is cooled again to -70°C, 2.8 g of sodium hydrogen carbonate is added and the mixture is washed with two 50 ml portions of ethyl ether by decantation. The insoluble matter is dissolved in water and subjected to column chromatography on Amberlite® XAD-2 using water and 10% methanol as the developing solvents. The fractions containing the desired product are combined and lyophilized to give 370 mg of sodium [3S,4S]-*trans*-3-[[2-(2-chloroacetamidothiazol-4-yl)-2-(Z)-methoxyimino]acetamido-4-(3,5-dimethylisoxazol4-yl)-2-azetidinone-1-sulfonate.

$[\alpha]_D^{22}$ = -20.7° (c=0.145, water)

f) Sodium [3S,4S]-*trans*-3-[[2-(2-chloroacetamidothiazol-4-yl)-2-(Z)-methoxyimino]acetamido]-4-(3,5-dimethylisoxazol-4-yl)-2-azetidinone-1-sulfonate (300 mg) is dissolved in 3 ml of water, 125 mg of sodium methyldithiocarbamate is added, and the mixture is stirred at room temperature for an hour and then shaken with 5 ml of ethyl acetate. The aqueous layer is separated and subjected to column chromatography on Amberlite® XAD-2 using water as the developing solvent. The fractions containing the desired product are combined and lyophilised to give 200 mg of sodium (3S,4S]-*trans*-3-[[2-(2-aminothiazol-4-yl)-2-(Z)-methoxyimino]acetamido]-4-(3,5-dimethylisoxazol-4-yl)-2-azetidinone-1-sulfonate.

$[\alpha]_D^{22}$ = -11.8° (C=0.245, water).

IR (KBr) $cm^{-1}$: 1770, 1665, 1630, 1530, 1280, 1050.

NMR($D_2$) δ: 2.36 (3H, s, $CH_3$), 2.48 (3H, s, $CH_3$), 4.00 (3H, s, $OCH_3$), 5.04 (1H, d, J=2Hz, $C_4$-H), 4.10 (1H, d, J=2Hz, $C_3$-H), 6.96 (1H, s, proton at 5 position of the thiazole ring).

| Elemental analysis for: $C_{14}H_{15}N_6N_9O_7S_2{\cdot}5/2H_2O$ | | | |
|---|---|---|---|
| | C (%) | H (%) | N (%) |
| Calcd. : | 32.88 | 3.94 | 16.44 |
| Found: | 32.72 | 3.95 | 16.35 |

In the same methods as the above Examples, the following compounds are produced:

Sodium *cis*-3-[2-(2-chloroacetamido-4-thiazolyl)-2-(Z-(p-nitrobenzyloxycarbonylmethoxyimino)-acetamido]-4-methylcarbamoyl-2-azetidinone-1-sulfonate.

Sodium *cis*-3-[2-(2-amino-4-thiazolyl )-2-(Z)-(p-nitrobenzyloxycarbonylmethoxyiminolacetamido]-4-methylcarbamoyl-2-azetidinone-1-sulfonate.

*cis*-3-[2-(2-Amino-4-thiazolyl )-2-(Z)-carboxymethoxyiminoacetamido]-4-methylcarbamoyl-2-azetidinone-1-sulfonic acid.

*cis*-3-[2-(2-Amino-4-thiazolyl)-2-(Z)-(1-carboxy-1-methylethoxyimino)acetamido]-4-ureidomethyl-2-azetidinone-1-sulfonic acid.

*cis*-3-[2-(2-Amino-4-thiazolyl)-2-(Z)-(1-carboxy-1-methylethoxyimino)acetamido]-4-(N-sulfocarbamoyl)aminomethyl-2-azetidinone-1-sulfonic acid trisodium salt.

Sodium *cis*-3-benzyloxycarboxamido-4-(t-butoxycarbonylcarbazoyl)-2-azetidinone-1-sulfonate.

*cis*-3-[2-(2-Amino-4-thiazolyl)-2-(Z)-carboxymethoxyiminoacetamido]-4-semicarbazidocarbonyl-2-azetidinone-1-sulfonic acid.

*cis*-3-[2-(2-Amino-4-thiazolyl)-2-(Z)-(1-carboxy-1-methylethoxyimino)acetamido]-4-carbamoylmethylaminocarbonyl-2-azetidinone-1-sulfonic acid.

Sodium *cis*-4-carbamoyl-3-(D-2-(6,7-dihydroxychromone-3-carboxamido)-2-(4-hydroxyphenyl)acetamido]-2-azetidinone-1-sulfonate.

Sodium *cis*-3-(t-butoxycarboxamido)-4-methylsulfonyloxymethyl-2-azetidinone-1-sulfonate.

*cis*-3-[2-(2-Amino-4-thiazolyl)-2-(Z)-(1-carboxy-1-methylethoxyimino)acetamido]-4-methylsulfinylmethyl-2-azetidinone-1-sulfonic acid (a mixture of the α-sulfoxide compound and β-sulfoxide compound).

*cis*-3-[2-(2-Amino-4-thiazolyl)-2-(Z)-(1-carboxy-1-methylethoxyimino)acetamido]-4-methylsulfonylmethyl-2-azetidinone-1-sulfonic acid.

Sodium *cis*-3-[2-(2-chloroacetamido-4-thiazolyl)-2-(Z)-[1-methyl-1-(p-nitrobenzyloxycarbonyl)ethoxyimino] acetamido]-4-chloroacetoxymethyl-2-azetidinone-1-sulfonate.

*cis*-3-[2-(2-Amino-4-thiazolyl )-2-(Z)-1-carboxy-1-methylethoxyimino)acetamido]-4-hydroxymethyl-2-azetidinone-1-sulfonic acid.

*cis*-3-[2-(2-Amino-4-thiazolyl)-2-(Z)-[1-methyl-1-(p-nitrobenzyloxycarbonyl )ethoxyimino]acetamido-4-sulfaminocarbonyloxymethyl-2-azetidinone-1-sulfonic acid disodium salt.

*cis*-3-[2-(2-Amino-4-thiazolyl)-2-(Z)-(1-carboxy-1-methylethoxyimino)acetamido]-4-sulfaminocarbonyloxymethyl-2-azetidinone-1-sulfonic acid trisodium salt.

Sodium *cis*-3-[2-(2-chloroacetamido-4-thiazolyl)-2-(Z)-[1-methy)-1-(p-nitrobenzyloxycarbonyl)ethoxyimino] acetamido]-4-chloroacetamidocarbonyloxymethyl-2-azetidinone-1-sulfonate.

Sodium *cis*-[3-(2-chloroacetamido-4-thiazolyl-2-(Z)-[1-methyl-1-(p-nitrobenzyloxycarbonyl)ethoxyimino] acetamido]-4-(2-methoxycarbonylethyl)-2-azetidinone-1-sulfonate.

*cis*-3-[2-(2-Amino-4-thiazolyl)-2-(Z)-(1-carboxy-1-methylethoxyimino)acetamido]-4-(2-methoxycarbonylethyl)-2-azetidinone-1-sulfonic acid.

*cis*-3-[2-(2-Amino-4-thiazolyl)-2-(Z)-(1-carboxy-1-methylethoxyimino)acetamido]-4-carboxy-2-azetidinone-1-sulfonic acid.

Sodium [3S, 4S]-3-[[2-(2-aminothiazol-4-yl)-2-(Z)-methoxyimino]acetamido]-4-(2-(E)-methoxyiminopropyl)-2-azetidinone-1-sulfonate.

Sodium [3S, 45]-3-[[2-(2-aminothiazol-4-yl-2-(Z)-methoxyimino]acetamido]-4-(2-(Z)-methoxyiminopropyl(-2-azetidinone-1-sulfonate.

Sodium [3S, 4S]-3-[[2-(2-aminothiazol-4-yl)-2-(Z)-(1-carboxy-1-methylethoxyimino)acetamido]-4-propyl-2-azetidinone-1-sulfonate.

Sodium [3S, 4S]-4-(1-acetyl-3,5-dimethylpyrazol-4-yl)-3-[[2-(2-aminothiazol-4-yl)-2-(Z)-methoxyimino]acetamido]-1-sulfonate.

*cis*-3-[2-(2-Amino-4-thiazolyl)-2-(Z)-carboxymethoxyiminoacetamido]-4-formamidomethyl-2-azetidinone-1-sulfonic acid.

The following Test Example, Examples and Reference Examples will illustrate the aspect of the compounds [III]' of the invention in more detail. However, they are given only for the purpose of illustration. They are by no means limitative of the present invention. Modifications may be made without departing from the scope of this aspect of the invention.

The column chromatographic elution procedure in any of the Examples and Reference Examples was carried out, unless otherwise stated, under observation by TLC (thin layer chromatography). In the TLC observation, Merck $60F_{254}$ TLC plates and the same solvent systems as used in the column chromatographic elution were used with a UV detector for detection. The spot on the TLC plate was sprayed with 48% hydrobromic acid, heated for hydrolysis and then sprayed with the ninhydrin reagent. Upon reheating, the spot turned red to reddish violet. Using this phenomenon as an additional detection method, the eluate fractions containing the desired product were identified and collected. When two or more solvent systems were used as the developing solvents, unless otherwise stated, the solvent used first was for elution of the byproduct and the solvent used subsequently was for elution of the desired product. In the column chromatographic purification using Amberlite®, water and aqueous ethanol solutions with gradually increasing ethanol concentrations were used in that order as the developing solvents unless otherwise stated in the relevant Examples and Reference Examples. Unless otherwise stated, anhydrous sodium sulfate was used as the desiccant in drying the solution containing the desired product.

"Amberlite"® is a product of Rohm & Haas Co., U.S.A. and "Dowex"® is product of Dow Chemical Co. The NMR spectrometry was performed using an EM 390 (90 MHz) or T 60 (60 MHz) spectrometer with tetramethylsilane as the internal or external standard, and the total δ values were given in ppm. In the Examples and Reference Examples, the symbols have the meanings respectively given in the following:

| | |
|---|---|
| s: | singlet |
| d: | doublet |
| q: | quartet |
| ABq : | AB type quartet |
| d.d: | double doublet |
| m: | multiplet |
| sh: | shoulder |
| br.: | broad |
| J: | coupling constant |
| Hz: | Hertz |
| mg: | milligram(s) |
| g: | gram(s) |
| ml: | milliliter(s) |
| Ph: | phenyl |
| MeOH: | methanol |

$CHCl_3$: chloroform
DMSO: dimethyl sulfoxide

Test Example

For the products respectively obtained in Example 2' and Example 4', the MIC values (mcg/ml) were determined by the method mentioned below and the results are shown below in the table.

Method

The MIC values for the test compounds were determined by the agar dilution method. Thus, 1.0 ml of each serially diluted aqueous solution of each test compound was poured into a petri dish. Then, 9.0 ml of Trypticase soy agar was added and mixed with the aqueous solution. A suspension of the test organism (about $10^6$ CFU/ml) was smeared onto the mixed agar plate and incubated overnight at 37°C. The lowest concentration of the test compound that completely inhibited the growth of the test organism was reported as the minimal inhibitory concentration (MIC).

Test organisms

(1) Enterobacter cloacae IFO 12937
(2) Klebsiella pneumoniae TN 1711
(3) Pseudomonas aeruginosa GN 3407

Results

| | (mcg/ml) | | |
|---|---|---|---|
| | Test organism | | |
| Test compound | (1) | (2) | (3) |
| Example 2' | 0.05 | 0.05 | 1.56 |
| Example 4' | 0.39 | 0.1 | 6.25 |

Reference Example 1'

To 600 ml of acetonitrile are added 23.54 g of *cis*-3-amino-4-methoxycarboxyl-1-(2,4-dimethoxybenzyl)-2-azetidinone and 16.17 g of di-(p-toluoyl)-D-tartaric acid monohydrate and the mixture is warmed for dissolution. The solution is filtered and allowed to cool. The crystalline precipitate is collected by filtration and washed with cooled acetonitrile to give 20.3 of the salt, which is recrystallized from 300 ml of acetonitrile. The above procedure gives 16.3 g of the salt, melting at 165-168°C.
$[\alpha]_D^{22}$ + 71.9° (c=0.985, MeOH)

The above salt is dissolved in a mixture of 100 ml of water and 200 ml of tetrahydrofuran and, after addition of 6.1 g of sodium hydrogen carbonate, 4.2 ml of carbobenzoxy chloride is added dropwise under ice-cooling and stirring. The mixture is stirred under ice-cooling for an hour and then at room temperature for an hour. Tetrahydrofuran is distilled off under reduced pressure at 30°C or below (bath temperature). The residue is shaken with 400 ml of ethyl acetate and 200 ml of water and the aqueous layer is reextracted with 200 ml of ethyl acetate. The extracts are combined and washed twice with 2% aqueous sodium bicarbonate. The solution is further washed with aqueous sodium chloride, 1 N hydrochloric acid and aqueous sodium chloride in that order and dried. The solvent is then distilled off under reduced pressure and 30 ml of ether is added to the residue. The crystalline precipitate is collected by filtration and dissolved in 50 ml of ethyl acetate with warming, and the solution is filtered. To the filtrate is added 50 ml of hexane and the mixture is allowed to cool. The resulting colorless crystals are recovered by filtration to give 6.45 g (37.5%) of (3S, 4S)-*cis*-3-benzyloxycarboxamido-4-methoxycarbonyl-1-(2,4-dimethoxybenzyl)-2-azetidinone, melting at 120-121°C.
$[\alpha]_D^{22}$ + 24.4° (c=1.08, $CHCl_3$)

IRv $^{Nujol}_{max}$ $cm^{-1}$ : 3300, 1770, 1745, 1695.

| Elemental analysis: $C_{22}H_{24}N_2O_7$ | | | |
|---|---|---|---|
| | C(%) | H(%) | N(%) |
| Calcd.: | 61.67 | 5.65 | 6.54 |
| Found: | 61.50 | 5.59 | 6.37 |

Reference Example 2'

In 300 ml of tetrahydrofuran is dissolved 12.8 g of (3S,4S)-*cis*-3-benzyloxycarboxamido-4-methoxycarbonyl-1-(2,4-dimethoxybenzyl)-2-azetidinone and, under ice-cooling and stirring, a solution of 2.8 g of sodium borohydride in 150 ml of ice water is added dropwise over 10 minutes. After completion of the addition, the mixture is stirred under ice-cooling for an hour and at room temperature for 3 hours. Tetrahydrofuran is distilled off under reduced pressure at 30°C or below (bath temperature] and water is added to the residue. The mixture is filtered and the solid is washed with water and ethyl acetate to give 4.4 g of the crude crystalline product. The mother liquor and washings are combined and shaken. Thereafter the ethyl acetate layer is separated. The aqueous layer is reextracted with ethyl acetate. The ethyl acetate layers thus obtained are combined and washed with 1 N hydrochloric acid and aqueous sodium chloride, dried over anhydrous sodium sulfate, and concentrated under reduced pressure. The residue and the crystals previously obtained are combined and recrystallized from ethyl acetate to give 9.1 g (76%) of (3S, 4S)-*cis*-3-benzyloxycarboxamido-1-(2,4-dimethoxybenzyl)-4-hydroxymethyl-2-azetidinone as colorless crystals, melting at 137-138°C.

$[\alpha]^{25}_{D}$ - 32.7° (c=1, $CHCl_3$)

IR v $^{Nujol}_{max}$ $cm^{-1}$:

NMR ($CDCl_3$) δ: 3.5~3.9 (3H, $C_4$-H, $C_4$-$CH_2$), 3.78 (3H, s, $OCH_3$), 3.79 (3H, s, $OCH_3$), 4.35 (2H, s, $N_1$-$CH_2$), 4.9~5.2 (1H, m, $C_3$-H),

5.07(2H, s, $CH_2$ 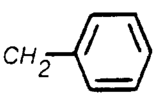 ),

6.06 (1H, d, J=10Hz, $C_3$-NH), 6.3~6.6 (2H, m, aromatic protons), 7.1~7.3 (1H, m, aromatic protons),

7.32(5H, s, 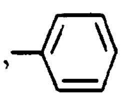 )

| Elemental analysis: $C_{21}H_{24}N_2O_6$ | | | |
|---|---|---|---|
| | C (%) | H (%) | N (%) |
| Calcd. | 62.99 | 6.04 | 7.00 |
| Found | 62.92 | 5.90 | 7.03 |

Reference Example 3'

In 40 ml of methylene chloride is dissolved 2.0 g of (3S, 4S)-*cis*-3-benzyloxycarboxamido-1-(2,4-dimethoxybenzyl)-4-hydroxymethyl-2-azetidinone and, under ice-cooling and stirring, 0.52 ml of chlorosulfonyl isocyanate is added. The mixture is stirred under ice-cooling for 30 minutes and, after addition of 0.35 ml of chlorosulfonyl isocyanate, the mixture is stirred for further 10 minutes. Then under ice-cooling, a solution of 1.26 g of sodium sulfite in 30 ml of water is added to the reaction mixture and the whole mixture is stirred at room temperature for an hour. The methylene chloride is distilled off under reduced pressure and the concentrate is extracted with chloroform. The extract is washed with aqueous sodium chloride and dried over anhydrous magnesium sulfate. The solvent is then distilled off under reduced pressure and ether is added to the residue. The mixture is filtered to give 2.46 g of the crude crystalline product, which is recrystallized from ethyl acetate-hexane to give 1.72 g (77.7%) of (3S, 4S(-*cis*-3-benzyloxycarboxamido-4-carbamoyloxymethyl-1-(2,4-dimethoxybenzyl)-2-azetidinone as colorless crystals, melting at 173-180°C.

$[\alpha]_D^{24.5}$ + 34.5° (c=0.8, DMSO)

IR $\nu_{max}^{KBr}$ $cm^{-1}$: 3410, 3300, 1760, 1710.

NMR ($d_6$-DMSO) δ: 3.74 (3H, s, $OCH_3$), 3.76 (3H, s, $OCH_3$), 3.7~4.3 (3H, m, $C_4$-H, $C_4$-$CH_2$), 4.20 (2H, ABq, J=15Hz, $N_1$-$CH_2$), 4.92 (1H, d.d, J=5, 10Hz, $C_3$-H), 5.05 (2H, s, $CH_2$ph), 7.35 (5H, s, ph), 7.87 (1H, d, J=10Hz, $C_3$-NH)

| Elemental analysis: $C_{22}H_{25}N_3O_7$ | | | |
|---|---|---|---|
| | C(%) | H(%) | N(%) |
| Calcd. | 59.59 | 5.68 | 9.48 |
| Found | 59.30 | 5.70 | 9.57 |

Reference Example 4'

In a mixture of 36 ml of acetonitrile and 18 ml of water are suspended 1.60 g of (3S, 4S)-*cis*-3-benzyloxycarboxamido-4-carbamoyloxymethyl-1-(2,4-dimethoxybenzyl)-2-azetidinone, 1.41 g of potassium persulfate and 0.85 g of dipotassium phosphate, and the suspension is stirred in an argon atmosphere at 95°C (bath temperature) for 80 minutes. The acetonitrile is distilled off under reduced pressure and 10 ml of aqueous sodium chloride is added to the residue. The mixture is extracted with ethyl acetate-tetrahydrofuran and the extract is washed with 5% aqueous sodium bicarbonate and aqueous sodium chloride and dried over anhydrous magnesium sulfate. The solvent is then distilled off under reduced pressure and the solid residue is recrystallized from ethyl acetate to give 426 mg (40.3%) of (3S, 4S)-*cis*-3-benzyloxycarboxamido-4-carbamoyloxymethyl-2-azetidinone as light-yellow crystals. The mother liquor is concentrated and purified by silica gel column chromatography (silica gel, 70 g; eluent: $CHCl_3$-MeOH-ethyl acetate (85:10:5)] to give 353 mg of colorless crystals as a further crop.

Total yield: 779 mg (73.6%)

m.p.: 191-192°C

$[\alpha]_D^{25}$ + 60.6° (c=1, MeOH)

IR $\nu_{max}^{KBr}$ $cm^{-1}$ : 3400, 3300, 1755, (sh), 1745, 1695.

NMR ($d_6$-DMSO) δ: 3.70~4.25 (3H, $C_4$-H, $C_4$-$CH_2$), 4.95 (1H, d.d, J=5, 10Hz, $C_3$-H), 5.05 (2H, s, *$CH_2$*ph), 6.47 (2H, br.s, $CONH_2$), 7.33 (5H, s, ph), 7.92 (1H, d, J=10Hz, $C_3$-NH), 8.30 (1H, br.s, $N_1$-H)

| Elemental analysis: $C_{13}H_{15}N_3O_5$ | | | |
|---|---|---|---|
| | C(%) | H(%) | N(%) |
| Calcd. | 53.23 | 5.15 | 14.32 |
| Found | 52.83 | 5.02 | 14.26 |

Reference Example 5'

In 10 ml of dioxane is dissolved 293 mg of (3S, 4S)-*cis*-3-benzyloxycarboxamido-4-carbamoyloxymethyl-2-azetidinone and, after addition of 477 mg of sulfuric anhydride-pyridine complex, the mixture is stirred at room temperature for 14 hours. Dioxane is distilled off under reduced pressure and the residue is stirred with 20 ml of water and 20 ml of Dowex® 50W (Na) at room temperature for an hour. The resin is filtered off and the filtrate is concentrated under reduced pressure. The residue is chromatographed on an Amberlite® XAD-2 column, elution being carried out with water, 5% ethanol and 10% ethanol in that order. The fractions containing the desired product are combined, concentrated under reduced pressure, and lyophilized to give 270 mg (64%) of sodium (3S, 4S)-*cis*-3-benzyloxycarboxamido-4-carbamoyloxymethyl-2-azetidinone-1-sulfonate as a colorless powder.

$[\alpha]_D^{25}$ + 29.4° (c=0.715, $H_2O$)

IR $\nu_{max}^{KBr}$ $cm^{-1}$ : 3500, 3370, 3320, 1795, 1760, 1730, 1690

NMR ($d_6$-DMSO) δ: 3.85 ~ 4.40 (3H, $C_4$-H, $C_4$-$CH_2$), 4.92 (1H, d.d, J=5, 10Hz, $C_3$-H), 6.10 ~ 6.65 (1H, $CONH_2$), 7.35 (5H, s, ph), 7.98 (1H, d, J=10Hz, $C_3$-NH)

| Elemental analysis: $C_{13}H_{14}N_3NaO_8S \cdot 1\frac{1}{2}H_2O$ | | | |
|---|---|---|---|
| | C(%) | H(%) | N(%) |
| Calcd. | 36.97 | 4.06 | 9.95 |
| Found | 37.24 | 4.13 | 10.02 |

Reference Example 6'

In 50 ml of methanol is dissolved 674 mg of (3S,4S)-*cis*-3-benzyloxycarboxamido-4-carbamoyloxymethyl-2-azetidinone and, after addition of 300 mg of 5% palladium-on-carbon, the mixture is stirred in a hydrogen atmosphere at room temperature for 30 minutes. The catalyst is then filtered off and the filtrate is concentrated under reduced pressure. The residue is dissolved in 20 ml of a 1:1 mixture of tetrahydrofuran and water and, under ice-cooling and stirring, 504 mg of sodium bicarbonate is added. Then, 1.62 g of 2-(2-chloroacetamido4-thiazolyl)-(Z)-2-(1-methyl-1 (p-nitrobenzyloxycarbonyl)ethoxyimino)acetyl chloride hydrochloride is added and the mixture is stirred under ice-cooling for 30 minutes. To the reaction mixture is added 10 ml of aqueous sodium chloride and the mixture is extracted with ethyl acetate. The extract is washed with aqueous sodium bicarbonate and aqueous sodium chloride and dried over anhydrous magnesium sulfate. The solvent is then distilled off under reduced pressure and the residue is purified by silica gel column chromatography (silica gel, 60 g; eluent: ethyl acetate). The fractions containing the desired product are combined and the solvent is distilled off under reduced pressure. Ether is added to the residue and the colorless solid precipitate is collected by filtration to give 1.25 g (87%) of (3S,4S)-*cis*-3-[2-(2-chloroacetamido4-thiazolyl)-(Z)-2-[1-methyl-1-(4-nitrobenzyloxy-carbonyl)ethoxyiminoacetamido]-4-carbamoyloxymethyl-2-azetidinone, melting at 190-195°C (decompn.).

$[\alpha]_D^{25}$ + 34.5° (c=0.1435, MeOH)

IR $\nu_{max}^{KBr}$ $cm^{-1}$ : 3450, 3300, 1753, 1740 (sh), 1690, 1660

NMR ($d_6$-DMSO) δ:1.51 (3H, s, $CH_3$), 1.53 (3H, s, $CH_3$), 3.8 ~ 4.4 (3H, $C_4$-H, $C_4$-$CH_2$), 4.37 (2H, s, $ClCH_2$), 5.2 ~ 5.45 (3H, $C_3$-H, $CH_2$ph), 6.53 (2H, br.s, $CONH_2$), 7.38 (1H, s, proton at position of the thiazole ring), 7.62 (2H, d, J=8Hz, aromatic protons), 8.07 (2H, d, J=8Hz, aromatic protons), 8.50 (1H, br.s, $N_1$-H), 9.23 (1H, d, J=9Hz, $C_3$-NH)

Example 1'

In a mixture of 10 ml of water and 10 ml of tetrahydrofuran is dissolved 422 mg of sodium (3S,4S)-*cis*-3-benzyloxycarboxamido-4-carbamoyloxymethyl-2-azetidinone-1-sulfonate and, after addition of 422 mg of 10% palladium-on-carbon, the mixture is stirred in a hydrogen atmosphere at room temperature for an hour. The catalyst is then filtered off and washed with 30 ml of a 1:1 mixture of water and tetrahydrofuran. The filtrate and washings are combined and, under ice-cooling and stirring, 202 mg of sodium bicarbonate is added. Then, 614 mg of 2-(2-chloroacetamido-4-thiazolyl)-(Z)-2-(p-nitro-benzyloxycarbonylmethoxyimino)acetyl chloride hydrochloride is added and the mixture is stirred under ice-cooling for 30 minutes, then adjusted to pH 5 with 1 N hydrochloric acid, and concentrated to 30 ml under reduced pressure. To the residue is added 10 ml of tetrahydrofuran and, after addition of 129 mg of sodium N-methyldithiocarbamate, the mixture is stirred at room temperature. After 40 and 80 minutes, 129 mg portions of sodium N-methyldithiocarbamate are added respectively. Stirring is thus continued for 2 hours in total. Tetrahydrofuran is then distilled off under reduced pressure and the residual aqueous solution is washed with ether and concentrated again under reduced pressure. The concentrate is chromatographed on a column of Amberlite® XAD-2 (200 ml), elution being carried out with water, 5% ethanol, 10% ethanol, 15% ethanol and 20% ethanol in that order. The fractions containing the desired product are combined, concentrated under reduced pressure and lyophilized to give 500 mg (76%) of sodium (3S,4S)-*cis*-3-[2-(2-amino-4-thiazolyl)-(Z)-2-(p-nitrobenzyloxycarbonylmethoxyimino)acetamido)-4-carbamoyloxymethyl-2-azetidinone-1-sulfonate as a light-yellow powder.

$[\alpha]_D^{26}$ + 10.1° (c=1, $H_2O$)

IR $\nu_{max}^{KBr}$, $cm^{-1}$ : 1760, 1720 (br.sh), 1670

NMR ($d_6$-DMSO) δ: 3.9 ~ 4.4 (3H, $C_4$-H, $C_4$-$CH_2$), 4.78 (2H, s, *$OCH_2$*-$COOCH_2$), 5.28 (1H, d.d, J=4.5, 10Hz, $C_3$-H), 5.35 (2H, s, $OCH_2$-COO*$CH_2$*), 6.45 (2H, br.s, $CONH_2$), 6.76 (1H, s, proton at position 5 of the thiazole ring), 7.18 (2H, br.s, amino at position 2 of the thiazole ring), 7.68 (2H, d, J=8Hz, aromatic protons), 8.19 (2H, d, J=8Hz, aromatic protons), 9.18 (1H, d, J=10Hz, $C_3$-NH).

| Elemental analysis: $C_{19}H_{18}N_7NaO_{12}S_2 \cdot 2H_2O$ | | | |
|---|---|---|---|
| | C(%) | H (%) | N (%) |
| Calcd. | 34.60 | 3.36 | 14.87 |
| Found | 34.44 | 3.10 | 14.82 |

Example 2'

In 20 ml of water is dissolved 350 mg of sodium (3S,4S)-*cis*-3-(2-(2-amino-4-thiazolyl)-(Z)-2-(p-nitrobenzyloxycarbonylmethoxyimino)acetamido)-4-carbamoyloxymethyl-2-azetidinone-1-sulfonate and, after addition of 350 mg

of 10% palladium-on-carbon, the mixture is stirred in a hydrogen atmosphere at room temperature for an hour. The catalyst is then filtered off and washed with water. The filtrate and washings are combined and stirred with 40 ml of Dowex® 50W (H) under ice-cooling for an hour. The resin is filtered off and washed with a mixture of water and acetone. The filtrate and washings are combined and concentrated under reduced pressure. The residue is chromatographed on a column of Amberlite® XAD-2 (150 ml), elution being carried out with water and 5% ethanol in that order. The fractions containing the desired product are combined, concentrated under reduced pressure and lyophilized to give 164 mg (61%) of (3S,4S)-*cis*-3-[2-(2-amino-4-thiazolyl)-2-(Z)-carboxymethoxyimino)acetamido)-4-carbamoyloxymethyl-2-azetidinone-1-sulfonic acid as a colorless powder.

$[\alpha]_D^{26}$ - 45° (c=1, DMSO)

IR $\nu_{max}^{KBr}$ $cm^{-1}$: 1760, 1715, 1670, 1640

NMR ($d_6$-DMSO) δ: 3.9 ~ 4.4 (3H, $C_4$-H, $C_4$-$CH_2$), 4.66 (2H, s, NO-$CH_2$), 5.28 (1H, d.d, J=4.5, 10Hz, $C_3$-H), 6.92 (1H, s, proton at position 5 of the thiazole nuclear), 9.33 (1H, d, J=10Hz, $C_3$-NH)

Example 3'

In 5 ml of N,N-dimethylformamide is dissolved 1.13 g of (3S,4S)-*cis*-3-[2-(2-chloroacetamido-4-thiazolyl)-2-(Z)-(1-methyl-1-(4-nitrobenzyloxycarbonyl)ethoxyimino)acetamido)-4-carbamoyloxymethyl-2-azetidinone and, under cooling at -78°C, 2.08 ml of sulfuric anhydride-N,N-dimethylformamide complex solution (1.56 M) is added. The mixture is stirred under ice-cooling for 3 hours and, under cooling at -78°C, 0.23 ml of the same complex solution as above is further added. The whole mixture is stirred under ice-cooling for an additional hour and, upon addition of 0.29 ml of pyridine and then 100 ml of ether, a syrupy precipitate separates out. The upper ether layer is discarded and the syrupy product is dissolved in water and the solution was stirred with 30 ml of Dowex® 50W (Na) at room temperature for an hour. The resin is then filtered off and the filtrate is concentrated to about 30 ml under reduced pressure. To the residue is added 232 mg of sodium N-methyldithiocarbamate and the mixture is stirred at room temperature. After 1 and 2 hours, 232 mg portions of sodium N-methyldithiocarbamate are added respectively. Stirring is thus continued for 3 hours in total. The reaction mixture is washed with ether and concentrated under reduced pressure. The residue is chromatographed on a column of Amberlite® XAD-2 (180 ml), elution being carried out with water, 5% ethanol, 10% ethanol, 15% ethanol and 20% ethanol in that order. The fractions containing the desired product are combined, concentrated under reduced pressure and lyophilized to give 579 mg (46.4%) of sodium (3S,4S)-*cis*-3-[2-(2-amino-4-thiazolyl)-(Z)-2-[1-methyl-1-(4-nitrobenzyloxycarbonyl)ethoxyimino]acetamido]-4-carbamoyloxymethyl-2-azetidinone-1-sulfonate as a light-yellow powder.

$[\alpha]_D^{25}$ + 6.5° (c=1, H2O)

IR $\nu_{max}^{KBr}$ $cm^{-1}$: 1760, 1725, 1670

NMR ($d_6$-DMSO) δ: 1.47 (6H, s, 2x$CH_3$), 3.95 ~ 4.35 (3H, $C_4$-H, $C_4$-$CH_2$), 5.20 ~ 5.45 (3H, $C_3$-H, $OCH_2$), 6.43 (2H, br.s, $CONH_2$), 6.68 H, s, proton at position 5 of the thiazole ring), 7.23 (2H, br.s, amino at position 2 of the thiazole ring), 7.63 (2H, d, J=8Hz, aromatic protons), 8.11 (2H, d, J=8Hz, aromatic protons), 9.00 (1H, d, J=9Hz, $C_30$-NH)

| Elemental analysis: $C_{21}H_{22}N_7NaO_{12}S_2 \cdot 2½H_2O$ | | | |
|---|---|---|---|
| | C(%) | H(%) | N(%) |
| Calcd. | 36.21 | 3.91 | 14.08 |
| Found | 36.36 | 3.73 | 14.05 |

Example 4'

In 17 ml of water is dissolved 344 mg of sodium (3S,4S)-*cis*-3-[2-(2-amino-4-thiazolyl)-(Z)-2-[1-methyl-1-(4-nitrobenzyloxycarbonyl)ethoxyimino]acetamido]-4-carbamoyloxymethyl-2-azetidinone-1-sulfonate and, after addition of 344 mg of 10% palladium-on-carbon, the mixture is stirred in a hydrogen atmosphere at room temperature for 50 minutes. The catalyst is then filtered off and washed with water. After the filtrate and washings are combined, 42 mg of sodium bicarbonate is added under ice-cooling, and the mixture is washed with ethyl acetate. The aqueous solution is stirred with 40 ml of Dowex® 50W (H) under ice-cooling for an hour. The resin is filtered off and the filtrate is concentrated under reduced pressure. The residue is chromatographed on a column of Amberlite XAD-2 (150 ml), elution being carried out with water, 5% ethanol and 10% ethanol in that order. The fractions containing the desired product are combined, concentrated under reduced pressure and lyophilized to give 185 mg (70%) of (3S,4S)-*cis*-3-[2-(2-amino-4-thiazolyl)-(z)-2-(1-carboxy-1-methylethoxyimino)acetamido]-4-carbamoyloxymethyl-2-azetidinone-1-sulfonic acid as a colorless powder.

$[\alpha]_D^{25}$ + 34.3° (c=1, $H_2O$)

IR $\nu_{max}^{KBr}$: $cm^{-1}$ : 1760, 1715 (br.), 1635

NMR ($d_6$-DMSO) δ: 1.46 (6H, s, $2xCH_3$), 3.95 ~ 4.4 (3H, $C_4$-H, $C_4$-$CH_2$), 5.31 (1H, d.d, J=4.5, 10Hz, $C_3$-H), 6.91 (1H, s, proton at posiiton 5 of the thiazole ring), 9.14 (1H, d, J=10Hz, $C_3$-NH)

| Elemental analysis: $C_{14}H_{18}N_6O_{10}S_2 \cdot 2H_2O$ | | | |
|---|---|---|---|
| | C(%) | H(%) | N(%) |
| Calcd. | 31.70 | 4.18 | 15.84 |
| Found | 31.93 | 4.39 | 15.56 |

Example 5'

A wet cake comprising 169 g of (3S,4S)-*cis*-3-[2-(2-amino-4-thiazolyl)-(Z)-2-carboxymethoxyiminoacetamido]-4-carbamoyloxymethyl-2-azetidinone-1-sulfonic acid and 337 g of water, and 60.3 g of sodium hydrogen carbonate were added in alternate portions to 200 ml of water with constant stirring at the pH of ≦5 and the temperature of 0° to 5°C. The resulting solution was stirred under reduced pressure to remove the carbon dioxide gas. Then, 0.6 g of sodium hydrogen carbonate was further added in several portions and a further amount of carbon dioxide gas was removed under reduced pressure to give a solution of pH 5.9. To this solution was added 8.4 g of activated carbon and after 5 minutes' stirring at 5°C, the carbon was separated and washed with 140 ml of water. The filtrate and washings were combined, and 2.70 l of ethanol was added. The mixture was stirred at 25°C. Starting 40 minutes after crystals began to separate out, 2.04 l of ethanol was added dropwise over 20 minutes, the whole mixture was stirred at 25 ± 2°C for 50 minutes, and then the crystals were recovered by filtration, washed with a mixture of 420 ml of ethanol and 60 ml of water and dried in vacua at 25-30°C. The above procedure provided 181 g crystals of (3S,4S) - *cis* - 3 - [2 - (2 amino - 4 - thiazolyl) - (Z) - 2 - carboxymethoxyiminoacetamido] - 4 - carbamoyloxymethyl - 2 - azetidinone - 1 - sulfonic acid disodium salt.

$^1$H-NMR ($D_2O$, δ ppm): 4.2-4.8 (m, CH$CH_2$OCO, O$CH_2$COONa) 5.6 (1H, d, J=5Hz, O=C-CH), 7.0 (1H, s, H-thiazole).

| Elemental analysis: | | | | | |
|---|---|---|---|---|---|
| Calcd. for $C_{12}H_{12}N_6O_{10}S_2Na_2 \cdot 0.46H_2O$ | C, 27.79%; | H, 2.51%; | N, 16.19%; | S, 12.35%; | Na, 8.9% |
| Found | C. 27.91%; | H, 2.62%; | N, 16.37%; | S, 12.22%; | Na, 8.2%. |

The powder X-ray diffraction pattern of this product (Fig. 1; CuKα, 40KV, 45mA) showed the crystallinity thereof.

Example 6'

A wet cake containing 7.07 g of (3S,4S) - *cis* - 3 - [2 - (2 - amino - 4 - thiazolyl) - (Z) - 2 - carboxymethoxyiminoacetamido] - 4 - carbamoyloxymethyl - 2 - azetidinone - 1 - sulfonic acid and 20 g of water and 4.56 g of sodium acetate trihydrate were added in alternate portions to 10 ml of water with constant stirring. To the resulting solution was added 0.14 g of activated carbon and after 5 minutes' stirring at 5°C, the carbon was separated and washed with 5 ml of water. The filtrate and washings were combined and 112 ml of ethanol was added. The mixture was stirred at 25°C. Starting 30 minutes after crystals began to separate out, 84 ml of ethanol was added dropwise over 15 minutes. The mixture was stirred for an hour, after which the crystals were recovered by filtration, washed with a mixture of 28 ml of ethanol and 4 ml of water and dried in vacua. The above procedure provided 7.40 g of crystals of (3S,4S) - *cis* - 3 - [2 -(2 - amino - 4 -thiazolyl) - (Z) - 2 - carboxymethoxyiminoacetamido] - 3 -carbamoyloxymethyl - 2 - azetidinone - 1 - sulfonic acid disodium salt.

The NMR spectrum and the powder X-ray diffraction pattern of this product were in good agreement with those of the compound obtained in Example 5'.

| Elemental analysis: | | | | | |
|---|---|---|---|---|---|
| Calcd. for $C_{12}H_{12}N_6O_{10}S_2Na_2 \cdot 0.49H_2O$ | C, 27.76%; | H, 2.52% ; | N, 16.19%; | S, 12.35%; | Na, 8.9% |
| Found | C, 27.94% ; | H, 2.80%; | N, 16.52%; | S, 12.59%; | Na, 8.7%. |

Example 7'

1) After 191.2 g (1.25 mol) of p-nitrobenzyl alcohol is dissolved in 250 ml of methylene chloride, 98.8 g of pyridine is added, and the mixture is cooled to -5° to 0°C. A solution of 193.75 g (1.25 mol) of γ-chloroacetoacetyl chloride (γ-CAC) in 431 ml of methylene chloride is added at the same temperature over an hour and, after completion of the addition, the mixture is stirred for 30 minutes. The resulting pyridine hydrochloride is filtered off. The filtrate and washings are combined and washed with two 2 l-portions of water. The organic layer is dried over sodium sulfate and the solvent is then distilled off to give 354 g of p-nitrobenzyl γ-chloroacetoacetate as an oil.

NMR ($CDCl_3$) δ: 3.80 (2H, s, $COCH_2COO$), 4.30 (2H, s, $ClCH_2CO$), 5.27 (2H, s, $COOCH_2C_6H_4$), 7.50 (2H, d, J=8Hz, aromatic protons) 8.15 (2H, d, J=8Hz, aromatic protons).

2) In 100 ml of acetic acid is dissolved 50 g (0.184 mol, uncorrected for purity) of the crude p-nitrobenzyl γ-chloroacetoacetate obtained in 1), and the solution is cooled to 5°C or lower, and a solution of 12.7 g (0.184 mol) of sodium nitrite in 50 ml of water is added dropwise thereto at 5°C or lower over a period of an hour. After completion of the addition, the mixture is stirred for 30 minutes and poured into 600 ml of ice water and extracted with 300-ml and 200-ml portions of ethyl acetate. The organic layers are combined and washed with saturated aqueous sodium chloride, dried over sodium sulfate and concentrated to dryness to give 54.0 g of p-nitrobenzyl a-hydroxyimino-y-chloroacetoacetate as an oil.

NMR ($CDCl_3$) δ: 4.60 (2H, s, $ClCH_2CO$), 5.40 (2H, s, $COOCH_2C_6H_4$), 7.52 (2H, d, J=8Hz, aromatic protons), 8.12 (2H, d, J=8Hz, aromatic protons).

3) In a mixture of 225 ml of ethanol and 225 ml of water is dissolved 50 g (0.167 mol, uncorrected for purity) of the crude p-nitrobenzyl α-hydroxyimino γ-chloroacetoacetate obtained in 2), and 12.7 g (0.167 mol) of thiourea and 22.7 g (0.167 mol) of sodium acetate trihydrate are added. The reaction is allowed to proceed at room temperature for 6 hours. On addition of 900 ml of water, an oily product separates out. After 30 minutes' stirring, the oil is separated and 400 ml of ethyl acetate is added, whereupon crystals separate out. After cooling, the crystals are recovered by filtration, washed with a small amount of ether and dried to give 14.9 g of p-nitrobenzyl 2-(2-amino-4-thiazolyl)-2-hydroxyiminoacetate.

IRν (KBr) $cm^{-1}$ : 3400, 3300, 3180, 3100, 1730, 1615, 1525, 1360.

NMR ($d_6$-DMSO) δ: 5.57 (2H, s, $COOCH_2C_6H_4$), 6.95 (1H, s, thiazole-5H), 7.22 (2H, s, $NH_2$-), 7.80 (2H, d, J=8Hz, aromatic protons), 8.35 (2H, d, J=8Hz, aromatic protons).

4) In 60 ml of acetonitrile is suspended 3.0 g (9.3 mmol) of p-nitrobenzyl 2-(2-amino-4-thiazolyl)-2-hydroxyiminoacetate as obtained in 3), and 2.0 g (1.03 mmol) of t-butyl bromoacetate, 0.3 ml of water and 5.14 g (37 mmol) of anhydrous potassium carbonate are added in that order. The mixture is stirred at 40-41°C for an hour. After completion of the reaction, the mixture is poured into 300 ml of water and extracted with 300 ml of ethyl acetate. The organic layer is washed with three 300-ml portions of 5% aqueous sodium chloride, dried over sodium sulfate and concentrated to about 30 ml under reduced pressure. To the concentrate is added 100 ml of ether, and the mixture is cooled to 5°C or lower. The resulting crystalline precipitate is collected by filtration, washed with a small amount of ether, and dried in vacua to give 2.7 g of p-nitrobenzyl (Z)-2-(2-amino-4-thiazolyl)-2-(t-butoxycarbonylmethoxyimino)acetate.

IRν (KBr) $cm^{-1}$ : 3420, 3250, 3150, 1735, 1620, 1528, 1390, 1360.

NMR ($d_6$-DMSO) δ: 1.47 (9H, s, $CH_3 \times 3$), 4.61 12H, s, $NOCH_2COO$), 5.50 (2H, z, $COOCH_2C_6H_4$), 6.92 (1H, s, thiazole-5H), 7.24 (2H, br. $NH_2$-), 7.72 (2H, d, J=8Hz, aromatic protons), 8.26 (2H, d, J=8Hz, aromatic protons).

5) In 1.2 l of tetrahydrofuran is dissolved 20 g (45.9 mmol) of p-nitrobenzyl (Z)-2-(2-amino-4-thiazolyl)-2-(t-butoxycarbonylmethoxyi mino)acetate and, after addition of 20 g of 10% palladium-on-carbon, hydrogen gas is bubbled into the solution. After completion of the reaction, the catalyst is filtered off, and the filtrate is poured into 300 ml of water. The mixture is adjusted to about pH 8 with 5% aqueous sodium bicarbonate and washed with three 200-ml portions of ethyl acetate. The aqueous layer is adjusted to pH about 2 with 10% HCl and cooled to 5°C or lower. The resulting crystalline precipitate is collected by filtration, washed with water and dried under reduced pressure to give 11.1 g of (Z)-2-(2-amino-4-thiazolyl)-2-(t-butoxycarbonylmethoxyimino)acetic acid.

IRν (KBr) $cm^{-1}$ : 3310, 3125, 1740, 1640, 1605, 1585.

NMR ($d_6$-DMSO) δ: 1.48 (9H, s, $CH_3 \times 3$), 4.58 (2H, s, $OCH_2COO$), 6.86 (1H, s, thiazole-5H),

6) In 140 ml of dry acetonitrile is suspended 5.42 g (18 mmol) of (Z)-2-(2-amino-4-thiazolyl)-2-(t-butoxycarbonylmethoxyimino)acetic acid, 2.96 ml (27 mmol) of N-methyl morpholine and then 7.2 g (21.6 mmol) of bis-benzothiazol-2-yl disulfide are added, and the mixture is cooled to 0°C. A solution of 5.38 ml (31.4 mmol) of triethyl phosphite in 35 ml of dry acetonitrile is added dropwise over 4.5 hours and the mixture is stirred at the same temperature for 30 minutes and then cooled to -10°C. The resulting crystalline precipitate is collected by filtration, washed with

a small amount of acetonitrile and dried under reduced pressure to give 5.1 g of (Z)-2-(2-amino-4-thiazolyl)-2-(t-butoxycarbonylmethoxyimino)acetic acid 2-benzothiazolylthiol ester.

IRν (KBr) $cm^{-1}$ : 3400, 3120, 1738, 1710, 1620, 1540, 1450, 1415, 1370.

NMR ($d_6$-DMSO) δ: 1.50 (9H, s, $CH_3$ × 3), 4.78 2H, s, NOC$H_2$COO), 7.10 (1H, s, thiazole-5H), 7.4-7.65 (2H, m, aromatic protons), 8.0-8.3 (2H, m, aromatic protons).

Example 8'

A flask of 1.0 l capacity is charged with 0.06 kg (0.2508 mol) of (3S,4S(-3-amino-4-carbamoyloxymethyl-2-azetidinone-1-sulfonic acid and 0.9 1 of methylene chloride to make a suspension, 0.070 l (0.2508 × 2 mol) of triethylamine and then 0.124 kg (0.2508 × 1.1 mol) of (Z)-2-(2-amino-4-thiazolyl-2-t-butoxycarbonylmethoxyimino) acetic acid 2-benzothiazolylthio ester are added to the suspension under stirring at 10-20°C, and the mixture is stirred at 25-27°C for 4 hours. The insoluble matter is filtered off and the filtrate is further stirred for about an hour and extracted with 0.9 l of water. The aqueous layer is washed with 0.19 l of methylene chloride, 0.38 l of ethyl acetate and 0.19 l of methylene chloride in that order. After degassing, 0.45 l of concentrated hydrochloric acid is added and the mixture is stirred at 25°C for about 2 hours. To the resulting slurry is added 0.9 l of water and the mixture is stirred at about 25°C for about 2 hours and then allowed to stand at 0-2°C overnight. The resulting precipitates are collected by filtration and washed with about 0.6 l of cold water to give about 0.27 kg of (3S,4S)-3-[2-(2-amino-4-thiazolyl)-(2)-2-(carboxymethoxyimino)acetamido]-4-carbamoyloxymethyl-2-azetidinone-1-sulfonic acid as wet crystals.

Example 9'

1.62 g of (3S,4S)-3-amino-4-carbamoyloxymethyl-2-oxo-1-azetidine-sulfonic acid-sodium salt in 180 ml of acetone-water (2:1) are stirred with 3.87 g of (Z)-2-(2-amino-4-thiazolyl)-2-[[1-(t-butoxycarbonyl)-1-methylethoxy]imino]-acetic acid 2-benzothiazolyl-thioester at room temperature for 15 hours. After removal of the acetone in vacuo and the addition of 50 ml of water, crystals are obtained which are washed with water. The mother liquor is evaporated under reduced pressure at 37°C and chromatographed (MCI Gel, water as eluting agent). After lyophilization (3S,4S) - 3 - [(2 - amino -4 - thiazolyl) - 2 - (Z) - [[1 - (t - butoxycarbonyl) - 1 - methylethoxy] - imino] - acetamido] - 4 - carbamoyloxymethyl - 2 - oxo - 1 - azetidine-sulfonic acid-sodium salt is obtained.

IR (KBr) $cm^{-1}$: 1766, 1723, 1683, 1617, 1531, 1458, 1369.

NMR ($d_6$DMSO, δ ppm): 1.35 (15H, s), 4.0-4.15 (3H, $H_4$ and $CH_2$-OCONH$_2$), 5.25 (1H, dd, $H_3$), 6.5 (2H, broad, $CONH_2$), 6.7 (1H, s, H-thiazol), 7.25 (2H, s, $NH_2$), 8.9 (1H, d, CO-NH).

The above 2-benzothiazolyl-thioester can be manufactured as follows:

43 g of 2-(2-amino-4-thiazolyl)-2-(Z)-hydroxyiminoacetic acid-ethylester in 1.2 l of dimethylformamide are treated under nitrogen with 89.2 g of 2-bromo-2-methyl-propionic acid-t-butylester and 110.6 g of powdered potassium carbonate. After stirring for 12 hours at 45°C the reaction mixture is cooled to room temperature, 4.5 l of water are added, and the mixture is extracted with 3.5 l of ethyl acetate. After washing of the organic extract with water, drying with magnesium sulfate and evaporation to dryness 2 - (2 - amino - 4 - thiazolyl) - 2 - [[(Z) - 1 - (t - butoxycarbonyl) - 1 - methylethoxy]imino] - acetic acid-ethyl ester is obtained, which after recrystallization from ether melts at 172°C.

240 g of the so obtained ethyl ester are stirred for 12 hours at 50°C in 1.3 l of methanol and 1.34 l of 1 N aqueous caustic soda solution. After evaporation of the methanol, washing of the aqueous phase with ethyl acetate and the addition of 1.34 l of 1 N aqueous hydrochloric acid the product crystallizes out. The crystals are filtered off at 0°C, washed successively with water, acetonitrile and ether and dried in vacua at 40°C. After stirring for 2 hours in acetonitrile (so as to remove crystal water), filtration and drying in vacua at 40°C 2 - (2 - amino 4 - thiazolyl) - 2 - [[(Z) - 1 - (t - butoxycarbonyl) - 1 - methylethoxy]imino] - acetic acid, melting at 178-179°C, is obtained.

28.8 g of the so obtained acetic acid derivative are dispersed in 360 ml of acetonitrile. With stirring 14.4 ml of N-methylmorpholine are added, and after 10 minutes 34.6 g of 2,2-dithio-bis-benzthiazole. The suspension is cooled to 0°C, 20.2 ml of triethylphosphite are slowly added within the course of 2 hours, and the suspension is stirred for another 12 hours at 0°C. The product is filtered off, washed successively with cold acetonitrile, isopropyl ether and petroleum ether and dried at room temperature in vacuo. 2 - (2 - Amino - 4 - tiazolyl) - 2 - [[(Z) - 1 - (t - butoxycarbonyl) - 1 - methylethoxy]imino]acetic acid - 2 - benzothiazolyl - thioester, melting at 139-140°C, is obtained.

Example 10'

2.28 g of (3S,4S) - 3 - [(2 - amino - 4 - thiazolyl) - 2 - (Z) - [[1 - (t - butoxycarbonyl) - 1 - methylethoxy]imino]

acetamido]- 4 - carbamoyloxymethyl - 2 - oxo - 1 - azetidinesulfonic acid-sodium salt are stirred at 0°C with 5 ml of trifluoroacetic acid. After additional stirring at room temperature for 30 minutes the excess trifluoroacetic acid is removed in vacua and the remaining oil treated with 100 ml of ether. The resulting crystals are filtered off, washed with ether and dried in vacua. The product is purified by reverse phase chromatography and lyophilized. (3S,4S) - 3 - [(Z) - 2 - (2 - Amino - 4 - thiazolyl) - 2 - [[1 - carboxy - 1 - methylethoxy]imino]acetamido] - 4 - carbamoyloxymethyl - 2 - oxo - 1 - azetidine-sulfonic acid is obtained; $[\alpha]_D$ = +35.7° (c=0.3 in water).

| Elemental analysis | | | |
|---|---|---|---|
| Calcd. for $C_{14}H_{18}N_6O_{10}S_2$ | C, 34.01; | H, 3.67; | N, 17.00 |
| Found | C,34.52; | H,3.72; | N,16.63. |

IR (KBr) $cm^{-1}$ : 1764, 1722, 1680, 1637.

NMR ($d_6$DMSO, δ ppm): 1.50 (6H, s, × $CH_3$), 4.00-4.20 (3H, CH-$CH_2$), 5.35 (1H, dd, 4, 5 and 9Hz, $H_3$), 6.50 (3H, broad, $NH^+_3$ or COH, $CONH_2$), 6.90 (1H, s, thiazole-5H), 9.15 (1H, d, 9Hz, CONH).

Example 11'

In analogy to Example 9' reaction of 2 - (2 - amino - 4 - thiazolyl) - 2 - [[(Z) - (p - nitrobenzyloxycarbonyl) - methoxy]imino]acetic acid - 2 - benzothiazolyl - thioester with (3S,4S) - 3 - amino - 4 - carbamoyloxymethyl - 2 - oxo - 1 - azetidine sulfonic acid-sodium salt yields (3S,4S) - 3 - [(Z) - 2 - (2 - amino 4 - thiazolyl) - 2 - [[(p - nitrobenzyloxy-carbonyl)methoxyimino]acetamido]- 4 - carbamoyloxymethyl - 2 - oxo - 1 - azetidine - sulfonic acid - sodium salt.

| Elemental analysis | | | | |
|---|---|---|---|---|
| Calcd. for $C_{19}H_{18}N_7O_{12}S_2Na$ | C, 36.60; | H, 2.91; | N, 15.73; | S, 10.28 |
| Found | C,37.00; | H,2.88; | N,15.74; | S,10.45 |

IR (KBr) $cm^{-1}$ : 3353, 1761, 1729, 1524, 1348.

NMR ($d_6$DMSO, δ ppm): 4.0-4.2(3H, m, CH-$CH_2$), 4.7 (2H, s, O-$CH_2$), 5.30 (1H, dd, NH-CH-), 5.32 (2H, s, O-$CH_2$), 6.70 (2H, broad, $NH_2$), 6.9 (1H, s, S-CH-), 7.10(2H, broad, $NH_2$), 7.70 and 8.2 (2 × 2H, 2d, 2 × 3Hz, Ar), 9.15 (1H, d, 9Hz, NHCO).

The 2-benzothiazolyl-thioester used above can be manufactured as follows:

6.1 g of 2-(2-amino-4-thiazolyl)-2-(Z)-hydroxyiminoacetic acid-t-butylester in 250 ml of dry acetonitrile are stirred at room temperature with 13.7 g of bromoacetic acid-4-nitrobenzylester and 12.9 ml of N-ethyldiisopropylamine. After 5 minutes 7.5 g of sodium iodide are added, and the reaction mixture is stirred for an additional 3½ hours in an argon atmosphere at room temperature. After evaporation of the solvent, dilution with 500 ml of ethyl acetate, washing with water, drying over sodium sulfate and evaporation to dryness 2-(2-amino-4-thiazolyl)-2-[[(p-nitrobenzyloxycarbonyl)-methoxy]imino]acetic acid-t-butylester is obtained, which after crystallization from ethyl acetate-n-hexane melts at 146.8°C (dec.).

5.0 g of the so obtained t-butylester in 86 ml of acetic acid are stirred with 5.2 ml of boron trifluoride etherate. After stirring for 5 hours at room temperature and mixing with 260 ml of water the precipitate obtained is filtered off and dried at 40°C in vacua. 2 - (2 - Amino - 4 - thiazolyl) - 2 - [[(Z) - (p - nitro-benzyloxycarbonyl)methoxy]imino]acetic acid, melting at 175°C (dec.), is obtained.

1.9 g of the above acetic acid derivative in 30 ml of acetonitrile are stirred with 1.4 ml of N-methylmorpholine, 2.0 g of 2,2-dithio-bis-benzthiazole and 1.14 ml of triethylphosphite. After stirring for 1 hour at room temperature the reaction mixture is cooled to 0°C and filtered. The filtrate is evaporated to dryness and the residue crystallized from methylene chloride. 2 - (2 - Amino - 4 - thiazolyl) - 2 - [[(Z) - (p - nitro-benzyloxycarbonyl)methoxy]imino]azetic acid-2-benzothiazolyl-thioester is obtained melting at 124-126°C.

## Claims

**1.** A 1-sulfo-2-oxazetidine derivative of the formula

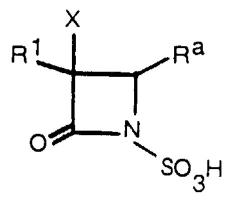

(I)

wherein X is a hydrogen atom or a methoxy group and

(1) when R is a $C_{1-6}$ alkoxycarbonylamino, a $C_{7-9}$ aralkyloxycarbonylamino or a group of the formula:

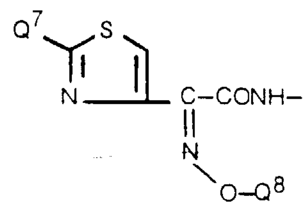

wherein

$Q^7$ is amino group or a protected amino group; and $Q^8$ is a $C_{1-6}$ alkyl group, a group of the formula -$CH_2COOQ^9$ or a group of the formula

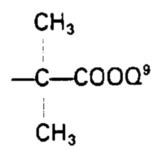

wherein $COOQ^9$ is carboxyl or a $C_{7-9}$ aralkyloxycarbonyl group which may be substituted with nitro group;

$R^a$ is a group of the formula: -$COQ^{0a}$ wherein $Q^{0a}$ is hydrazino, carbamoylhydrazino, $C_{1-6}$- alkoxycarbonylhydrazino or carbamoyl-($C_{1-4}$) alkylamino, or a group of the formula: -$(CH_2)_n$a-$R^{4b}$ wherein $n^a$ is an integer of 1 to 3, and $R^{4b}$ is carbamoylamino, (N-sulfocarbamoyl)amino pyridinio, $C_{1-6}$-alkylsulfinyl, $C_{1-6}$-alkylsulfonyl, 1-$C_{1-6}$-alkoxyimino-$C_{1-6}$-alkyl or formylamino group,

(2) when $R^1$ is a group of the formula:

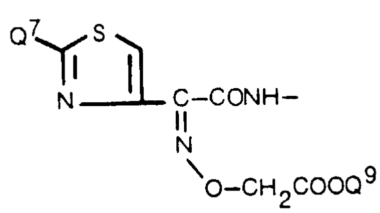

wherein

$Q^7$ and $COOQ^9$ are the same as defined above;

$R^a$ is a $C_{1-6}$-alkylaminocarbonyl group,

(3) when $R^1$ is D-2-(6,7-dihydroxychromone-3-carboxamido)-2-(4-hydroxyphenyl)-acetamido; $R^a$ is carbamoyl group,

(4) when $R^1$ is a group of the formula:

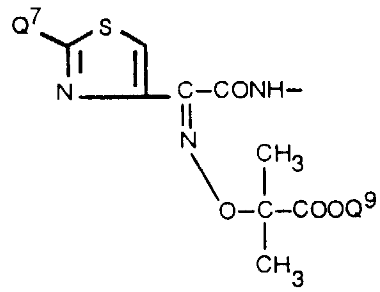

wherein $Q^7$ and $COOQ^9$ are the same as defined above; $R^a$ is carboxyl ; a group of the formula: $-CH_2CH_2R^{4c}$ wherein $R^{4c}$ is a $C_{1-6}$-alkoxycarbonyl or $C_{2-4}$-acyloxy group ; monochloroacetoxymethyl; hydroxymethyl or propyl group,

(5) when $R^1$ is a $C_{1-6}$-alkoxycarbonylamino group; $R^a$ is $-CH_2OSO_2CH_3$,

(6) when $R^1$ is a group of the formula:

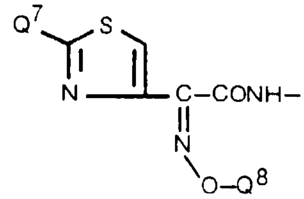

wherein $Q^7$ and $Q^8$ are the same as mentioned above; $R^a$ is sulfaminocarbonyloxymethyl, halo-$C_{1-6}$-alkylcarbonylcarbamoyloxymethyl or pyrazolyl or isoxazolyl group which may be substituted with a $C_{1-6}$-alkyl or/and $C_{2-7}$-acyl group, or

(7) when $R^1$ is a group of the formula:

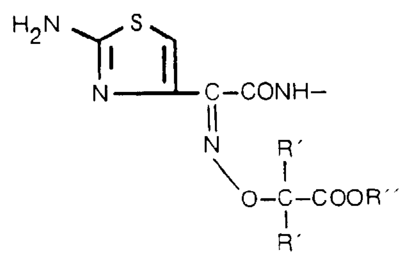

wherein R' is hydrogen atom or a $C_{1-6}$-alkyl group and R" is hydrogen atom, a $C_{1-6}$-alkyl group or a $C_{7-9}$-aralkyl group which may be substituted with nitro group; $R^a$ is carbamoyloxymethyl and the derivative has the (3S, 4S)-configuration; or a pharmaceutically acceptable salt or ester thereof.

**2.** A compound as claimed in Claim 1, which is a 1-sulfo-2-azetidinone derivative of the formula

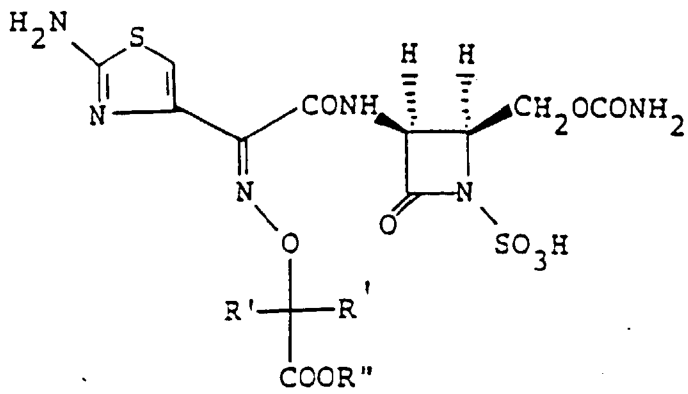

wherein R' and R" are as defined in Claim 1, said derivative having the (3S,4S)-configuration; or a pharmaceutically acceptable salt or ester thereof.

**3.** A compound as claimed in Claim 2, wherein R' is a hydrogen atom.

**4.** A compound as claimed in Claim 2, wherein R' is a $(C_{1-4})$alkyl group.

**5.** A compound as claimed in Claim 4, wherein the $(C_{1-4})$-alkyl group is methyl.

**6.** A compound as claimed in Claim 2, wherein R" is a hydrogen atom.

**7.** A compound as claimed in Claim 2, wherein the salt is a salt with a nontoxic cation, a basic amino acid or a polyhydroxyalkylamine with respect to the sulfa and/or carboxyl group, or a salt with an organic acid, inorganic acid or acidic amino acid with respect to the amino group.

**8.** A compound as claimed in Claim 7, wherein the salt with a nontoxic cation is mono- or di-sodium salt.

**9.** A compound as claimed in Claim 8, wherein the disodium salt is in a crystalline form.

**10.** A compound as claimed in Claim 2, wherein the ester is an α-$(C_{1-4})$alkoxy$(C_{1-4})$alkyl, $(C_{1-4})$alkylthiomethyl, α-$(C_{2-6})$acyloxy$(C_{1-4})$alkyl or α-$(C_{1-4})$alkoxycarbonyloxy$(C_{1-4})$alkyl ester.

**11.** A compound as claimed in Claim 2, which is (3S,4S)-*cis*-3-[2-(2-amino-4-thiazolyl)-(Z)-2-carboxymethoxyimino) acetamido]-4-carbamoyloxymethyl-2-azetidinone-1-sulfonic acid.

**12.** A compound as claimed in Claim 2, which is crystalline (3S,4S)-*cis*-3-[2-(2-amino-4-thiazolyl)-(Z)-2carboxymethoxyiminoacetamido]-4-carbamoyloxymethyl-2-azetidinone-1-sulfonic acid disodium salt.

**13.** A compound as claimed in Claim 2, which is (3S,4S)-*cis*-3-[2-(2-amino-4-thiazolyl)-(Z)-2-(1-carboxy-1-methylethoxyimino)acetamido]-4-carbamoyloxymethyl-2-azetidinone-1-sulfonic acid.

**14.** A method of producing a 1-sulfo-2-oxazetidine derivative of the formula

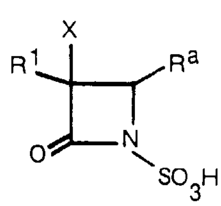

wherein the symbols are as defined in Claim 1, or a pharmaceutically acceptable salt or ester thereof, which comprises sulfonating a compound of the formula

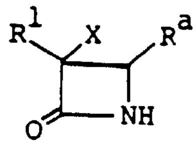

wherein all symbols are as defined in Claim 1, or a salt or ester thereof, and if necessary, removing the protective group.

**15.** A method of producing a 1-sulfo-2-oxoazetidine derivative of the formula

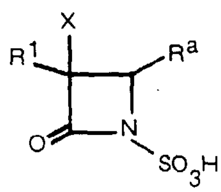

wherein the symbols are as defined in Claim 1, or a pharmaceutically acceptable salt or ester thereof, which comprises acylating a compound of the formula

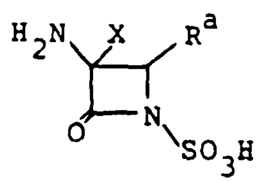

wherein all symbols are as defined in Claim 1, or a salt or ester thereof, and if necessary, removing the protective group.

**16.** A method according to Claim 14, wherein a 1-sulfo-2-azetidinone derivative of the formula

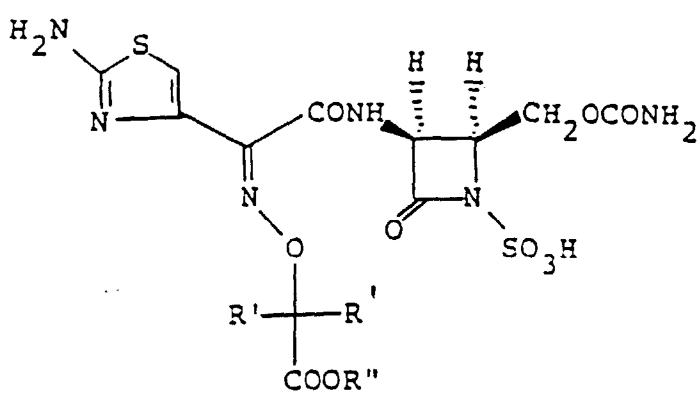

wherein R' and R" are as defined in Claim 1, which derivative has the (3S,4S)-configuration, or a pharmaceutically acceptable salt or ester thereof, is produced by sulfonating a compound of the formula

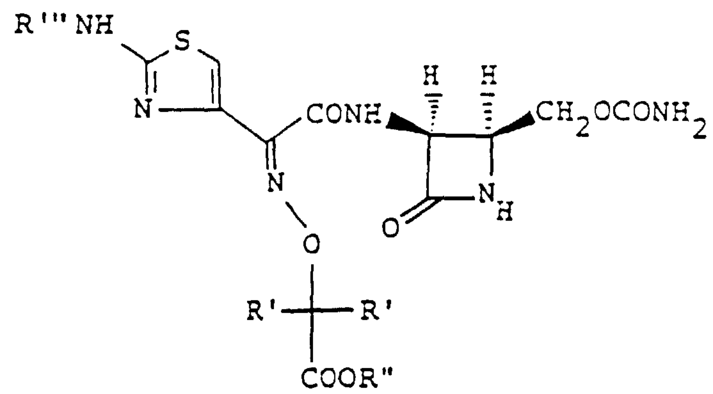

wherein R' and R" are as defined in Claim 1 and R"' is an amino-protecting group, or a salt thereof and then removing the protective group and, if necessary, the ester residue.

**17.** A method according to Claim 15, wherein a 1-sulfo-2-azetidinone derivative of the formula

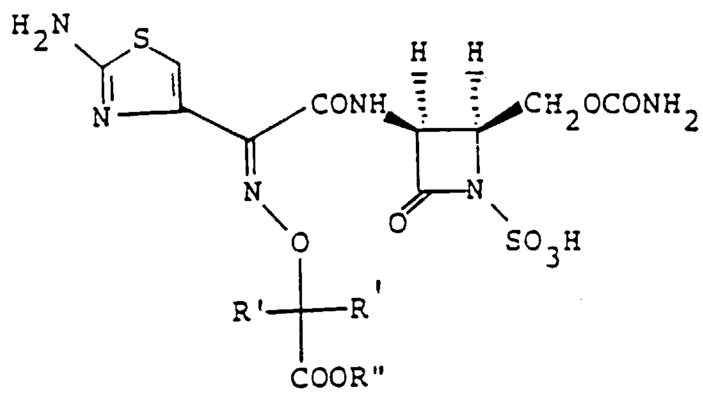

wherein R' and R" are as defined in Claim 1, which derivative has the (3S,4S)-configuration, or a pharmaceutically acceptable salt or ester thereof, is produced by reacting (3S,4S)-*cis*-3-amino-4-carbamoyloxymethyl-2-azetidinone-1-sulfonic acid or a salt or ester thereof, with a carboxylic acid of the formula

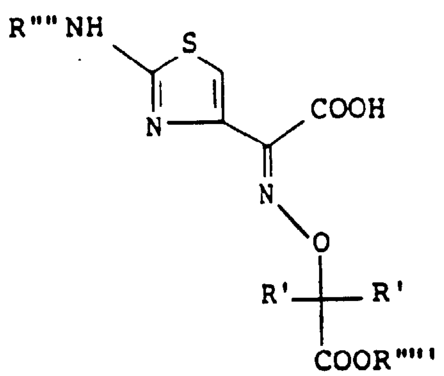

wherein R' is as defined in Claim 1, R"" is a hydrogen atom or an amino-protecting group and R""' is an ester residue, or a functional derivative thereof and then removing the protective group and, if necessary, the ester residue.

**18.** A method according to Claim 17, wherein the functional derivative is an active thioester derivative.

**19.** A method according to Claim 18, wherein the active thioester is 2-benzothiazolylthioester.

**20.** An antimicrobial preparation which contains an effective dose of a 1-sulfo-2-oxoazetidine derivative of the formula

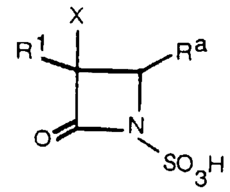

wherein the symbols are as defined in Claim 1, or a pharmaceutically acceptable salt or ester thereof as an active component, together with a suitable carrier or carriers.

**21.** An antimicrobial preparation which contains a β-lactam antibiotic and as beta-lactamase inhibitor an effective amount of a 1-sulfo-2-oxoazetidine derivative of the formula

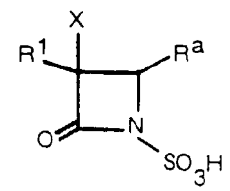

wherein the symbols are as defined in Claim 1, or a pharmaceutically acceptable salt or ester thereof, together with a suitable carrier or carriers.

**22.** A compound of the formula

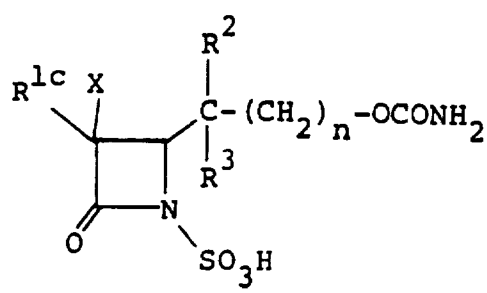

wherein $R^{1c}$ is an amino group which may optionally be protected; X is hydrogen or methoxy; n is an integer of 0 to 3; and $R^2$ and $R^3$, which may be the same or different, stand for hydrogen, alkyl, cycloalkyl*, aralkyl*, aryl*, heterocyclic* group, alkoxycarbonyl or acyl, or $R^2$ and $R^3$ taken together stand for oxo; and the above groups with a superscript asterisk "*" may be substituted with one to three substituents which may be the same or different, or a salt or ester thereof.

**23.** A compound as claimed in Claim 31, which is a compound of the formula

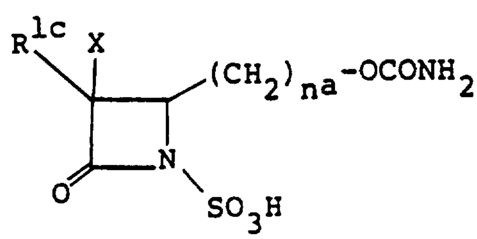

wherein $R^{1c}$ is an amino group which may optionally be protected; X is hydrogen or methoxy; and $n^a$ is an integer

of 1 to 3.

**24.** A compound as claimed in Claim 37, wherein $R^{1c}$ is amino group.

**25.** A compound as claimed in Claim 37, wherein $R^{1c}$ is a protected amino group.

**26.** A compound as claimed in Claim 39, wherein the protecting group of the protected amino group is an aromatic acyl, aliphatic acyl, esterified carboxyl or non-acyl amino-protecting group.

**27.** A compound as claimed in Claim 39, wherein the protecting group of the protected amino group is an esterified carboxyl group.

**28.** A compound as claimed in Claim 41, wherein the esterified carboxyl group is benzyloxycarbonyl.

**29.** A compound as claimed in Claim 37, wherein X is hydrogen.

**30.** A compound as claimed in Claim 37, wherein $n^a$ is 1.

**31.** A compound as claimed in Claim 36, wherein the group represented by the symbol $R^{1c}$ has β-configuration and the group represented by the symbol X has α-configuration, respectively, to the azetidine ring.

**32.** A compound as claimed in Claim 36, wherein the group represented by the symbol

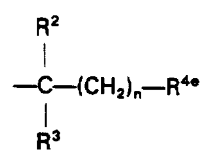

has *cis* configuration to the group $R^{1c}$.

**33.** A compound as claimed in Claim 36, which is sodium *cis*-3-benzyloxycarboxamido-4-carbamoyloxymethyl-2-azetidinone-1-sulfonate.

**34.** A compound as claimed in Claim 36, which is sodium (3S,4S)-*cis*-3-benzyloxycarboxamido-4-carbamoyloxymethyl-2-azetidinone-1-sulfonate.

**35.** A compound as claimed in Claim 36, which is *cis*-3-amino-4-carbamoyloxymethyl-2-azetidinone-1-sulfonic acid.

**36.** A compound as claimed in Claim 36, which is (3S,4S)-*cis*-3-amino-4-carbamoyloxymethyl-2-azetidinone-1-sulfonic acid.

**37.** A method for producing a compound of the formula

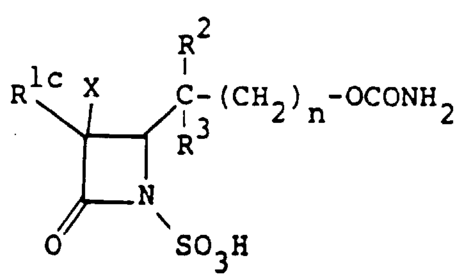

wherein $R^{1c}$ is an amino group which may optionally be protected; X is hydrogen or methoxy; n is an integer of 0 to 3; and $R^2$ and $R^3$, which may be the same or different, stand for hydrogen, alkyl, cycloalkyl*, aralkyl*, aryl*,

heterocyclic* group, alkoxycarbonyl or acyl, or $R^2$ and $R^3$ taken together stand for oxo; and the above groups with a superscript asterisk "*" may be substituted with one to three substituents which may be the same or different, or a salt or ester thereof, which comprises sulfonating a compound of the formula

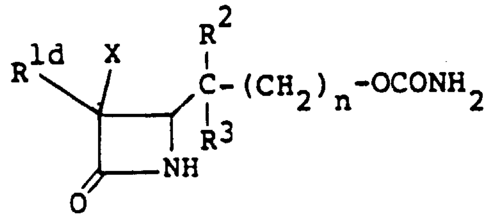

wherein $R^{1d}$ is an amino group which may optionally be protected and the other groups are as defined above, or a salt or ester thereof, and if necessary, removing the protective group.

**Patentansprüche**

**1.** 1-Sulfo-2-oxoazetidin-Derivat der Formel

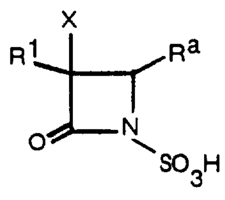

in der

X ein Wasserstoff-Atom oder eine Methoxy-Gruppe ist und,

(1) wenn $R^1$ eine $C_{1-5}$-Alkoxycarbonylamino-Gruppe, eine $C_{7-9}$-Aralkyloxycarbonylamono-Gruppe oder eine Gruppe der Formel

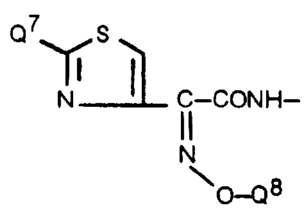

ist, in der $Q^7$ eine Amino-Gruppe oder eine geschützte Amino-Gruppe ist und $Q^8$ eine $C_{1-6}$-Alkyl-Gruppe, eine Gruppe der Formel $-CH_2COOQ^9$ oder eine Gruppe der Formel

$$\begin{array}{c} CH_3 \\ | \\ -C-COOQ^9 \\ | \\ CH_3 \end{array}$$

ist, worin $COOQ^9$ Carboxyl oder eine $C_{7-9}$-Aralkyloxycarbonyl-Gruppe ist, die mit einer Nitro-Gruppe substituiert sein kann,

$R^a$ eine Gruppe der Formel $-COQ^{0a}$ ist, worin, $Q^{0a}$ eine Hydrazino-, Carbamoylhydrazino-, $C_{1-6}$-Alkoxycarbonylhydrazino- oder Carbamoyl-$(C_{1-4})$-alkylamino-Gruppe oder eine Gruppe der Formel $-(CH_2)_na-R^{4b}$ ist, worin $n^a$ eine ganze Zahl von 1 bis 3 ist und $R^{4b}$ eine Carbamoylamino-, (N-Sulfocarbamoyl)amino-, Pyridino,

$C_{1-6}$-Alkylsulfinyl-, $C_{1-6}$-Alkylsulfonyl-, $C_{1-6}$-Alkoxyimino-$C_{1-6}$-alkyl- oder Formylamino-Gruppe ist,
(2) wenn $R^1$ eine Gruppe der Formel

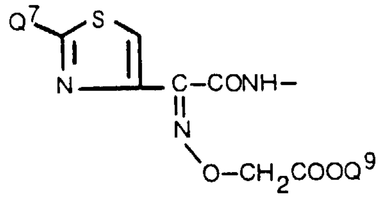

ist, in der $Q^7$ und $COOQ^9$ die im Vorstehenden angegebenen Bedeutungen haben,
$R^a$ eine $C_{1-6}$-Alkylaminocarbonyl-Gruppe ist,
(3) wenn $R^1$ D-2-(6,7-Dihydroxychromon-3-carboxamidol-2-(4-hydroxyphenyl)acetamido ist,
$R^a$ eine Carbamoyl-Gruppe ist,
(4) wenn $R^1$ eine Gruppe der Formel

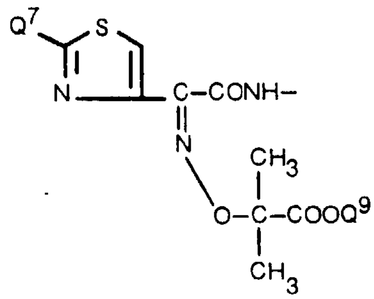

ist, in der $Q^7$ und $COOQ^9$ die im Vorstehenden angegebenen Bedeutungen haben,
$R^a$ eine Carboxyl-Gruppe, eine Gruppe der Formel $-CH_2CH_2R^{4c}$, worin $R^{4c}$,eine $C_{1-6}$-Alkoxycarbonyl-Gruppe oder eine $C_{2-4}$-Acyloxy-Gruppe ist, eine Monochloroacetoxymethyl-Gruppe, eine Hydroxymethyl-Gruppe oder eine Propyl-Gruppe ist,
(5) wenn $R^1$ eine $C_{1-6}$-Alkoxycarbonylamino-Gruppe ist,
$R^a$ eine $-CH_2OSO_2CH_3$-Gruppe ist,
(6) wenn $R^1$ eine Gruppe der Formel

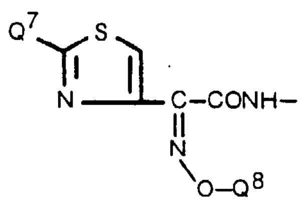

ist, in der $Q^7$ und $Q^8$ die im Vorstehenden angegebenen Bedeutungen haben,
$R^a$ eine Sulfaminocarbonyloxymethyl-Gruppe, eine Halogeno-$C_{1-6}$-alkylcarbonylcarbamoyloxymethyl-Gruppe oder eine Pyrazolyl- oder Isoxazolyl-Gruppe ist, die mit einer $C_{1-6}$-Alkyl- und/oder einer $C_{2-7}$-Acyl-Gruppe substituiert sein kann, oder
(7) wenn $R^1$ eine Gruppe der Formel

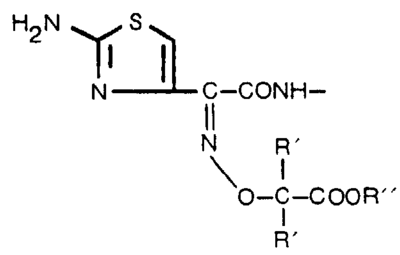

ist, in der R' ein Wasserstoff-Atom oder eine $C_{1-6}$-Alkyl-Gruppe ist und R" ein Wasserstoff-Atom, eine $C_{1-6}$-Alkyl-Gruppe oder eine $C_{7-9}$-Aralkyl-Gruppe ist, die mit einer Nitro-Gruppe substituiert sein kann,

$R^a$ eine Carbamoyloxymethyl-Gruppe ist und das Derivat die (3S,4S)-Konfiguration besitzt, oder ein pharmazeutisch annehmbares Salz oder ein pharmazeutisch annehmbarer Ester desselben.

**2.** Verbindung nach Anspruch 1, die ein 1-Sulfo-2-azetidinon-Derivat der Formel

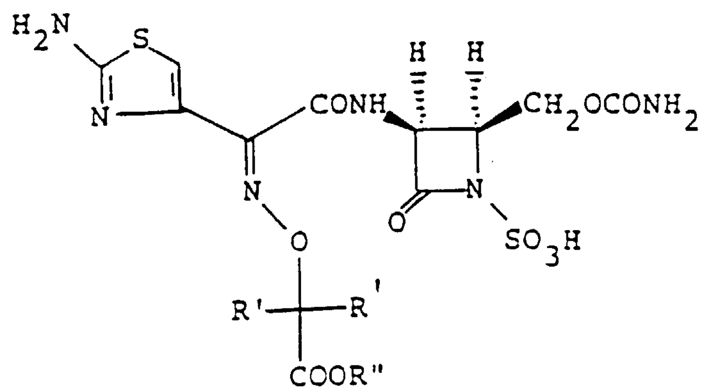

ist, worin R' und R" die in Anspruch 1 angegebenen Bedeutungen haben, wobei das Derivat die (3S,4S)-Konfiguration besitzt, oder ein pharmazeutisch annehmbares Salz oder ein phramazeutisch annehmbarer Ester desselben.

**3.** Verbindung nach Anspruch 2, worin R' ein Wasserstoff-Atom ist.

**4.** Verbindung nach Anspruch 2, worin R' eine $C_{1-4}$-Alkyl-Gruppe ist.

**5.** Verbindung nach Anspruch 4, worin die $C_{1-4}$-Alkyl-Gruppe Methyl ist.

**6.** Verbindung nach Anspruch 2, worin R" ein Wasserstoff-Atom ist.

**7.** Verbindung nach Anspruch 2, worin das Salz ein Salz mit einem nicht-toxischen Kation, einer basischen Aminosäure oder einem Polyhydroxyalkylamin in bezug auf die Sulfo- und/oder Carboxyl-Gruppe oder ein Salz mit einer organischen Säure, anorganischen Säure oder sauren Aminosäure in bezug auf die Amino-Gruppe ist.

**8.** Verbindung nach Anspruch 7, worin das Salz mit einem nicht-toxischen Kation das Mono- oder Dinatrium-Salz ist.

**9.** Verbindung nach Anspruch 8, worin das Dinatrium-Salz in kristalliner Form vorliegt.

**10.** Verbindung nach Anspruch 2, worin der Ester ein α-($C_{1-4}$)-Alkoxy-($C_{1-4}$)-alkyl-, ($C_{1-4}$)-Alkylthiomethyl-, α-$C_{2-6}$)-Acyloxy-($C_{1-4}$)-alkyl- oder α-($C_{1-4}$)-Alkoxycarbonyloxy-($C_{1-4}$)-alkyl-ester ist.

**11.** Verbindung nach Anspruch 2, die (3S,4S)-cis-3-[2-(2-Amino-4-thiazolyl)-(Z)-2-carboxymethoxyiminoacetamido]-4-carbamoyloxymethyl-2-azetidinon-1-sulfonsäure ist.

**12.** Verbindung nach Anspruch 2, die kristallines (3S,4S)-cis-3-[2-(2-Amino-4-thiazolyl)-(Z)-2-carboxymethoxyiminoacetamido]-4-carbamoyloxymethyl-2-azetidinon-1-sulfonsäure-dinatrium-Salz ist.

**13.** Verbindung nach Anspruch 2, die (3S,4S)-cis-3-[2-(2-Amino-4-thiazolyl)-(Z)-2-(1-carboxy-1-methylethoxyimino) acetamido]-4-carbamoyloxymethyl-2-azetidinon-1-sulfonsäure ist.

**14.** Verfahren zur Herstellung eines 1-Sulfo-2-oxoazetidin-Derivats der Formel

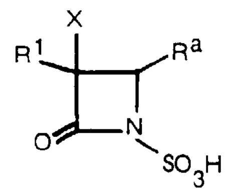

in der die Symbole die in Anspruch 1 angegebenen Bedeutungen haben, oder eines pharmazeutisch annehmbaren Salzes oder Esters desselben, umfassend das Sulfonieren einer Verbindung der Formel

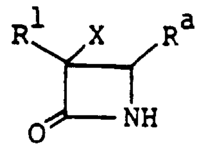

in der die Symbole die in Anspruch 1 angegebenen Bedeutungen haben, oder eines Salzes oder Esters derselben und erforderlichenfalls das Entfernen der Schutzgruppe.

**15.** Verfahren zur Herstellung eines 1-Sulfo-2-oxoazetidin-Derivats der Formel

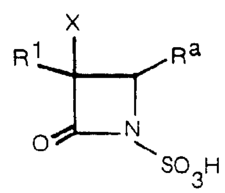

in der die Symbole die in Anspruch 1 angegebenen Bedeutungen haben, oder eines pharmazeutisch annehmbaren Salzes oder Esters desselben, umfassend das Acylieren einer Verbindung der Formel

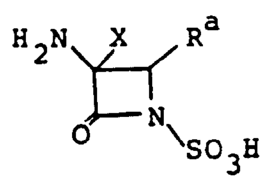

in der die Symbole die in Anspruch 1 angegebenen Bedeutungen haben, oder eines Salzes oder Esters derselben und erforderlichenfalls das Entfernen der Schutzgruppe.

**16.** Verfahren nach Anspruch 14, worin ein 1-Sulfo-2-oxoazetidin-Derivat der Formel

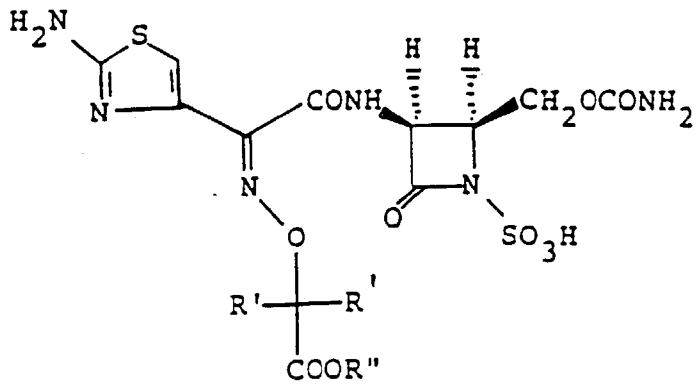

in der R' und R" die in Anspruch 1 angegebenen Bedeutungen haben, wobei das Derivat die (3S,4S)-Konfiguration besitzt, oder ein pharmazeutisch annehmbares Salz oder ein pharmazeutisch annehmbarer Ester desselben durch Sulfonieren einer Verbindung der Formel

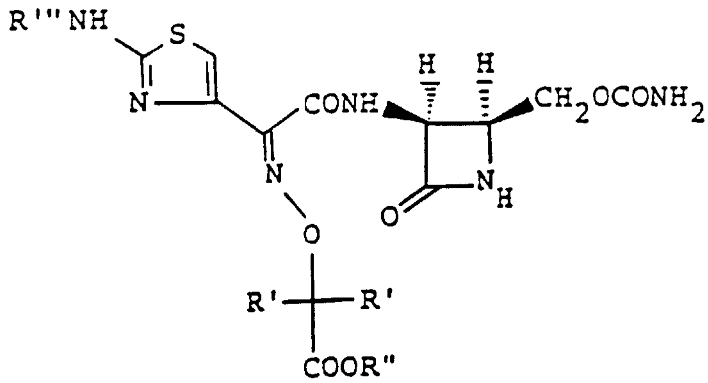

in der R' und R" die in Anspruch 1 angegebenen Bedeutungen haben und R"' eine Schutzgruppe für die Amino-Gruppe ist, oder eines Salzes derselben, und danach Abspalten der Schutzgruppe und erforderlichenfalls des Ester-Restes erzeugt wird.

**17.** Verfahren nach Anspruch 15, worin ein 1-Sulfo-2-oxoazetidin-Derivat der Formel

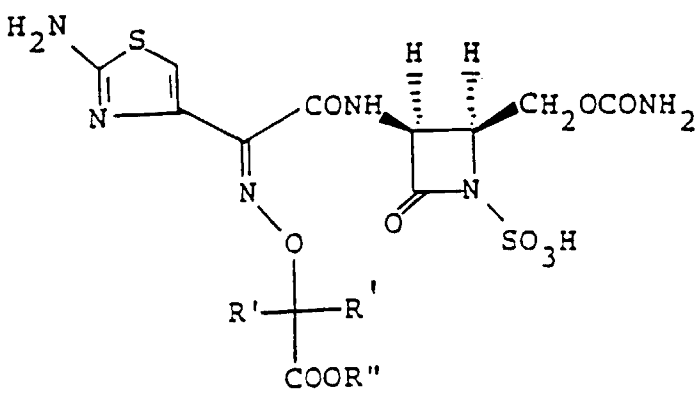

in der R' und R" die in Anspruch 1 angegebenen Bedutungen haben, wobei das Derivat die (35,45)-Konfiguration besitzt, oder ein pharamzeutisch annehmbares Salz oder ein pharmazeutisch annehmbarer Ester desselben durch Umsetzen von (3S,4S)-cis-3-Amino-4-carbamoyloxymethyl-2-azetidinon-1-sulfonsäure oder eines Salzes oder Esters derselben mit einer Carbonsäure der Formel

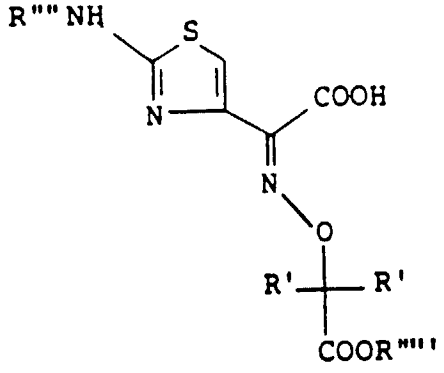

in der R' die in Anspruch 1 angegebenen Bedeutungen hat, R"" ein Waserstoff-Atom oder eine Schutzgruppe für die Amino-Gruppe ist und R""' ein Ester-Rest oder ein funktionelles Derivat desselben ist, und danach Abspalten der Schutzgruppe und erforderlichenfalls des Ester-Restes erzeugt wird.

**18.** Verfahren nach Anspruch 17, worin das funktionelle Derivat ein aktives Thioester-Derivat ist.

**19.** Verfahren nach Anspruch 18, worin der aktive Thioester 2-Benzothiazolylthioester ist.

**20.** Antimikrobielles Präparat, das eine wirksame Dosis eines 1-Sulfo-2-oxoazetidin-Derivats der Formel

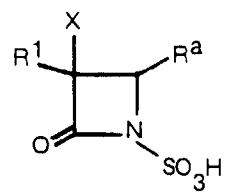

worin die Symbole die in Anspruch 1 angegebenen Bedeutungen haben, oder ein pharmazeutisch annehmbares Salz oder einen pharmazeutisch annehmbaren Ester desselben als Wirkstoff zusammen mit einem oder mehreren geeigneten Trägern enthält.

**21.** Antimikrobielles Präparat, das ein β-Lactam-Antibiotikum und als beta-Lactamase-Inhibitor eine wirksame Menge eines 1-Sulfo-2-oxoazetidin-Derivates der Formel

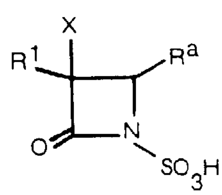

worin die Symbole die in Anspruch 1 angegebenen Bedeutungen haben, oder ein pharamzeutisch annehmbares Salz oder einen pharmazeutisch annehmbaren Ester desselben als Wirkstoff zusammen mit einem oder mehreren geeigneten Trägern enthält.

**22.** Verbindung der Formel

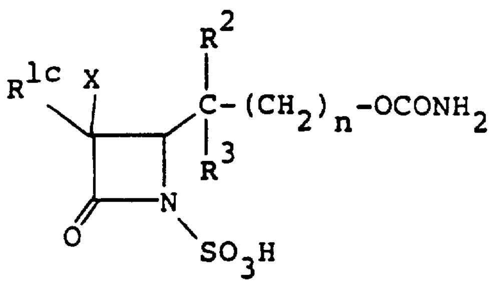

in der

$R^{1c}$ eine Amino-Gruppe ist, die gegebenenfalls geschützt sein kann,

X Wasserstoff oder Methoxy ist.

n eine ganze Zahl von 0 bis 3 ist und

$R^2$ und $R^3$, die gleich oder verschieden sein können, für Wasserstoff, Alkyl, Cycloalkyl*, Aralkyl*, Aryl*, eine heterocyclische* Gruppe, Alkoxycarbonyl oder Acyl stehen oder $R^2$ und $R^3$, zusammen genommen, für Oxo stehen und die vorsehenden Gruppen mit einem Sternchen "*" mit ein bis drei Substituenten substituiert sein können, die gleich oder verschieden sein können,

oder ein Salz oder Ester derselben.

**23.** Verbindung nach Anspruch 22, die eine Verbindung der Formel

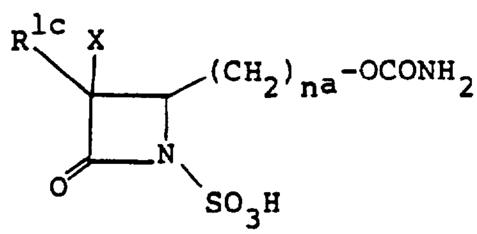

ist, in der $R^{1c}$ eine Amino-Gruppe ist, die gegebenenfalls geschützt sein kann, X Wasserstoff oder Methoxy ist, und $n^a$ eine ganze Zahl von 1 bis 3 ist.

**24.** Verbindung nach Anspruch 22, worin $R^{1c}$ eine Amino-Gruppe ist.

**25.** Verbindung nach Anspruch 22, worin $R^{1c}$ eine geschützte Amino-Gruppe ist.

**26.** Verbindung nach Anspruch 25, worin die Schutzgruppe der geschützten Amino-Gruppe eine aromatische Acyl-, aliphatische Acyl-, veresterte Carboxyl- oder Nicht-Acyl-Amino-Schutzgruppe ist.

**27.** Verbindung nach Anspruch 25, worin die Schutzgruppe der geschützten Amino-Gruppe eine veresterte Carboxyl-Gruppe ist.

**28.** Verbindung nach Anspruch 27, worin die veresterte Carboxyl-Gruppe Benzyloxycarbonyl ist.

**29.** Verbindung nach Anspruch 24, worin X Wasserstoff ist.

**30.** Verbindung nach Anspruch 24, worin $n^a$ 1 ist.

**31.** Verbindung nach Anspruch 23, worin die durch das Symbol $R^{1c}$ dargestellte Gruppe β-Konfiguration besitzt und die durch das Symbol X dargestellte Gruppe α-Konfiguration besitzt, jeweils relativ zu dem Azetidin-Ring.

**32.** Verbindung nach Anspruch 23, worin die durch das Symbol

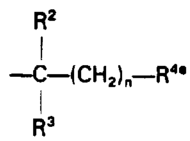

dargestellte Gruppe cis-Konfiguration in bezug auf die gruppe $R^{1c}$ hat.

**33.** Verbindung nach Anspruch 23, die Natrium-cis-3-benzyloxycarboxamido-4-carbamoyloxymethyl2-azetidinon-1-sulfonat ist.

**34.** Verbindung nach Anspruch 23, die Natrium-(3S,4S)-cis-3-benzyloxycarboxamido-4-carbamoyloxymethyl-2-azetidinon-1-sulfonat ist.

**35.** Verbindung nach Anspruch 23, die cis-3-Amino-4-carbamoyloxymethyl-2-azetidinon-1-sulfonsäure ist.

**36.** Verbindung nach Anspruch 23, die (3S,4S)-cis-3-Amino-4-carbamoyloxymethyl-2-azetidinon-1-sulfonsäure ist.

**37.** Verfahren zur Herstellung einer Verbindung der Formel

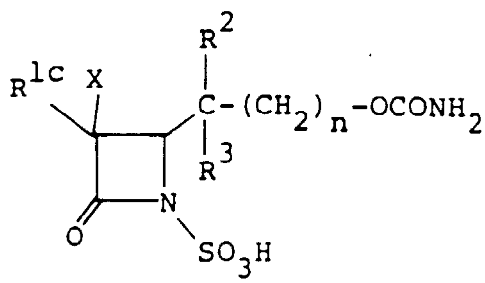

in der $R^{1c}$ eine Amino-Gruppe ist, die gegebenenfalls geschützt sein kann, X Wasserstoff oder Methoxy ist, n eine ganze Zahl von 0 bis 3 ist und $R^2$ und $R^3$, die gleich oder verschieden sein können, für Wasserstoff, Alkyl, Cycloalkyl*, Aralkyl*, Aryl*, eine heterocyclische* Gruppe, Alkoxycarbonyl oder Acyl stehen oder $R^2$ und $R^3$, zusammen genommen, für Oxo stehen und die vorstehenden Gruppen mit einem Sternchen "*" mit ein bis drei Substituenten substituiert sein können, die gleich oder verschieden sein können, oder ein Salz oder Ester derselben, umfassend das Sulfonieren einer Verbindung der Formel

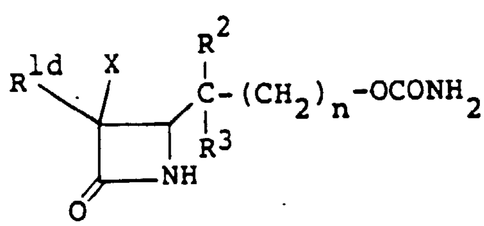

in der $R^{1d}$ eine Amino-Gruppe ist, die gegebenenfalls geschützt sein kann, und die anderen Gruppen die im Vorstehenden angegebenen Bedeutungen haben, oder eines Salzes oder Esters derselben und erforderlichenfalls das Abspalten der Schutzgruppe.

**Revendications**

**1.** Dérivé 1-sulfo-2-oxoazetidine de formule:

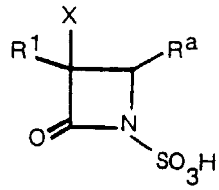

dans laquelle

(1) si X est un atome d'hydrogène ou un groupe méthoxy et $R^1$ est un groupe (alcoxy en $C_{1-6}$)-carbonyl-amino, un groupe (aralkyl en $C_{7-9}$)-oxycarbonylamino, ou un groupe de formule:

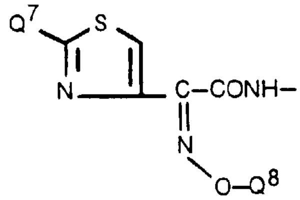

dans laquelle
$Q^7$ est un groupe amino ou un groupe amino protégé, et $Q^8$ est un groupe alkyle en $C_{1-6}$, un groupe de formule $-CH_2COOQ^9$ ou un groupe de formule $-C(CH_3)_2-COOQ^9$, $COOQ^9$ représentant un groupe carboxyle ou un groupe (aralkyle en $C_{7-9}$)-oxycarbonyle qui peut être substitué par un groupe nitro,
$R^a$ représente un groupe de formule $-COQ^{0a}$ dans laquelle $Q^{0a}$ représente un groupe hydrazino, carbamoylhydrazino, (alcoxy en $C_{1-6}$)-carbonylhydrazino ou carbamoyl-(alkyle en $C_{1-4}$)-amino, ou bien un groupe de formule $-(CH_2)_{na}-R^{4b}$, dans laquelle na représente un nombre entier valant de 1 à 3, et $R^{4b}$ représente un groupe carbamoylamino, (N-sulfocarbamoyl)amino, pyridino, alkylsulfinyl en $C_{1-6}$, alkylsulfonyl en $C_{1-6}$, 1-(alcoxyimino en $C_{1-6}$)-(alkyl en $C_{1-6}$), ou formylamino;
(2) si $R^1$ est un groupe de formule:

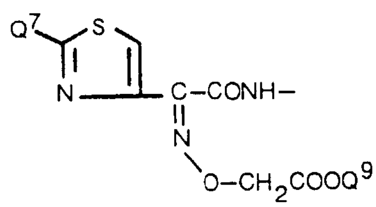

dans laquelle $Q^7$ et $COOQ^9$ ont les mêmes significations que celles définies ci-dessus,
$R^a$ représente un groupe (alkyle en $C_{1-6}$)-aminocarbonyle;
(3) si $R^1$ représente un groupe D-2-(6,7-dihydroxychromone-3-carboxamido)-2-(4-hydroxyphényl)-acétamido,
$R^a$ est un groupe carbamoyle
(4) si $R^1$ est un groupe de formule

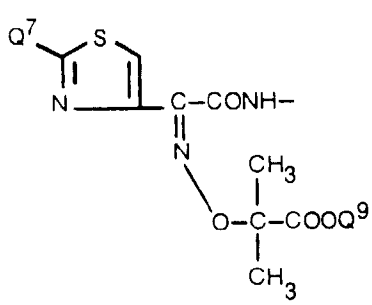

dans laquelle

$Q^7$ est $COOQ^9$ ont les mêmes significations que celles définies ci-dessus,

$R^a$ représente un groupe carboxyle, un groupe de formule $-CH_2CH_2R^{4c}$ dans laquelle $R^{4c}$ est un groupe (alcoxy en $C_{1-6}$)-carbonyle ou acyloxy en $C_{2-4}$, un groupe monochloroacétoxyméthyle, un groupe hydroxyméthyle ou un groupe propyle;

(5) si $R^1$ est un groupe (alcoxy en $C_{1-6}$)-carbonylamino,

$R^a$ représente le groupe $-CH_2OSO_2CH_3$;

(6) si $R^1$ est un groupe de formule:

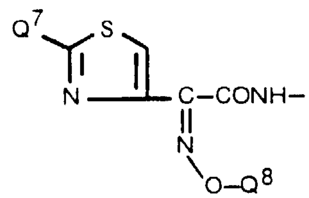

dans laquelle

$Q^7$ et $Q^8$ ont les mêmes significations que celles mentionnées ci-dessus,

$R^a$ représente un groupe sulfaminocarbonyloxyméthyle, un groupe halogéno-(alkyle en $C_{1-6}$)-carbonylcarbamoyloxyméthyle, un groupe pyrazolyle ou un groupe isoxazolyle, qui peut être substitué par un groupe alkyle en $C_{1-6}$ et/ou un groupe acyle en $C_{2-7}$; ou bien

(7) si $R^1$ est un groupe de formule:

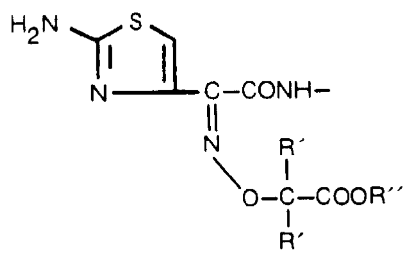

dans laquelle

R' représente un atome d'hydrogène ou un groupe alkyle en $C_{1-6}$, et R" répresente un atome d'hydrogène, un groupe alkyle en $C_{1-6}$ ou un groupe aralkyle en $C_{7-9}$ qui peut être substitué par un groupe nitro,

$R^a$ représente le groupe carbamoyloxyméthyle, et le dérivé présente la configuration (3S,4S); ou un sel ou ester pharamaceutiquement acceptable de ce dérivé.

**2.** Composé conforme à la revendication 1, qui est un dérivé 1-sulfo-2-azétidinone de formule:

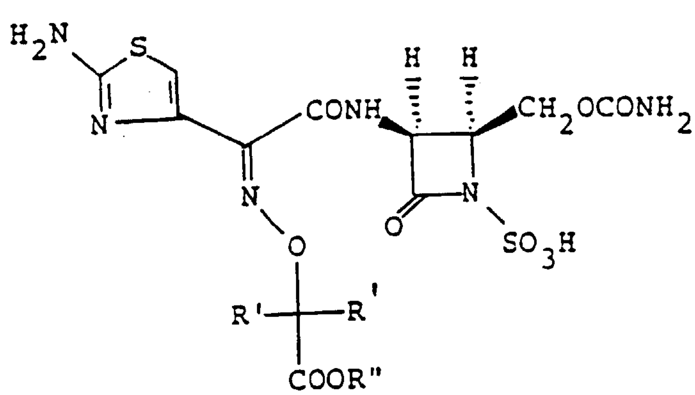

dans laquelle R' et R" sont tels que définis dans la revendication 1, ledit dérivé possédant la configuration (3S, 4S); ou un sel ou ester pharmaceutiquement acceptable de ce dérivé.

**3.** Composé conforme à la revendication 2, dans lequel R' est un atome d'hydrogène.

**4.** Composé conforme à la revendication 2, dans lequel R' répresente un groupe alkyle en $C_{1-4}$.

**5.** Composé conforme à la revendication 4, dans lequel le groupe alkyle en $C_{1-4}$ est un groupe méthyle.

**6.** Composé conforme à la revendication 2, dans lequel R" est un atome d'hydrogène.

**7.** Composé conforme à la revendication 2, dans lequel le sel est un sel d'un cation non toxique, d'un acide aminé à caractère basique ou d'une polyhydroxyalkylamine avec le groupe sulfo et/ou le groupe carboxyle, ou bien un sel d'un acide organique, d'un acide minéral ou d'un acide aminé à caractère acide, avec le groupe amino.

**8.** Composé conforme à la revendication 7, dans lequel le sel d'un cation non toxique est un sel mono-sodique ou disodique.

**9.** Composé conforme à la revendication 8, dans lequel le sel disodique est sous forme cristallisé.

**10.** Composé conforme à la revendication 2, dans lequel l'ester est un ester α-(alcoxy en $C_{1-4}$)-(alkyle en $C_{1-4}$), de (alkyle en $C_{1-4}$)-thiométhyle, d'α-(acyloxy en $C_{2-6}$)-(alkyle en $C_{1-4}$) ou d'α-(alcoxy en $C_{1-4}$)-carbonyloxy-(alkyle en $C_{1-4}$).

**11.** Composé conforme à la revendication 2, qui est l'acide (3S,4S)-*cis*-3-(2-(2-amino-4-thiazolyl)-(Z)-2-carboxyméthoxyimino)-acétamido)4-carbamoyloxyméthyl-2-azétidinone-1-sulfonique.

**12.** Composé conforme à la revendication 2, qui est le sel disodique cristallisé de l'acide (3S,4S)-*cis*-3-(2-(2-amino-4-thiazolyl) - (Z) - 2 - carboxyméthoxyiminoacétamido) - 4 - carbamoyloxyméthyl - 2 - azétidinone - 1 - sulfonique.

**13.** Composé conforme à la revendication 2, qui est l'acide (3S,4S)-*cis*-3-(2-amino-4-thiazolyl)-(Z)-2-(1-carboxy-1-méthyléthoxyimino)acétamido)-4-carbamoyloxyméthyl-2-azétidinone-1-sulfonique.

**14.** Procédé de production d'un dérivé 1-sulfo-2-oxoazétidine de formule:

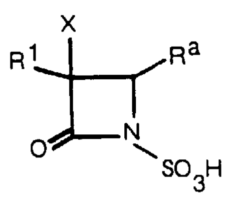

dans laquelle les significations des symboles sont les mêmes que dans la revendication 1, ou d'un sel ou ester pharmaceutiquement acceptable de ce dérivé, qui comporte la sulfonation d'un composé de formule

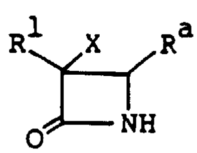

dans laquelle les significations de tous les symboles sont celles définies dans la revendication 1, ou d'un sel ou ester de celui-ci, et si nécessaire, l'élimination de tout groupe protecter.

**15.** Procédé de production d'un dérivé 1-sulfo-2-oxoazétidine de formule

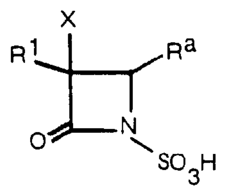

dans laquelle les symboles ont les significations définies dans la revendication 1, ou d'un sel ou ester pharmaceutiquement acceptable de ce dérivé, qui comporte l'acylation d'un composé de formule:

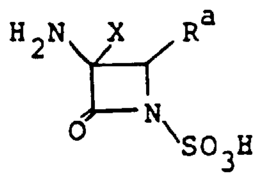

dans laquelle tous les symboles ont les significations définies dans la revendication 1, ou d'un sel ou ester de celui-ci, et si nécessaire, l'élimination de tout groupe protecteur.

**16.** Procédé conforme à la revendication 14, dans lequel un dérivé 1-sulfo-2-azétidinone de formule:

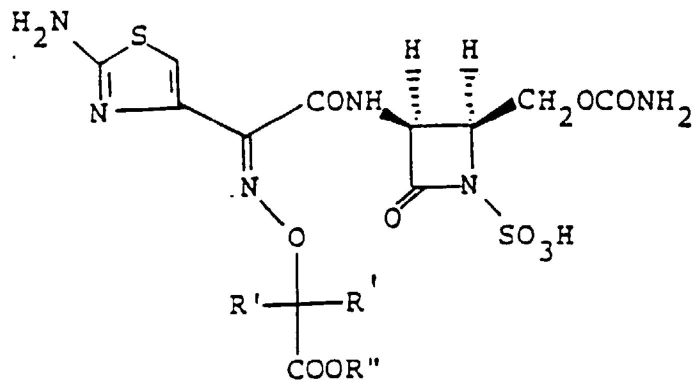

dans laquelle R' et R" ont les significations définies dans la revendication 1, ce dérivé présentant la configuration (3S,4S), ou un sel ou ester pharmaceutiquement acceptable de ce dérivé, est produit par sulfonation d'un composé de formule:

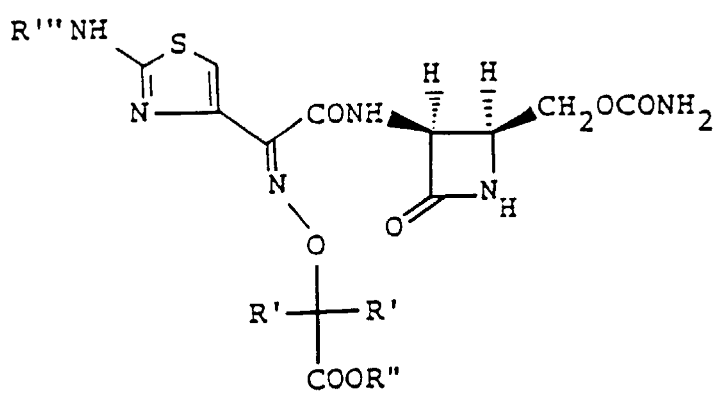

dans laquelle R' et R" ont les significations définies dans la revendication 1 et R"' est un groupe amino-protecteur,

ou d'un sel de ce composé, et élimination ultérieure du groupe protecteur et, si nécessaire, du résidu ester.

**17.** Procédé conforme à la revendication 15, dans lequel un dérivé 1-sulfo-2-azétidinone de formule:

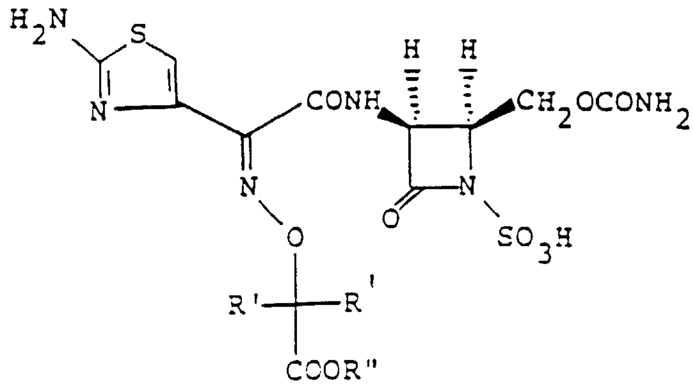

dans laquelle R' et R" ont les significations définies dans la revendication 1, ce dérivé présentant la configuration (3S,4S), ou un sel ou ester pharmaceutiquement acceptable de ce dérivé, est produit par réaction de l'acide (3S, 4S)-*cis*-3-amino-4-carbamoyloxyméthyl-2-azétidinone-1-sulfonique ou d'un sel ou ester de celui-ci avec un acide carboxylique de formule

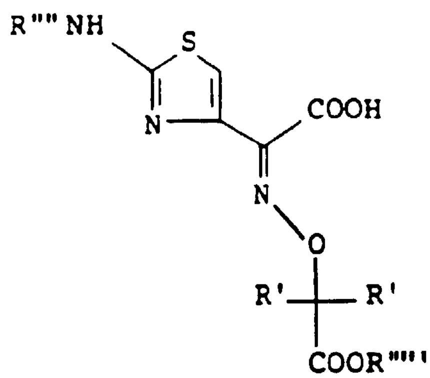

dans laquelle R' a la signification définie dans la revendication 1, R"" est un atome d'hydrogène ou un groupe amino-protecteur et R""' est un résidu ester, ou un dérivé fonctionnel de celui-ci, et élimination ultérieure du groupe protecteur et, si nécessaire, du résidu ester.

**18.** Procédé conforme à la revendication 17, dans lequel le dérivé fonctionnel est un dérivé thioester actif.

**19.** Procédé conforme à la revendication 18, dans lequel le thioester actif est un 2-benzothiazolylthioester.

**20.** Préparation anit-microbienne qui contient une dose efficace d'un dérivé 1-sulfo-2-oxoazétidine de formule:

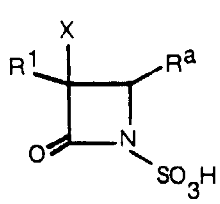

dans laquelle les symboles ont les significations définies dans la revendication 1, ou d'un sel ou ester pharmaceutiquement acceptable de ce dérivé en tant que composant actif, conjointement avec un véhicule ou des véhicules convenables.

**21.** Préparation anti-microbienne qui contient un antibiotique β-lactame et, en tant qu'inhibiteur de β-lactamase, une quantité efficace d'un dérivé 1-sulfo-2-oxoazétidine de formule:

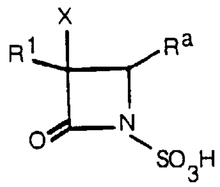

dans laquelle les symboles ont les significations définies dans la revendication 1, ou d'un sel ou ester pharmaceutiquement acceptable de ce dérivé, conjointement avec un véhicule ou des véhicules convenables.

**22.** Composé de formule:

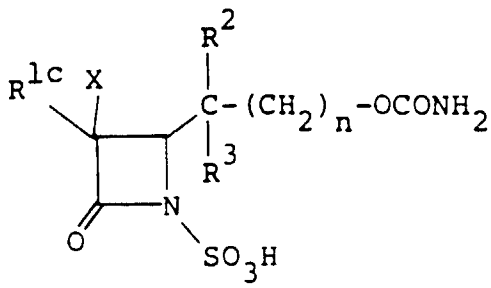

dans laquelle

$R^{1c}$ représente un groupe amino qui peut être éventuellement protégé;

X représente un atome d'hydrogène ou un groupe méthoxy;

n représente un nombre entier valant de 0 à 3; et

$R^2$ et $R^3$, qui peuvent être identiques ou différents, représentent chacun un atome d'hydrogène, un groupe alkyle, un groupe cycloalkyle*, un groupe aralkyle*, un groupe aryle*, un groupe hétérocyclique*, un groupe alcoxycarbonyle ou un groupe acyle, ou bien $R^2$ et $R^3$ représentent conjointement un groupe oxo;

les groupes désignés ci-dessus avec un exposant astérisque "*" pouvant porter de 1 à 3 substituants qui peuvent être identiques ou différents;

ou un sel ou ester de ce composé.

**23.** Composé conforme à la revendication 22, qui est un composé de formule:

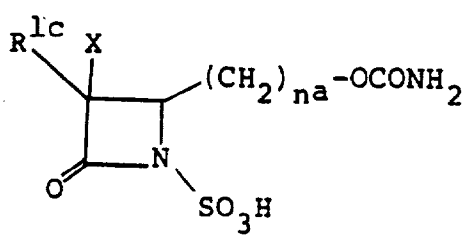

dans laquelle $R^{1c}$ représente

un groupe amino qui peut être éventuellement protégé;

X représente un atome d'hydrogène ou un groupe méthoxy; et

$n^a$ représente un nombre entier valant de 1 à 3.

**24.** Composé conforme à la revendication 22, dans lequel $R^{1c}$ représente un groupe amino.

**25.** Composé conforme à la revendication 22, dans lequel $R^{1c}$ représente un groupe amino protégé.

**26.** Composé conforme à la revendication 25, dans lequel le groupe protecteur du groupe amino protégé est un groupe acyle aromatique, un groupe acyle aliphatique, un groupe carboxyle estérifié ou un groupe aminoprotecteur non acyle.

**27.** Composé conforme à la revendication 25, dans lequel le groupe protecteur du groupe amino protégé est un groupe carboxyle estérifié.

**28.** Composé conforme à la revendication 27, dans lequel le groupe carboxyle estérifié est un groupe benzyloxycarbonyle.

**29.** Composé conforme à la revendication 24, dans lequel X représente un atome d'hydrogène.

**30.** Composé conforme à la revendication 24, dans lequel $n^a$ vaut 1.

**31.** Composé conforme à la revendication 23, dans lequel le groupe représenté par le symbole $R^{1c}$ présente la configuration β et le groupe représenté par le symbole X présente la configuration α, par rapport au noyau azétidine dans les deux cas.

**32.** Composé conforme à la revendication 23, dans lequel le groupe représenté par le symbole

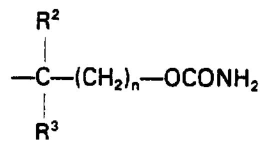

présente la configuration *cis* par rapport au groupe $R^{1c}$.

**33.** Composé conforme à la revendication 23, qui est le *cis*-3-benzyloxycarboxamido-4-carbamoyloxyméthyl-2-azétidinone-1-sulfonate de sodium.

**34.** Composé conforme à la revendication 23, qui est le (3S,4S)-*cis*-3-benzyloxycarboxamido-4-carbamoyloxyméthyl-2-azétidinone-1-sulfonate de sodium.

**35.** Composé conforme à la revendication 22, qui est l'acide *cis*-3-amino-4-carbamoyloxyméthyl-2-azétidinone-1-sulfonique.

**36.** Composé conforme à la revendication 23, qui est l'acide (3S,4S)-*cis*-3-amino-4-carbamoyloxyméthyl-2-azétidinone-1-sulfonique.

**37.** Procédé de production d'un composé de formule:

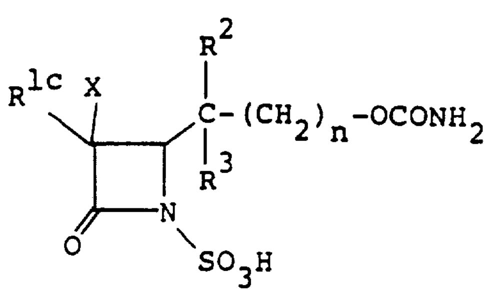

dans laquelle $R^{1c}$ est un groupe amino qui peut être éventuellement protégé;
X représente un atome d'hydrogène ou un groupe méthoxy;
n représente un nombre entier valant de 0 à 3; et
$R^2$ et $R^3$, qui peuvent être indentiques ou différents, représentent un atome d'hydrogène ou un groupe alkyle,

cycloalkyle*, aralkyle*, aryle*, hétérocyclique*, alcoxycarbonyle ou acyle, ou bien $R^2$ et $R^3$, pris conjointement, représentent un groupe oxo;

les groupes indiqués ci-dessus par un exposant astérique "*" pouvant porter de 1 à 3 substituants qui peuvent être identiques ou différents;

ou d'un sel ou ester de celui-ci, lequel procédé comporte la sulfonation d'un composé de formule:

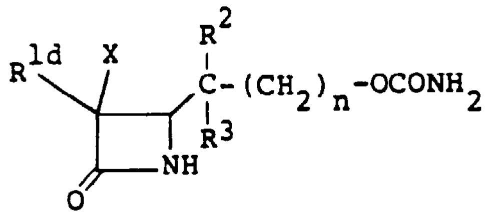

dans laquelle $R^{1d}$ représenté un groupe amino qui peut être éventuellement protégé, et les autres groupes sont tels que définis ci-dessus, ou d'un sel ou ester de celui-ci, et si nécessaire, l'élimination du groupe protecteur.

Fig. I

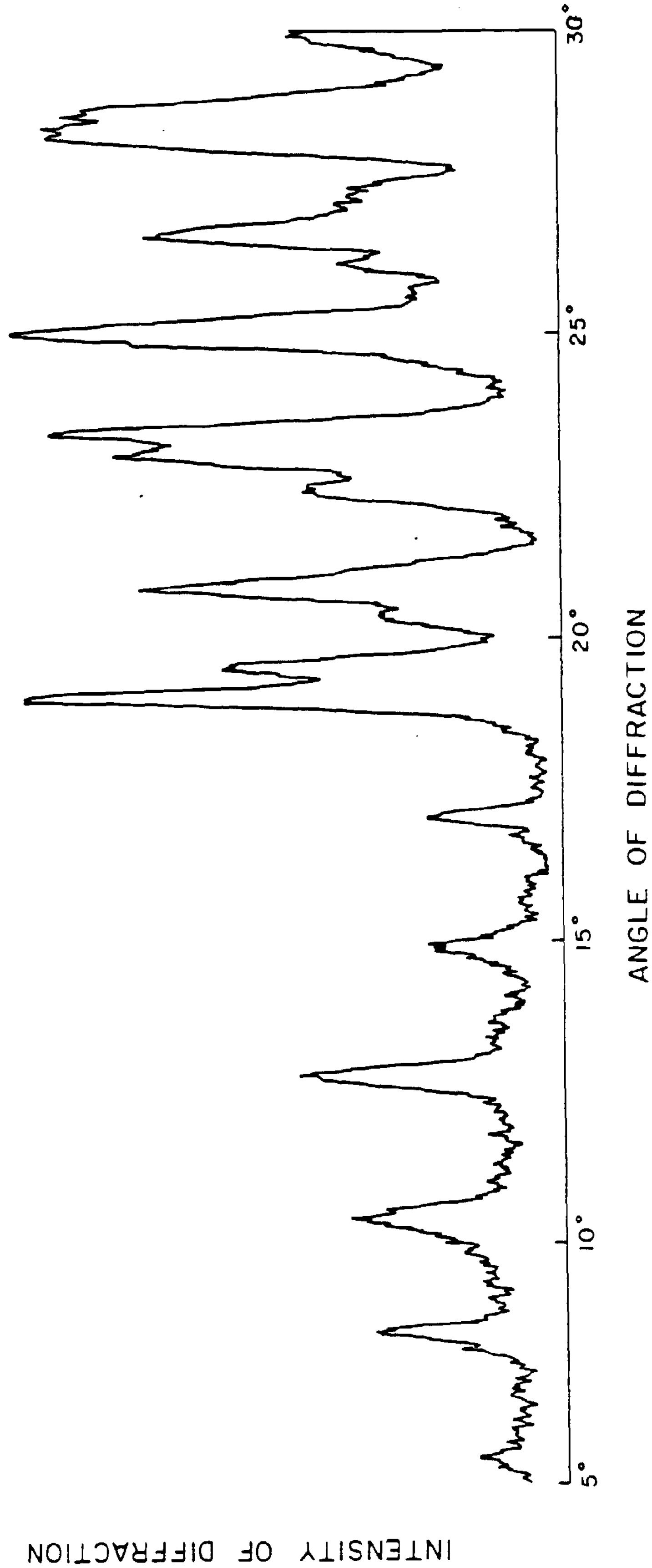
5°
10°
15°
20°
25°
30°
ANGLE OF DIFFRACTION
INTENSITY OF DIFFRACTION